(19) Europäisches Patentamt
European Patent Office
Office européen des brevets

(11) EP 3 765 002 B1

(12) EUROPEAN PATENT SPECIFICATION

(45) Date of publication and mention of the grant of the patent:
**05.03.2025 Bulletin 2025/10**

(21) Application number: **19766575.5**

(22) Date of filing: **15.03.2019**

(51) International Patent Classification (IPC):
***A61K 9/16*** *(2006.01)* ***A61K 31/12*** *(2006.01)*
***A61K 31/21*** *(2006.01)* ***A61P 25/28*** *(2006.01)*
***A61K 31/215*** *(2006.01)* ***A61K 31/23*** *(2006.01)*
***A61K 45/06*** *(2006.01)*

(52) Cooperative Patent Classification (CPC):
(C-Sets available)
**A61K 9/1617; A61K 9/1611; A61K 9/1641; A61K 9/1664; A61K 31/215; A61K 31/23; A61K 45/06; A61P 25/28** (Cont.)

(86) International application number:
**PCT/US2019/022478**

(87) International publication number:
**WO 2019/178482 (19.09.2019 Gazette 2019/38)**

(54) **PHARMACEUTICAL COMPOSITIONS HAVING HIGH DRUG LOADINGS OF MEDIUM CHAIN TRIGLYCERIDES AND METHODS RELATED THERETO**

PHARMAZEUTISCHE ZUSAMMENSETZUNGEN MIT HOHEM WIRKSTOFFGEHALT AN MITTELKETTIGEN TRIGLYCERIDEN UND VERFAHREN IM ZUSAMMENHANG DAMIT

COMPOSITIONS PHARMACEUTIQUES AYANT DES CHARGES MÉDICAMENTEUSES ÉLEVÉES DE TRIGLYCÉRIDES À CHAÎNE MOYENNE ET PROCÉDÉS ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2018 US 201862643479 P**

(43) Date of publication of application:
**20.01.2021 Bulletin 2021/03**

(73) Proprietor: **Cerecin Inc.**
**Denver, CO 80206 (US)**

(72) Inventors:
- **BOIVIN, Taryn**
  **Surrey, British Columbia V4A 9W5 (CA)**
- **DUBOSE, Devon B.**
  **Bend, Oregon 97703 (US)**
- **HENDERSON, Samuel T.**
  **Golden, Colorado 80403 (US)**
- **HOSTETLER, Christi Lynn**
  **Bend, Oregon 97702 (US)**
- **LYON, David K.**
  **Bend, Oregon 97702 (US)**
- **SATHER, Craig A.**
  **Bend, Oregon 97701 (US)**
- **SHAFFER, Matthew J.**
  **Bend, Oregon 97701 (US)**

(74) Representative: **Mewburn Ellis LLP**
**Aurora Building**
**Counterslip**
**Bristol BS1 6BX (GB)**

(56) References cited:
**EP-A1- 2 319 508**
**WO-A1-2017/149392**
**WO-A1-2017/149392**
**WO-A2-00/67728**
**US-A1- 2008 009 467**
**US-A1- 2016 354 335**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 31/215, A61K 2300/00;**
**A61K 31/23, A61K 2300/00**

## Description

### FIELD OF THE INVENTION

This disclosure relates to pharmaceutical compositions comprising high drug loadings of medium chain triglycerides, as well as methods of making and methods of using such compositions.

### BACKGROUND OF THE INVENTION

Medium Chain Tryglycerides (MCTs) are comprised of fatty acids with chain length between 5-12 carbons. MCTs have been researched extensively and have known nutritional and pharmaceutical uses. MCTs having melting points which are liquid at room temperature. Further, MCTs are relatively small and are ionizable at physiological pH, and hence are generally soluble in aqueous solutions.

When intended to be used as a pharmaceutical composition, it is often more desirable to prepare compositions of active ingredients that are present as a liquid at room temperature, such as MCTs, as a solid dosage form.

As such, there is a need in the art for solid dosage form compositions of MCTs, particularly at active ingredient to excipient levels (herein referred to as drug loading) sufficiently high for pharmaceutical use.

EP 2 319 508 A1 describes methods and compositions for treating or preventing the occurrence of senile dementia of the Alzheimer's type, or other conditions arising from reduced neuronal metabolism. WO 2017 /149392 Al discloses self-emulsifying compositions comprising at least one CB2 receptor modulator, a self-emulsifying vehicle and optionally one antipsychotic agent for use in the treatment of mental disorders.

### SUMMARY OF THE INVENTION

The invention is defined in the appended claims.

In one aspect, the invention relates to a pharmaceutical composition comprising a high drug loading of an MCT, i.e., caprylic triglyceride, and at least one matrix forming agent. According to the invention, the matrix forming agent is selected from the group consisting of glyceryl dibehenate, carnauba wax, or combinations thereof. The composition may form discrete particles including the high drug loading of an MCT and a matrix forming agent. In certain embodiments, the particles have an average diameter, of, e.g., between about 50 μm and about 350 μm. In other embodiments, the particles exhibit a Carr Index of flowability of less than 10%. The composition may comprise a dissolution enhancer, a flow aid, a stiffening agent, and combinations thereof.

According to the invention, the MCT is a caprylic triglyceride, which is present in an amount of between 30% and 50%, by weight of the total composition. According to the invention, the at least one matrix forming agent is selected from the group consisting of glyceryl dibehenate, carnauba wax and combinations thereof, and the matrix forming agent is present in an amount of between about 20% and about 70%, by weight of the total composition.

Not according to the invention but as described herein, the glyceride may be selected from glyceryl behenate, glyceryl trimyristate, glyceryl trilaurate, glyceryl tristearate, glyceryl monostearate, glyceryl palmitostearate, and glyceryl triacetate, and combinations thereof. The natural wax, if present in the inventive compositions, is carnauba wax.

In certain aspects, the dissolution enhancer is selected from the group consisting of poly(ethylene glycol)-block-poly(propylene glycol)-block-poly(ethylene glycol) copolymer, polyethylene glycol copolymer, starch, rice starch, lecithin, polyvinylpyrrolidone (PVP), polyoxylglycerides, polyvinylpyrrolidone-vinyl acetate copolymer (PVP-VA), hydroxypropyl methylcellulose (HPMC), hypromellose acetate succinate (HPMCAS), Low-substituted hydroxypropyl cellulose (L-HPC), sorbitan esters, polyethylene glycol, sorbitan fatty acid esters, and combinations thereof.

In yet other aspects, the flow aid is selected from the group consisting of amphorous silica gel, silica aerogel, magnesium alumino metasilicates, calcium silicate, ordered mesoporous silica, micronized talc, and combinations thereof.

In yet other aspects, the stiffening agent is selected from the group consisting of carnauba wax, candelilla wax, and combinations thereof.

Not according to the present invention but as described herein, when the pharmaceutical composition is subjected to dissolution testing using USP-II Sink dissolution post acid exposure methodology, at least 40% but not more than 60% of the caprylic triglyceride releases from the composition in the first 60 minutes, at least 60% but not more than 80% of the caprylic triglyceride releases from the composition in the 120 minutes, and at least 80% of the caprylic triglyceride releases from the composition in the first 240 minutes. In other embodiments, when the pharmaceutical composition is subjected to dissolution testing using USP-II Sink dissolution post acid exposure methodology, at least 90% of the caprylic triglyceride releases from the composition in the first 360 minutes.

In yet other aspects, the invention relates to a pharmaceutical composition for use in methods of treating a disease or disorder associated with reduced cognitive function in a subject in need thereof, the method comprising

administering to the subject a pharmaceutical composition of the disclosure in an amount effective to elevate ketone body concentrations in said subject to thereby treat said disease or disorder. In certain embodiments, the disease or disorder associated with reduced cognitive function is selected from Alzheimer's disease and Age-Associated Memory Impairment.

While multiple embodiments are disclosed, still other embodiments of the present disclosure will become apparent to those skilled in the art from the following detailed description, which shows and describes illustrative embodiments of the disclosure.

## BRIEF DESCRIPTION OF THE DRAWINGS

FIGS. 1A-D illustrate particle size and morphology of exemplary pharmaceutical formulations LMP-1, LMP-2, LMP-3, and LMP-4 in accordance with embodiments of the disclosure. FIG. 1A depicts an image of particles of LMP-1 (50/50 caprylic triglyceride / microcrystalline wax) at 6X magnification. FIG. 1B depicts an image of particles of LMP-2 (50/50 caprylic triglyceride / glyceryl dibehenate) at 5X magnification. FIG. 1C depicts an image of particles of LMP-3 (50/50 caprylic triglyceride / Kolliphor CSL) at 5X magnification. FIG. 1D depicts an image of particles of LMP-4 (50/50 caprylic triglyceride / Dynasan 118) at 5X magnification.

FIGS. 2A-D illustrate scanning electron microscopy (SEM) images of exemplary formulations LMP-1 (FIG. 2A), LMP-2 (FIG. 2B), LMP-3 (FIG. 2C), and LMP-4 (FIG. 2D) in accordance with embodiments of the disclosure at 50x magnification.

FIGS. 3A-D illustrate SEM images of exemplary formulations LMP-1 (FIG. 3A), LMP-2 (FIG. 3B), LMP-3 (FIG. 3C), and LMP-4 (FIG. 3D) in accordance with embodiments of the disclosure at 200x magnification.

FIGS. 4A-D illustrate SEM images of exemplary formulations LMP-1 (FIG. 4A), LMP-2 (FIG. 4B), LMP-3 (FIG. 4C), and LMP-4 (FIG. 4D) in accordance with embodiments of the disclosure at 200x magnification.

FIGS. 5A-C illustrate differential scanning calorimetry (DSC) heat flows of caprylic triglyceride (Miglyol 808) in accordance with aspects of the disclosure. FIG. 5A depicts Cooling DSC heat flows of caprylic triglyceride (Miglyol 808) as a function of ramp rate. FIG. 5B depicts heating DSC heat flows of caprylic triglyceride (Miglyol 808) as a function of ramp rate showing exotherms before melt. FIG. 5C depicts heating DSC heat flows of caprylic triglyceride as a function of ramp rate showing comparison of melting.

FIG. 6 illustrates the melting point profiles of exemplary formulations LMP-1, LMP-2, and LMP-3 in accordance with embodiments of the disclosure.

FIGS. 7A-C illustrate chromatograms of exemplary formulations LMP-1, LMP-2, and LMP-3 versus the excipient (microcrystalline wax, Compritol 888, or Kolliphor CSL) and caprylic triglyceride (Miglyol 808) in accordance with embodiments of the disclosure.

FIG. 8 shows dissolution visual results for exemplary formulations LMP-1, LMP-2, and LMP-3 in 0.25% SLS solution at ~37 °C, ~25□C, and ~5 °C. FIG. 8 also shows dissolution visual results for exemplary formulation LMP-1 in 0.5% SLS solution at ~37□C, ~25 °C, and ~5 °C in accordance with aspects of the disclosure.

FIG. 9 shows dissolution visual results for caprylic triglyceride (Miglyol 808) in a water solution, 0.25% SLS solution, and 0.25% SLS solution in accordance with aspects of the disclosure.

FIGS. 10A-D illustrate SEM images of exemplary formulations LMP-5 (FIG. 10A), LMP-7 (FIG. 10B), LMP-9 (FIG. 10C), and LMP-11 (FIG. 10D) at 50x magnification in accordance with aspects of the disclosure.

FIGS. 11A-D illustrate SEM images of exemplary formulations LMP-5 (FIG. 11A), LMP-7 (FIG. 11B), LMP-9 (FIG. 11C), and LMP-11 (FIG. 11D) at 1500x magnification in accordance with aspects of the disclosure.

FIG. 12 illustrates the heat-cool DSC scans of exemplary formulations of 50% loaded LMP-2, LMP-7, LMP-9, and LMP-11 in accordance with aspects of the disclosure.

FIG. 13 illustrates the cool-heat DSC scans of exemplary formulations of 50% loaded LMP-2, LMP-7, LMP-9, and LMP-11 in accordance with aspects of the disclosure.

FIGS. 14A-H illustrate cool-heat and heat-cool DSC scans of exemplary formulations LMP-2, LMP-7, LMP-9, and LMP-11 overlaid with excipient(s) in accordance with aspects of the disclosure.

FIGS. 15A-D illustrate chromatograms of exemplary formulations LMP-5 (FIG. 15A), LMP-7 (FIG. 15B), LMP-9 (FIG. 15C), and LMP-11 (FIG. 15D) in accordance with embodiments of the disclosure.

FIG. 16 illustrates the sink dissolution rates of exemplary formulations LMP-5, LMP-7, LMP-9, and LMP-11 in accordance with aspects of the disclosure.

FIGS. 17A-C illustrate the sink dissolution rate of exemplary formulations LMP-5 (FIG. 17A) and LMP-11 (FIG. 17B) after one month at 5 °C, 25 °C, and 40 °C, and LMP-5 after 1 month storage as described herein plus 1 week storage at ambient conditions (FIG. 17C) in accordance with aspects of the disclosure.

FIGS. 18A-H illustrate SEM images of exemplary formulation LMP-5 prior to storage (FIGS. 18A-B) and after storage

for one month (FIG. 18C-H) in accordance with aspects of the disclosure.

FIGS. 19A-H illustrate SEM images of exemplary formulation LMP-11 prior to storage (FIGS. 19A-B) and after storage for one month (FIGS. 19C-H) in accordance with aspects of the disclosure.

FIGS. 20A-H illustrate SEM images of exemplary formulations LMP BREC1650-155 (500X), -156 (500X), -157(500X), -158 (500X), -160 (500X), -161 (500X), -162 (500X), -156 (100X), and -161 (100X) in accordance with aspects of the disclosure.

FIG. 21 illustrates the sink dissolution rates of exemplary formulations LMP BREC1690-155, -156, -160, -161, and -162 in accordance with aspects of the disclosure.

FIG. 22 compares the sink dissolution rates of exemplary formulations LMP-2, LMP-5, LMP BREC1690-155, -156, and -162 in accordance with aspects of the disclosure.

FIG. 23 compares the sink dissolution rates of exemplary formulations LMP-11, LMP BREC1690-160 and -161 in accordance with aspects of the disclosure.

FIG. 24 illustrates the in vitro digestion profiles for exemplary formulations LMP-5 and LMP BREC1690-156, -157, and -162 (LMP -156,-157, and -162) in accordance with aspects of the disclosure.

FIG. 25 illustrates the in vitro digestion profiles for exemplary formulation LMP BREC1690-156 initially, after 2 weeks at 25°C/60%RH and 40°C/75%RH, and after 1 month at 25°C/60%RH and 40°C/75%RH in accordance with aspects of the disclosure.

FIG. 26 illustrates the in vitro digestion profiles for exemplary formulation LMP BREC1690-162 initially, after 2 weeks at 25°C/60%RH and 40°C/75%RH, and after 1 month at 25°C/60%RH and 40°C/75%RH in accordance with aspects of the disclosure.

FIGS. 27A-H illustrate SEM images of exemplary formulation LMP BREC1690-156 prior to storage (FIG. 27A) and after storage for two weeks (FIGS. 27B-D) in accordance with aspects of the disclosure.

FIGS. 28A-D illustrate SEM images of exemplary formulation LMP BREC1690-162 prior to storage (FIG. 28A) and after storage for two weeks (FIGS. 28B-D) in accordance with aspects of the disclosure.

FIGS. 29A-D illustrate SEM images of exemplary formulation LMP BREC1690-156 prior to storage (FIG. 29A) and after storage for one month (FIGS. 29B-D) in accordance with aspects of the disclosure.

FIGS. 30A-D illustrate SEM images of exemplary formulation LMP BREC1690-162 prior to storage (FIG. 30A) and after storage for one month (FIGS. 30B-D) in accordance with aspects of the disclosure.

FIGS. 31A-C show SEM images of 50/35/15 MCT/Carnauba Wax/Gelucire 50/13 LMPs at 50x (FIG. 31A), 300x (FIG. 31B), and 1500x magnification (FIG. 31C), in accordance with aspects of the disclosure.

FIGS. 32A-C show SEM images of 40/40/20 MCT/Carnauba Wax/Gelucire 50/13 LMPs at 50x (FIG. 32A), 300x (FIG. 32B), and 1500x magnification (FIG. 32C), in accordance with aspects of the disclosure.

FIGS. 33A-C show SEM images of 50/50 MCT/Rice Bran Wax LMPs at 50x (FIG. 33A), 300x (FIG. 33B), and 1500x magnification (FIG. 33C), in accordance with aspects of the disclosure.

FIGS. 34A-C show SEM images of 50/40/10 MCT/Compritol/PVP-17 LMPs at 50x (FIG. 34A), 300x (FIG. 34B), and 1500x magnification (FIG. 34C), in accordance with aspects of the disclosure.

FIGS. 35A-C show SEM images of 40/48/12 MCT/Compritol/PVP-17 LMPs at 50x (FIG. 35A), 300x (FIG. 35B), and 1500x magnification (FIG. 35C), in accordance with aspects of the disclosure.

## DETAILED DESCRIPTION OF THE INVENTION

By way of background, Medium Chain Triglyceride ("MCT"s) are metabolized differently from the more common Long Chain Triglycerides (LCTs). In particular, when compared to LCTs, MCTs are more readily digested to release medium chain fatty acids (MCFAs), which exhibit increased rates of portal absorption, and undergo obligate oxidation. The small size and decreased hydrophobicity of MCTs increases the rate of digestion and absorption relative to LCTs. When MCTs are ingested, they are first processed by lipases, which cleave the fatty acid chains from the glycerol backbone. Some lipases in the pre-duodenum preferentially hydrolyze MCTs over LCTs, and the released MCFAs are then partly absorbed directly by the stomach mucosa. Those MCFAs which are not absorbed in the stomach are absorbed directly into the portal vein and not packaged into lipoproteins. Since blood transports much more rapidly than lymph, MCFAs quickly arrive at the liver. In the liver MCFAs undergo obligate oxidation.

In contrast, long chain fatty acids (LCFAs) derived from normal dietary fat are re-esterified into LCTs and packaged into chylomicrons for transport in the lymph. This greatly slows the metabolism of LCTs relative to MCTs. In the fed state LCFAs undergo little oxidation in the liver, due mainly to the inhibitory effects of malonyl-CoA. When conditions favor fat storage, malonyl-CoA is produced as an intermediate in lipogenesis. Malonyl-CoA allosterically inhibits carnitine palmitoyltransferase I, and thereby inhibits LCFA transport into the mitochondria. This feedback mechanism prevents futile cycles of lipolysis and lipogenesis.

MCFAs are, to a large extent, immune to the regulations that control the oxidation of LCFAs. MCFAs enter the mitochondria without the use of carnitine palmitoyltransferase I, therefore MCFAs by-pass this regulatory step and are

oxidized regardless of the metabolic state of the organism. Importantly, since MCFAs enter the liver rapidly and are quickly oxidized, large amounts of ketone bodies are readily produced from MCFAs. As such, a large oral dose of MCTs (e.g., about 20 mL to 40 mL) will result in sustained hyperketonemia.

The present disclosure generally relates to pharmaceutical compositions comprising a high loading of at least one MCT, and methods of making and using such compositions. In certain embodiments, the MCT is caprylic triglyceride, as described herein.

In certain aspects of the disclosure, the pharmaceutical compositions provide for preferential release of the high drug loading of MCT in the lower gastrointestinal tract of a user. Without intending to be limited by theory, preferential release of MCT in the lower gastrointestinal tract, including the colon may provide reduced stomach upset and related adverse events as compared to standard administration of non-formulated MCT oil. Further, the improved bioavailability of the MCT may generally lead to increased ketone body production *in vivo,* as compared to standard administration of non-formulated MCT oil.

In certain aspects, the pharmaceutical compositions of the invention are a pharmaceutical composition comprising a high drug loading of an MCT, i.e., caprylic triglyceride, and a matrix forming excipient. According to the invention, the matrix forming agent is selected from the group consisting of glyceryl dibehenate, carnauba wax, or combinations thereof. The composition may form discrete particles including the high drug loading of an MCT and the matrix forming excipient. The composition may further include a dissolution enhancer, a flow aid, a stiffening agent, and combinations thereof. The pharmaceutical compositions may comprise the components in amounts as claimed.

The pharmaceutical compositions of the disclosure provide small, discrete particles having an average particle size e.g., between about 50 $\mu$m and about 350 $\mu$m, between about 100 $\mu$m and about 300 $\mu$m in diameter, etc.

In certain aspects, the pharmaceutical compositions of the disclosure provide discrete particles that form flowable powders. In certain embodiments, the flowable powders exhibit a Carr Index of less than about 20%, 15%, 10%, etc.

In certain embodiments, the pharmaceutical compositions may include a high drug load of at least one MCT, namely caprylic triglyceride, of between 30% by weight of the total composition to 50% by weight of the total composition.

As used herein, unless other specified, "% by weight" refers to "% by weight of the total composition".

Generally, MCTs refer to any glycerol molecule ester-linked to three fatty acid molecules, each fatty acid molecule having a carbon chain of 5-12 carbons. In certain embodiments, the pharmaceutical compositions may comprise an MCT represented by the following general formula:

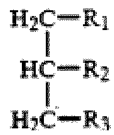

wherein $R_1$, $R_2$ and $R_3$ are fatty acids having 5-12 carbons in the carbon backbone esterified to the a glycerol backbone.

The MCTs of the disclosure may be prepared by any process known in the art, such as direct esterification, rearrangement, fractionation, transesterification, or the like. Sources of the MCT include any suitable source, semi-synthetic, synthetic or natural. Examples of natural sources of MCT include plant sources such as coconuts and coconut oil, palm kernels and palm kernel oils, and animal sources such as milk from any of a variety of species, e.g., goats. For example, the lipids may be prepared by the rearrangement of a vegetable oil such as coconut oil. The length and distribution of the chain length may vary depending on the source oil. For example, MCT containing 1-10% C6, 30-60% C8, 30-60% C10, 1-10% C10 are commonly derived from palm and coconut oils.

In accordance with the present invention, the pharmaceutical compositions of the invention comprise MCTs that have greater than about 95% C8 at $R_1$, $R_2$ and $R_3$, and are herein referred to herein as caprylic triglyceride ("CT"). Exemplary sources of CT include MIGLYOL® 808 or NEOBEE® 895. In certain aspects, CT may be obtained from coconut or palm kernel oil, made by semi-synthetic esterification of octanoic acid to glycerin, etc.

In other embodiments, which are not claimed, the pharmaceutical compositions may comprise MCTs wherein $R_1$, $R_2$, and $R_3$ are fatty acids containing a six-carbon backbone (tri-C6:0). Tri-C6:0 MCT are absorbed very rapidly by the gastrointestinal tract in a number of animal model systems. The high rate of absorption results in rapid perfusion of the liver, and a potent ketogenic response. In another embodiment, the pharmaceutical compositions may comprise MCTs wherein $R_1$, $R_2$, and $R_3$ are fatty acids containing an eight-carbon backbone (tri-C8:0). In another embodiment, the pharmaceutical compositions may comprise MCTs wherein $R_1$, $R_2$, and $R_3$ are fatty acids containing a ten-carbon backbone (tri-C10:0). In another embodiment, the pharmaceutical compositions may comprise MCTs wherein $R_1$, $R_2$, and $R_3$ are a mixture of C8:0 and C10:0 fatty acids. In another embodiment, the pharmaceutical compositions may comprise MCTs wherein $R_1$, $R_2$ and $R_3$ are a mixture of C6:0, C8:0, C10:0, and C12:0 fatty acids. In another embodiment, the pharmaceutical compositions may comprise MCTs wherein greater than 95% of $R_1$, $R_2$ and $R_3$ are 8 carbons in length. In yet another embodiment, the pharmaceutical compositions may comprise MCTs wherein the $R_1$, $R_2$, and $R_3$ carbon chains are 6-carbon or 10-carbon

chains. In another embodiment, the pharmaceutical compositions may comprise MCTs wherein about 50% of $R_1$, $R_2$ and $R_3$ are 8 carbons in length and about 50% of $R_1$, $R_2$ and $R_3$ 10 carbons in length. In one embodiment, the pharmaceutical compositions may comprise MCTs wherein $R_1$, $R_2$ and $R_3$ are 6, 8, 10 or 12 carbon chain length, or mixtures thereof. According to the invention, the pharmaceutical formulations comprise caprylic triglyceride.

According to the invention, the pharmaceutical compositions of the invention include at least one matrix forming agent. According to the invention, the matrix forming agent is selected from the group consisting of glyceryl dibehenate, carnauba wax, or combinations thereof. The matrix forming agent may be present in amounts sufficient to provide desired particle formation. According to the invention, the matrix forming agent is present in an amount of between about 20% and about 70%, between about 40% and about 70%, between about 50% and about 60%, etc., by weight of the total composition.

Any suitable glyceride known in the art which provides the desired particle formation may be used be used as the matrix forming agent. By way of example, the glyceride may be glyceryl behenate, glyceryl dibehenate, glyceryl trimyristate, glyceryl trilaurate, glyceryl tristearate, glyceryl monostearate, glyceryl palmitostearate, and glyceryl triacetate, etc. According to the invention, the glyceride is glyceryl dibehenate, e.g., COMPRITOL® 888.

Any suitable natural wax known in the art which provides the desired particle formation may be used be used as the matrix forming agent. By way of example, the natural wax may include standard natural waxes and hydrogenated oils, e.g., including hydrogenated castor oil (HCO), carnauba wax, rice bran wax, According to the invention, the natural wax is carnauba wax.

As discussed above, in certain embodiments, the pharmaceutical composition may include a dissolution enhancer, a flow aid, a stiffening agent, and combinations thereof.

Without intending to be limited, the dissolution enhancer may generally facilitate dissolution of the high drug loading of MCT by increasing the rate of water penetration into the composition. Any suitable dissolution enhancer known in the art which provides the desired particle formation and dissolution properties may be used. By way of example, the dissolution enhancer may be selected from poly(ethylene glycol)-block-poly(propylene glycol)-block-poly(ethylene glycol) copolymer, polyethylene glycol copolymer, starch, rice starch, lecithin, polyvinylpyrrolidone (PVP), polyvinylpyrrolidone-vinyl acetate copolymer (PVP-VA), polyoxylglycerides, hydroxypropyl methylcellulose (HPMC), hypromellose acetate succinate (HPMCAS), low-substituted hydroxypropyl cellulose (L-HPC), sorbitan esters, polyethylene glycol, sorbitan fatty acid esters, and combinations thereof. In certain embodiments, the dissolution enhancer is poly(ethylene glycol)-block-poly(propylene glycol)-block-poly(ethylene glycol) copolymer. Exemplary dissolution enhancers include the POLOXAMER® 407, GELUCIRE® 50/13, etc. Any suitable amount of dissolution enhancer may be used to achieve the desired dissolution of the MCT. In certain embodiments, the dissolution enhancer is present in an amount of about 1% to about 20%, about 1% to about 15%, about 1% to about 10%, about 2% to about 20%, about 2% to about 15%, by weight of the total composition, etc.

In certain aspects, the pharmaceutical compositions of the disclosure also include a flow aid. Any suitable flow aid known in the art which provides the desired particle formation and flow properties may be used. In certain embodiments, the flow aid is a silica compound, e.g., colloidal silicon dioxide (AEROSIL®, CAB-O-SIL®), amorphous silica gel (SYLOID®, SYLYSIA®), granulated silicon dioxide (AEROPERL®), silica aerogel, magnesium alumino metasilicates (NEUILIN®), calcium silicate (FLORITE®), and ordered mesoporous silicates. The flow aide may be present in the composition in any suitable amount to provide the desired flowability. For example, in certain embodiments, the flow aide may be present in an amount of at least about 2% by weight, at least about 5% by weight, at least about 10% by weight, at least about 15% by weight, at least about 20% by weight, at least about 25% by weight, at least about 30% by weight, between about 2.0% and about 30% by weight, between about 2.0% and about 20% by weight, etc.

In certain aspects, the pharmaceutical compositions of the disclosure also include a flow aid. In certain embodiments, the flow aid is a silica compound, e.g., colloidal silicon dioxide (AEROSIL®, CAB-O-SIL®), amorphous silica gel (SYLOID®, SYLYSIA®), granulated silicon dioxide (AEROPERL®), silica aerogel, magnesium alumino metasilicates (NEUILIN®), calcium silicate (FLORITE®), and ordered mesoporous silicates. In certain embodiments, the flow aid may be amphorous silica gel, silica aerogel, magnesium alumino metasilicates, calcium silicate, ordered mesoporous silica, micronized talc, and combinations thereof. In certain embodiments, the flow aid comprises micronized talc. The flow aid may be present in the composition in any suitable amount to provide the desired flowability. For example, in certain embodiments, the flow aid may be present in an amount of at least about 2% by weight, at least about 5% by weight, etc. In certain embodiments, the flow aid may each be present in an amount ranging from about 0.1% % to 5.0% from about 0.1 wt% to about 4.0 wt%, from about 0.1 wt% to about 3.0 wt%, etc.

In certain aspects, the pharmaceutical compositions of the disclosure also include a stiffening agent. Any suitable stiffening agent known in the art which provides the desired particle formation may be used. In certain embodiments, the stiffening agent may be carnauba wax, candelilla wax, and combinations thereof. In certain embodiments, the stiffening agent is carnauba wax. Any suitable amount of stiffening agent may be used to achieve the desired particle formation. In certain embodiments, the stiffening agent is present in an amount of about 1% to about 60%, about 5% to about 60%, about 10% to about 60%, about 20% to about 60%, by weight of the total composition, etc.

In certain embodiments, the pharmaceutical compositions may comprise a high drug loading of at least one MCT such as caprylic triglyceride, and at least one matrix forming agent. The composition may form discrete particles including the high drug loading of an MCT and the matrix forming agent. In certain embodiments, the matrix forming agent may be selected from glycerides, natural waxes, and combinations thereof. The glyceride may be selected from glyceryl behenate, glyceral dibehenate, glyceryl trimyristate, glyceryl trilaurate, glyceryl tristearate, glyceryl monostearate, glyceryl palmitostearate, and glyceryl triacetate, and combinations thereof. The natural wax may include standard natural waxes and hydrogenated oils, and may be selected from hydrogenated castor oil (HCO), carnauba wax, rice bran wax, and combinations thereof. In certain embodiments, the particles have an average diameter of, e.g., between about 50 μm and about 350 μm. In other embodiments, the particles exhibit a Carr Index of flowability of less than 10%. The composition may further include a dissolution enhancer, a flow aid, a stiffening agent, and combinations thereof.

Any suitable method for making the pharmaceutical compositions of the disclosure may be used. In certain aspects of the disclosure, it has been found that reproducibility, particle size and morphology, and yield may be controlled by modifying manufacturing process as illustrated in the examples herein. Methods for making the pharmaceutical compositions do not form part of the invention.

In certain aspects, the disclosure relates to the claimed compositions for use in methods of treating a disease or disorder associated with reduced cognitive function in a subject in need thereof, the method comprising administering to the subject a pharmaceutical composition of the disclosure in an amount effective to elevate ketone body concentrations in said subject to thereby treat said disease or disorder. In certain embodiments, the pharmaceutical composition of the disclosure may be administered outside of the context of a ketogenic diet. For instance, in the context of the present disclosure, carbohydrates may be consumed at the same time as pharmaceutical compositions disclosed herein.

In accordance with certain aspects of the disclosure, diseases and disorders associated with reduced cognitive function include h Age-Associated Memory Impairment (AAMI), Alzheimer's Disease (AD), Parkinson's Disease, Friedreich's Ataxia (FRDA), GLUT1-deficient Epilepsy, Leprechaunism, and Rabson-Mendenhall Syndrome, Coronary Arterial Bypass Graft (CABG) dementia, anesthesia-induced memory loss, Huntington's Disease, and many others.

In another embodiment, the patient has or is at risk of developing disease-related reduced cognitive function caused by reduced neuronal metabolism, for example, reduced cognitive function associated with Alzheimer's Disease (AD), Parkinson's Disease, Friedreich's Ataxia (FRDA), GLUT1-deficient Epilepsy, Leprechaunism, and Rabson-Mendenhall Syndrome, Coronary Arterial Bypass Graft (CABG) dementia, anesthesia-induced memory loss, Huntington's Disease, and many others.

As used herein, reduced neuronal metabolism refers to all possible mechanisms that could lead to a reduction in neuronal metabolism. Such mechanisms include, but are not limited to mitochondrial dysfunction, free radical attack, generation of reactive oxygen species (ROS), ROS-induced neuronal apoptosis, defective glucose transport or glycolysis, imbalance in membrane ionic potential, dysfunction in calcium flux, and the like.

According to the present disclosure, high blood ketone levels will provide an energy source for brain cells that have compromised glucose metabolism, leading to improved performance in cognitive function. As used herein, "subject" and "patient" are used interchangeably, and refer to any mammal, including humans that may benefit from treatment of disease and conditions associated with or resulting from reduced neuronal metabolism.

"Effective amount" refers to an amount of a compound, material, or pharmaceutical composition, as described herein that is effective to achieve a particular biological result. Effectiveness for treatment of the aforementioned conditions may be assessed by improved results from at least one neuropsychological test. These neuropsychological tests are known in the art and include Clinical Global Impression of Change (CGIC), Rey Auditory Verbal Learning Test (RAVLT), First-Last Names Association Test (FLN), Telephone Dialing Test (TDT), Memory Assessment Clinics Self-Rating Scale (MAC-S), Symbol Digit Coding (SDC), SDC Delayed Recall Task (DRT), Divided Attention Test (DAT), Visual Sequence Comparison (VSC), DAT Dual Task (DAT Dual), Mini-Mental State Examination (MMSE), and Geriatric Depression Scale (GDS), among others.

The term "cognitive function" refers to the special, normal, or proper physiologic activity of the brain, including, without limitation, at least one of the following: mental stability, memory/recall abilities, problem solving abilities, reasoning abilities, thinking abilities, judging abilities, capacity for learning, perception, intuition, attention, and awareness. "Enhanced cognitive function" or "improved cognitive function" refers to any improvement in the special, normal, or proper physiologic activity of the brain, including, without limitation, at least one of the following: mental stability, memory/recall abilities, problem solving abilities, reasoning abilities, thinking abilities, judging abilities, capacity for learning, perception, intuition, attention, and awareness, as measured by any means suitable in the art. "Reduced cognitive function" or "impaired cognitive function" refers to any decline in the special, normal, or proper physiologic activity of the brain.

In another embodiment, the methods of the present disclosure further comprise determination of the patients' genotype or particular alleles. In one embodiment, the patient's alleles of the apolipoprotein E gene are determined. It has been found that non-E4 carriers performed better than those with the E4 allele when elevated ketone body levels were induced with MCT. Also, those with the E4 allele had higher fasting ketone body levels and the levels continued to rise at the two hour time interval. Therefore, E4 carriers may require higher ketone levels or agents that increase the ability to use the

ketone bodies that are present.

In one embodiment, the pharmaceutical compositions of the disclosure are administered orally. Therapeutically effective amounts of the therapeutic agents can be any amount or dose sufficient to bring about the desired effect and depend, in part, on the severity and stage of the condition, the size and condition of the patient, as well as other factors readily known to those skilled in the art. The dosages can be given as a single dose, or as several doses, for example, divided over the course of several weeks, as discussed elsewhere herein.

The pharmaceutical compositions of the disclosure, in one embodiment, are administered in a dosage required to increase blood ketone bodies to a level required to treat and/or prevent the occurrence of any disease- or age-associated cognitive decline, such as AD, AAMI, and the like. Appropriate dosages may be determined by one of skill in the art.

In one embodiment, oral administration of a pharmaceutical composition of the disclosure results in hyperketonemia. Hyperketonemia, in one embodiment, results in ketone bodies being utilized for energy in the brain even in the presence of glucose. Additionally, hyperketonemia results in a substantial (39%) increase in cerebral blood flow (Hasselbalch, S.G., et al., Changes in cerebral blood flow and carbohydrate metabolism during acute hyperketonemia, Am J Physiol, 1996, 270:E746-51). Hyperketonemia has been reported to reduce cognitive dysfunction associated with systemic hypoglycemia in normal humans (Veneman, T., et al., Effect of hyperketonemia and hyperlacticacidemia on symptoms, cognitive dysfunction, and counterregulatory hormone responses during hypoglycemia in normal humans, Diabetes, 1994, 43:1311-7). Please note that systemic hypoglycemia is distinct from the local defects in glucose metabolism that occur in any disease- or age-associated cognitive decline, such as AD, AAMI, and the like.

Administration can be on an as-needed or as-desired basis, for example, once-monthly, once-weekly, daily, or more than once daily. Similarly, administration can be every other day, week, or month, every third day, week, or month, every fourth day, week, or month, and the like. Administration can be multiple times per day. When utilized as a supplement to ordinary dietetic requirements, the composition may be administered directly to the patient or otherwise contacted with or admixed with daily feed or food.

The pharmaceutical compositions provided herein are, in one embodiment, intended for "long term" consumption, sometimes referred to herein as for 'extended' periods. "Long term" administration as used herein generally refers to periods in excess of one month. Periods of longer than two, three, or four months comprise one embodiment of the instant disclosure. Also included are embodiments comprising more extended periods that include longer than 5, 6, 7, 8, 9, or 10 months. Periods in excess of 11 months or 1 year are also included. Longer terms use extending over 1, 2, 3 or more years are also contemplated herein. "Regular basis" as used herein refers to at least weekly, dosing with or consumption of the compositions. More frequent dosing or consumption, such as twice or thrice weekly are included. Also included are regimens that comprise at least once daily consumption. The skilled artisan will appreciate that the blood level of ketone bodies, or a specific ketone body, achieved may be a valuable measure of dosing frequency. Any frequency, regardless of whether expressly exemplified herein, that allows maintenance of a blood level of the measured compound within acceptable ranges can be considered useful herein. The skilled artisan will appreciate that dosing frequency will be a function of the composition that is being consumed or administered, and some compositions may require more or less frequent administration to maintain a desired blood level of the measured compound (e.g., a ketone body).

Administration can be carried out on a regular basis, for example, as part of a treatment regimen in the patient. A treatment regimen may comprise causing the regular ingestion by the patient of a pharmaceutical composition of the disclosure in an amount effective to enhance cognitive function, memory, and behavior in the patient. Regular ingestion can be once a day, or two, three, four, or more times per day, on a daily or weekly basis. Similarly, regular administration can be every other day or week, every third day or week, every fourth day or week, every fifth day or week, or every sixth day or week, and in such a regimen, administration can be multiple times per day. The goal of regular administration is to provide the patient with optimal dose of a pharmaceutical composition of the disclosure, as exemplified herein.

Dosages of the inventive compositions, such as, for example, those comprising MCT, may be administered in an effective in an effective amount to increase the cognitive ability of patients afflicted with diseases of reduced neuronal metabolism, such as in patients with any disease- or age-associated cognitive decline, such as, AD, AAMI, and the like.

In one embodiment, the inventive compositions result in elevating ketone concentrations in the body, and in this embodiment, the compositions are administered in an amount that is effective to induce hyperketonemia. In one embodiment, hyperketonemia results in ketone bodies being utilized for energy in the brain.

In one embodiment, the composition increases the circulating concentration of at least one type of ketone body in the mammal or patient. In one embodiment, the circulating ketone body is D-beta-hydroxybutyrate. The amount of circulating ketone body can be measured at a number of times post administration, and in one embodiment, is measured at a time predicted to be near the peak concentration in the blood, but can also be measured before or after the predicted peak blood concentration level. Measured amounts at these off-peak times are then optionally adjusted to reflect the predicted level at the predicted peak time. In one embodiment, the predicted peak time is at about two hours. Peak circulating blood level and timing can vary depending on factors known to those of skill in the art, including individual digestive rates, co-ingestion or pre- or post-ingestion of foods, drinks, etc., as known to one of skill in the art. In one embodiment, the peak blood level reached of D-beta-hydroxybutyrate is between about 0.05 millimolar (mM) to about 50 mM. Another way to

determine whether blood levels of D-beta-hydroxybutyrate are raised to about 0.05 to about 50 mM is by measurement of D-beta-hydroxybutyrate urinary excretion a range in the range of about 5 mg/dL to about 160 mg/dL. In other embodiments, the peak blood level is raised to about 0.1 to about 50 mM, from about 0.1 to about 20 mM, from about 0.1 to about 10 mM, to about 0.1 to about 5 mM, more preferably raised to about 0.15 to about 2 mM, from about 0.15 to about 0.3 mM, and from about 0.2 to about 5 mM, although variations will necessarily occur depending on the formulation and host, for example, as discussed above. In other embodiments, the peak blood level reached of D-beta-hydroxybutyrate will be at least about 0.05 mM, at least about 0.1 mM, at least about 0.15 mM, at least about 0.2 mM, at least about 0.5 mM, at least about 1 mM, at least about 1.5 mM, at least about 2 mM, at least about 2.5 mM, at least about 3 mM, at least about 4 mM, at least about 5 mM, at least about 10 mM, at least about 15 mM, at least about 20 mM, at least about 30 mM, at least about 40 mM, and at least about 50 mM.

Effective amount of dosages of compounds for the inventive compositions, i.e., compounds capable of elevating ketone body concentrations in an amount effective for the treatment of or prevention of loss of cognitive function caused by reduced neuronal metabolism will be apparent to those skilled in the art. As discussed herein above, such effective amounts can be determined in light of disclosed blood ketone levels. Where the compound capable of elevating ketone body concentrations is MCT, the MCT dose, in one embodiment, is in the range of about 0.05 g/kg/day to about 10 g/kg/day of MCT. In other embodiments, the dose will be in the range of about 0.25 g/kg/day to about 5 g/kg/day of MCT. In other embodiments, the dose will be in the range of about 0.5 g/kg/day to about 2 g/kg/day of MCT. In other embodiments, the dose will be in the range of about 0.1 g/kg/day to about 2 g/kg/day. In other embodiments, the dose of MCT is at least about 0.05 g/kg/day, at least about 0.1 g/kg/day, at least about 0.15 g/kg/day, at least about 0.2 g/kg/day, at least about 0.5 g/kg/day, at least about 1 g/kg/day, at least about 1.5 g/kg/day, at least about 2 g/kg/day, at least about 2.5 g/kg/day, at least about 3 g/kg/day, at least about 4 g/kg/day, at least about 5 g/kg/day, at least about 10 g/kg/day, at least about 15 g/kg/day, at least about 20 g/kg/day, at least about 30 g/kg/day, at least about 40 g/kg/day, and at least about 50 g/kg/day.

Convenient unit dosage containers and/or formulations include tablets, capsules, lozenges, troches, hard candies, nutritional bars, nutritional drinks, metered sprays, creams, and suppositories, among others. The compositions may be combined with a pharmaceutically acceptable excipient such as gelatin, oil(s), and/or other pharmaceutically active agent(s). For example, the compositions may be advantageously combined and/or used in combination with other therapeutic or prophylactic agents, different from the subject compounds. In many instances, administration in conjunction with the subject compositions enhances the efficacy of such agents. For example, the compounds may be advantageously used in conjunction with antioxidants, compounds that enhance the efficiency of glucose utilization, and mixtures thereof.

The daily dose of MCT can also be measured in terms of grams of MCT per kg of body weight (BW) of the mammal. The daily dose of MCT can range from about 0.01 g/kg to about 10.0 g/kg BW of the mammal. Preferably, the daily dose of MCT is from about 0.1 g/kg to about 5 g/kg BW of the mammal. More preferably, the daily dose of MCT is from about 0.2 g/kg to about 3 g/kg of the mammal. Still more preferably, the daily dose of MCT is from about 0.5 g/kg to about 2 g/kg of the mammal.

In some embodiments, the inventive compounds may be co administered with carbohydrate, or be co-formulated with carbohydrate. Carbohydrate can include more than one type of carbohydrate. Appropriate carbohydrates are known in the art, and include simple sugars, such as glucose, fructose, sucrose, and the like, from conventional sources such as corn syrup, sugar beet, and the like. If co-formulated, the amount of carbohydrate to use can include at least about 0.1 g, at least about 1g, at least about 10 g, at least about 50 g, at least about 100 g, at least about 150 g, at least about 200 g, at least about 250 g, at least about 300 g, at least about 400 g. Amounts of carnitine can be at least about 1 g, at least about 50 g, at least about 100 g. The compositions can comprise from about 15% to about 40% carbohydrate, on a dry weight basis. Sources of such carbohydrates include grains or cereals such as rice, corn, sorghum, alfalfa, barley, soybeans, canola, oats, wheat, or mixtures thereof. The compositions also optionally comprise other components that comprise carbohydrates such as dried whey and other dairy products or by-products.

## EXAMPLES

### EXAMPLE 1 - Pharmaceutical Formulations- LMP-1, LMP-2, LMP-3, LMP-4

Exemplary and comparative pharmaceutical formulations were prepared, as described herein and reflected in Table 1-A below.

**Table 1-A**

| Formulations | | | | |
|---|---|---|---|---|
| Batch | Component | Function | Melting Point (°C) | Caprylic Acid (wt%) |
| LMP-2 (exemplary) | Compritol 888 ATO (glyceryl dibehenate) | Matrix | 65 - 77 | 50 |
| **LMP-1** (comparative) | Microcrystaline Wax (193/198) | Matrix | 54 - 102 | 50 |
| LMP-3 (comparative) | Kolliphor CSL (C16 and C18 fatty alcohols, and anionic emulsifiers) | Emulsifying wax + surfactants | 49 - 54 | 50 |
| **LMP-4** (comparative) | Dynasan 118 (glyceryl tristearate) | Digestable | 70 - 73 | 50 |

The formulations were made using a atomizer spinning disk processing unit equipped with a heat-jacketed melt tank. The tank was stirred using a Silverson high shear mixer outfitted with a general purpose 1.25-inch diameter working head. The atomizer was a 4-inch spinning disk driven by a variable speed motor. Product was contained and collected in an inflated 8-foot diameter disposable bag.

The excipients were placed into the heat-jacketed melt tank along with the caprylic acid (Miglyol 808) oil and allowed to melt. When the components were mostly melted the high shear mixing head was lowered into the melt. The rotor was turned on for approximately one hour at 900 - 1000 rpm to allow for uniform dispersion of the active within the melt. The mixer was stopped and a pressure lid is placed onto the tank. Melt was delivered to the spinning disk atomizer by the pressure head alone. With this set-up, the rate that melt was delivered to the spinning disk atomizer was dependent on the melt viscosity and the pressure on the tank. The spinning disk atomizer was made of nylon and was heated to temperature within seconds as the melt flows over it. The melt flowing off the edge of the spinning disk atomizer formed small spherical droplets which congealed and hardened before striking the containment bag. The resulting particle size distribution was influenced by the melt viscosity and flow rate to the disk, but primarily by the rotation rate of the spinning disk atomizer.

The four formulations were manufactured on the spinning disk apparatus described above. Table 1-B shows the manufacturing summary of all runs. All four formulations were processed on the spinning disk apparatus using a 4" nylon atomizer and a pressurized tank with head pressure as the feed system. LMP-1 producing the hardest particles and LMP-4 the softest.

| MSC Manufacturing Summary | | | | |
|---|---|---|---|---|
| Formulation No. | LMP-1 | LMP-2 | LMP-3 | LMP-4 |
| Mfg Ref BREC-1690 | -028 | -030 | -032 | -034 |
| Mfg Date | 20Jun2016 | 20Jun2016 | 22Jun2016 | 22Jun2016 |
| Composition | 50.0/50.0 Miglyol 808/ Microcrystalline wax | 50.0/50.0 Miglyol 808/ Compritol 888 ATO | 50.0/50.0 Miglyol 808/ Kolliphor CSL | 50.0/50.0 Miglyol 808/ Dynasan 118 |
| Atomizer | 4" unheated nylon disk | | | |
| Pump System | Head Pressure | | | |
| Bach Size | 700 g | | | |
| Jacket Temp | 115°C | 70°C | 50°C | 65°C |
| Bag Temp | ~25°C | | | |
| Product Collected | 335 g | 460 g | 208 g | 27 g |
| Yield | 48% | 66% | 30% | 4% |
| Disk Speed | 2000 rpm | 1750 rpm | 1750 rpm | 1750 rpm |
| Tank pressure | ~ 5 psig | ~ 3 psig | ~5 - 5.5 psig | ~ 4 - 5 psig |
| Run time (mm:ss) | 1:30 | 6:00 | 6:00 | 3:25 |
| Spray rate | 467 g/min | 117 g/min | 117 g/min | 200 g/min |

**Table 1-B**

With the exception of LMP-4, sufficient material was collected for full characterization of the formulations. For LMP-4, the powder handling properties were poor. The particles appear cohesive and soft, and in some cases left what appeared to be oil residue on surfaces of equipment and packaging.

**EXAMPLE 2 - Characterization of Formulations LMP-1, LMP-2, LMP-3, and LMP-4**

The formulations described in Table 1-A were characterized for particle size and morphology, density and flow, melting and congealing characteristics, melting point, and potency and purity.

Light microscopy was used to assess particle shape and morphology. FIG. 1A (LMP-1), FIG. 1B (LMP-2), FIG. 1C (LMP-3), and FIG. 1D (LMP-4) show the images. All lots produced spherical particles with very few non-spherical defects. In general, all of the manufactured particles ranged from ~50 to 300μm in diameter. Some variation in particle size was observed between formulations. LMP-1 was significantly larger than LMP-2. Particle size can be controlled by adjusting disk speed. LMPs 3 and 4 appear agglomerated.

SEM imaging was used to assess particle size, shape, and morphology. FIGS. 2A-2D, FIGS. 3A-3D, and FIGS. 4A-4D (LMP-1 in panel A, LMP-2 in panel B, LMP-3 in panel C, and LMP-4 in panel D) show the SEM images of the particles. All lots produced spherical particles with diameters ranging from 50 - 350 μm in diameter. Size and shape are consistent with the optical microscopy. The surface morphology of each LMP formulation can be clearly seen in FIGS. 4A-4D at 1000 times magnification.

The LMP-1 particles - with the microcrystalline wax matrix - have a relatively smooth surface with several small circular bumps. The Compritol based LMP-2 has a very rough surface but lack the characteristic surface morphology of Compritol. Compritol typically exhibits a smooth surface with hexagonal surface crystals. The difference in morphology suggests the caprylic acid is altering the crystal structure of Compritol. The surface of LMP-4 is very atypical. The surface appears phase separated. One phase may be predominantly liquid oil explaining the visual observation of an oily surface and the agglomeration.

The true density of selected LMPs was measure using an AccuPyc 1330 Pycnometer. See Table 2-A. The true density of the microcrystalline wax LMP (LMP-1) was less than one, potentially leading to issues during dissolution if the particles float in water. The Compritol 888 (glyceryl dibehenate) LMP (LMP-2) was slightly greater than one. LMP-1 floated and LMP-2 partially sank when suspended in a 0.25% SLS / water solution consistent with the AccuPyc true density data.

| Initial LMP Formulation Runs | | | | |
|---|---|---|---|---|
| Accupyc results | | | Bare cores | Cores with 2% talc |
| Batch | Component | Lot No: BREC- | True Density | True Density |
| LMP-1 | Microcrystaline Wax (193/198) | -028 | 0.9485 | 0.9363 |
| LMP-2 | Compritol 888 ATO | -030 | Not tested | 1.0127 |
| LMP-3 | Kolliphor CSL | -032 | Not tested | Not tested |
| LMP-4 | Dynasan 118 | -034 | Not tested | Not tested |

**Table 2-A**

Thermal characteristics of the LMPs, excipients, and active caprylic acid (Miglyol 808) oil were tested by Differential Scanning Calorimetry (DSC) to further understand the melting and congealing characteristics. Thermal characteristics of caprylic triglyceride (Miglyol 808) were analyzed by using a range of ramp rates for both the congealing of the oil and the subsequent thermal and melting events. Ramp rates of 10, 5, 2, 1, and 0.2°C/min were used because the congealing temperature is often dependent on cooling rate. As shown in FIG. 5A, the congealing temperature of caprylic triglyceride (Miglyol 808) is highly dependent on the cooling rate. When cooled at 10°C/min (near the max rate for the DSC instrument), a single exotherm was observed occurring at -60.5°C, with a change in enthalpy of 28.4 J/g. As the ramp rate decreased, the congealing onset temperature increased and the congealing profile became more complicated, likely due to form changes.

As seen in FIGs. 5B and 5C, during the heating cycle a variety of exotherms were observed, indicating form changes of the solid. For all ramp rates, the solid melts at 10 - 11°C indicating that by the melting point of caprylic triglyceride (Miglyol 808), all physical forms have transformed to the same polymorph prior to the melting of caprylic triglyceride (Miglyol 808). The data shows that cooling rates during manufacture of LMPs containing caprylic triglyceride (Miglyol 808) may impact the physical form of the congealed solid. LMP samples and their respective excipients were analyzed by DSC on a TA Q2000 DSC using two methods: 1) Heat-Cool and, 2) Cool-Heat. 30μL PE hermetic pans and lids, hermetically sealed at ambient conditions were used. The sample mass range was 5 - 15 mg. The Heat-Cool and Cool-Heat methods are listed in Table 2-B.

**Table 2-B**

| Heat-Cool Method | Cool-Heat method |
|---|---|
| 1: Data storage: Off | 1: Data storage: Off |
| 2: Equilibrate at 20.00°C | 2: Equilibrate at 20.00°C |
| 3: Isothermal for 3.00 min | 3: Isothermal for 3.00 min |
| 4: Data storage: On | 4: Data storage: On |
| 5: Ramp 10.00°C/min to 100.00°C (or 110.00°C) | 5: Ramp 10.00°C/min to -60.00°C (or -70.00°C) |
| 6: Data storage: Off | 6: Data storage: Off |
| 7: Mark end of cycle 1 | 7: Mark end of cycle 1 |
| 8: Equilibrate at 100.00°C (or 110.00°C) | 8: Equilibrate at -60.00°C (or - 70.00°C) |
| 9: Isothermal for 3.00 min | 9: Isothermal for 3.00 min |
| 10: Data storage: On | 10: Data storage: On |
| 11: Ramp 10.00°C/min to -60.00°C (or -70.00°C) | 11: Ramp 10.00°C/min to 160.00°C |
| 12: Data storage: Off | 12: Data storage: Off |
| 13: End of method | 13: End of method |

The primary goal for the Heat-Cool method was to measure the melting point and respective congealing point of the matrix. The primary goal for the Cool-Heat method was to detect any congealing of free caprylic triglyceride (Miglyol 808) oil in the solid LMP matrix - suggesting phase separated oil - and to identify exotherms before the melting point of caprylic triglyceride (Miglyol 808) - indicating form changes. Also, the melting point of the caprylic triglyceride (Miglyol 808) oil and the excipient were compared vs. the pure components of each.
Comparing the three LMP Formulations it is clear that LMPs 1 and 2 have more desirable characteristics than LMP-3 due to the low temperature onset for the melting point of 33°C and congealing onset of 44°C (see Table 2-C). These low temperatures would make processing a challenge and limit the storage and use of the LMPs in their final form. Ultimately, this insight eliminates the use of Kolliphor CSL from consideration as a major excipient component for LMPs containing caprylic triglyceride. LMPs 1 and 2 show promise, from a thermal perspective, for formulating caprylic triglyceride into stable multiparticulate drug formulation as the onset of melting and congealing of the sample matrix is sufficiently high to process into a stable formulation at room temperature.

**Table 2-C**

| | | **Melt** | | |
|---|---|---|---|---|
| | | onset, °C | max, °C | enthalpy, J/g |
| Microcrystalline Wax | Heat-Cool | 49.22 | 63.83 | 158.30 |
| | Cool-Heat | 49.16 | 66.02 | 157.30 |
| Compritol 888 | Heat-Cool | 70.55 | 72.38 | 128.30 |
| | Cool-Heat | 70.52 | 72.17 | 133.80 |
| Kolliphor CSL | Heat-Cool | 51.09 | 57.02 | 137.30 |
| | Cool-Heat | 48.97 | 57.31 | 193.30 |
| Carnauba Wax | Heat-Cool | 74.58 | 82.95 | 172.93 |
| | Cool-Heat | 73.83 | 83.19 | 178.20 |
| Miglyol 808 | Cool-Heat | 9.15 | 11.65 | 113.90 |

The thermal data shows that a majority of the caprylic triglyceride is phase separated from the excipient material. If a completely miscible system were present, only one melting point would be expected. This is far from the case, as all three LMPs exhibit a melting point for caprylic triglyceride at 10°C and a subsequent melt of the excipient. As all three LPMs are 50% caprylic triglyceride, the maximum expected enthalpy of the melting event would be ~57 J/g. The enthalpy for the caprylic triglyceride melt is 50, 50, and 61 J/g for LMPs-1, 2, and 3, respectively (see Tables 2-D and 2-E) suggesting that >85% of the caprylic triglyceride is phase separated in the LMP.

**Tables 2-D & E**

| Heat-Cool | | | | |
|---|---|---|---|---|
| | | LM-P-1 | LM-P-2 | LM-P-3 |
| **Melt** | onset, °C | 43.5 ± 0.1 | 61 ± 0 | 32.9 ± 0.1 |
| | max, °C | 58.4 ± 0.1 | 68.3 ± 0.2 | 39.4 ± 0.1 |
| | enthalpy, J/g | 83 ± 0.5 | 90.6 ± 0.8 | 63.4 ± 0.5 |
| **Congeal** | onset, °C | 83.8 ± 0.4 | 62.1 ± 0 | 44.2 ± 0.1 |
| | max, °C | 82.5 ± 0.1 | 61 ± 0.2 | 42.2 ± 1 |
| | enthalpy, J/g | 85.2 ± 2.4 | 74.5 ± 0.6 | 76.5 ± 1.4 |
| **Low Temp Congeal** | onset, °C | NA | -37.5 ± 0.3 | -36.5 ± 0.3 |
| | max, °C | NA | -41.7 ± 0.1 | -41 ± 0.1 |
| | enthalpy, J/g | NA | 12.8 ± 1.9 | 25.3 ± 0.4 |

| Cool-Heat | | | | |
|---|---|---|---|---|
| | | LM-P-1 | LM-P-2 | LMP-3 |
| **Exotherm, cooling** | onset, °C | NA | -12 ± 0.3 | -35.6 ± 0.1 |
| | max, °C | NA | -13.5 ± 0.3 | -41 ± 0.2 |
| | enthalpy, J/g | NA | 45 ± 0.1 | 26.7 ± 2.2 |
| **Exotherm 1, heating** | onset, °C | -52.1 ± 0.1 | NA | -15.7 ± 0.1 |
| | max, °C | -49.6 ± 0.2 | NA | -11.7 ± 0 |
| | enthalpy, J/g | 13.5 ± 0.7 | NA | 10.6 ± 0.3 |
| **Exotherm 2, heating** | onset, °C | -14 ± 0.2 | NA | NA |
| | max, °C | -10.7 ± 0.2 | NA | NA |
| | enthalpy, J/g | 13.3 ± 0.3 | NA | NA |
| **Melt 1 (CT)** | onset, °C | 6 ± 0.1 | 5.9 ± 0.1 | 7.5 ± 0 |
| | max, °C | 10.4 ± 0.2 | 9.9 ± 0.1 | 10.3 ± 0 |
| | enthalpy, J/g | 49.5 ± 0.5 | 50.3 ± 0.5 | 61.4 ± 1.2 |
| **Melt 2 (Excipient)** | onset, °C | 44.8 ± 1.1 | 61.8 ± 0.4 | 33.8 ± 1.6 |
| | max, °C | 60.2 ± 1.3 | 68.7 ± 0.3 | 39.2 ± 0.2 |
| | enthalpy, J/g | 82 ± 0.2 | 93.4 ± 1.6 | 63.6 ± 5.6 |

Visual melting point data was collected using an automated melting point apparatus (SRS Optimelt) at 2°C/min. Because of the wide thermal events by DSC, the insight gained by a visual assessment of the LMPs while heating allow for a greater degree of discrimination between the formulations that may be missed by calorimetry data alone.

Melting profiles, as analyzed by light intensity (FIG. 6), show that LMP-2 has the sharpest melting point profile of the three LMPs, with a melting point onset of 62°C with melting endpoint at 75°C. This observation is in contrast to the observation that when placed between gloved fingers, the LMPs appeared to melt. In light of this data, without wishing to bound by any one theory, the LMP-2 Compritol matrix may be compromised by the high oil content and physically breaks upon pressure from rubbing fingers together. LMP-1 starts to become translucent at 43°C, however no shrinking of the material is observed until 64°C. LMP-3 starts to melt immediately from the starting point of 40°C. Once melted the material is still opaque and does not become completely clear until 140°C, likely due to the SLS from the excipient (Kolliphor CSL) not becoming completely soluble in the melt until that point.

Potency and purity of the LMP formulations was analyzed by HPLC-CAD. Briefly, the samples were prepared by: weighing 10mgA powder into 20mL scintillation vial; adding 10mL LMP diluent; 50/50 ACN/IPA; sonicating 10 minutes; equilibrating to room temperature (RT); loading 5mL into BD syringe fitted with 0.2μm 25mm nylon filter; waste 4mL, collecting 5th mL into HPLC vial. The chromatography conditions are as described in Table 2-F.

**Table 2-F**

| | | | |
|---|---|---|---|
| System | Agilent 1100 or 1200 | | |
| Column | Agilent Poroshell 120 EC-C18, 3.0 × 150mm, 2.7μm (P/N: 693975-302) | | |
| Mobile Phase | A) 90/10 ACN/H2O B) 40/60 ACN/IPA | | |
| Gradient Program | **Minutes** | **%A** | **%B** |
| | **0** | **92** | **8** |
| | **16** | **0** | **100** |
| | **24** | **0** | **100** |
| | **24.1** | **92** | **8** |
| | **33** | **92** | **8** |
| Run Time | 33 minutes | | |
| Diluent | IPA or 9:1 IPA:H2O (water needed to reconstitute powder emulsifying samples, i.e. AC-1204 formulation and NADDs) | | |
| Injection volume | 2.5μL (needle wash: IPA) | | |
| Detection | CAD detection (Nebulizer Temperature: 35°C, Power Function: 1.30, Filter = 4, Range: 100pA) | | |
| Column Temp | 10.0°C | | |
| Flow | 0.60mL/min | | |
| Concentration | 1mg/mL (2.5μg on column) | | |

There was no significant change in impurities through RRT = 1.32, except for LMP-2, which has a peak at RRT = 1.11 of 0.27%. All peaks RRT = 1.36 to 2.55 are likely peaks due to excipients that were not extracted from the pure excipient alone. Without being bound to any particular theory, it is believed that the caprylic triglyceride (Miglyol 808) may be acting as a co-solvent for the excipient, aiding in extraction of additional components from the matrix compared to the pure component alone for the control.

All three formulations have assay values near 100% theoretical with values ranging from 97.2 - 99.6% label claim (LC). Table 2-G contains the tabulated peak data for caprylic triglyceride (Miglyol 808) versus the three LMP formulations. FIGs. 7A-7C show example chromatograms for the three LMPs versus the excipient and caprylic triglyceride (Miglyol 808) control. The chromatograms are zoomed and show the significant contribution of peaks due to the excipients. In addition, without being bound to any one theory, the peaks at RRT 0.31 and 0.34 may be C-8 diglycerides and as such are included as an impurity.

**Table 2-G**

| RRT | Peak ID | Miglyol 808 % | LMP-1 % | LMP-2 % | LMP-3 % |
|---|---|---|---|---|---|
| 0.31 | | 0.16% | 0.17% | 0.20% | 0.16% |
| 0.34 | | 0.10% | 0.10% | 0.14% | 0.10% |
| 0.54 | | ND | ND | 0.07% | ND |
| 0.65 | | 0.06% | 0.06% | 0.06% | 0.06% |
| 0.80 | C6:C8:C8 | <LOQ | <LOQ | <LOQ | <LOQ |
| 0.91 | | 0.06% | 0.06% | 0.06% | 0.06% |
| **1.00** | **Caprylic Triglyceride** | **98.39%** | **97.70%** | **96.97%** | **97.22%** |
| 1.10 | | 0.11% | 0.11% | 0.09% | 0.10% |
| 1.11 | | ND | ND | 0.27%* | ND |
| 1.19 | C10:C8:C8 | 0.98% | 0.98% | 0.97% | 0.95% |
| 1.30 | | 0.06% | 0.06% | ND | 0.06% |

(continued)

| RRT | Peak ID | Miglyol 808 % | LMP-1 % | LMP-2 % | LMP-3 % |
|---|---|---|---|---|---|
| 1.32 | | 0.07% | 0.07% | ND | 0.09% |
| 1.36 | | ND | ND | ND | 0.34% |
| 2.12 | | ND | 0.07% | ND | ND |
| 2.20 | | ND | 0.17% | ND | ND |
| 2.28 | | ND | 0.15% | ND | ND |
| 2.33 | | ND | ND | ND | 0.19% |
| 2.38 | | ND | 0.19% | ND | ND |
| 2.49 | | ND | 0.11% | ND | ND |
| 2.50 | | ND | ND | ND | 0.41% |
| 2.55 | | ND | ND | ND | 0.26% |
| --- | **Total Impurities** | **1.61%** | **2.30%** | **2.44%** | **2.78%** |
| | **Assay (%LC)** | **NA** | **97.2%** | **99.6%** | **98.7%** |
| LOQ>0.05%<br>*Peak corrected for area in compritol control. | | | | | |

**EXAMPLE 3 - Dissolution of LMP-1, LMP-2, LMP-3**

Visual dissolution studies were conducted at small scale using 20ml scintillation vials on LMP formulations 1 through 3. The test was conducted at 37°C, 25 °C and 5 °C using a 0.25% Sodium Laurel Sulfate (SLS) in water solution. A heated aluminum block and heated stir plate were used in the study. 500mg (250mgA, 25mgA/mL) of each LMP formulation was added to 10mL pre-heated 0.25% SLS solution. The samples were stirred using a Teflon stir bar on a heated stir plate to maintain temperature for 20 minutes. After 20 minutes, the LMP suspensions were allowed to settle and were assessed visually.

Images of the dissolution test are shown in FIG. 8. The vial containing LMP-1 remained clear after stirring for 20 minutes, suggesting caprylic triglyceride (Miglyol 808) oil did not release. Both LMP-2 and LMP-3 test vials were opaque suggesting either the oil released or the LMPs melted. The viscosity of the LMP-3 test vial increased significantly. However, this was not observed in the other formulations.

Similar results were observed when the dissolution test was performed at room temperature and 5°C. The vial containing LMP-1 was cloudier at the lower temperatures when compared to the 37 °C dissolution. However, they were much less cloudy than LMP-2 and LMP-3. LMP-2 became cloudier at 5 °C when compared to 37 °C but less cloudy at room temperature. No difference was seen between the three temperatures for LMP-3. The results indicate the cloudiness is not due to melting because the lipids are solid at room temperature and 5 °C.

Finally the test was repeated using only LMP-1 and a 0.5% SLS / water solution to access if additional surfactant would help with the apparent dissolution by increasing the solubility of the oil in water. The results are similar to the 0.25% SLS dissolution suggesting the lack of cloudiness, and thereby the dissolution, is not driven by the oil/water solubility.

An oil dissolution control test was conducted to determine how the caprylic triglyceride (Miglyol 808) oil dissolves into an SLS / water solution. Two SLS stock solutions were made at 0.25% and 0.5% SLS in water. 10 ml of each SLS solution was added to a scintillation vial along with a magnetic stir bar. 250 mg of caprylic triglyceride (Miglyol 808) oil was added to each vial and stirred for 20 minutes. Images were taken after 10 minutes and two days. The images are shown in FIG. 9. The majority of the oil floats on top of the water rather than going into solution. The vial does appear slightly hazy in the 10 minute photo indicating some oil is likely emulsified in the water phase. The haziness decreases after 2 days likely because the emulsified caprylic triglyceride (Miglyol) is separating into the oil phase.

No oil phase was observed for LMP-1 strongly suggesting the caprylic triglyceride (Miglyol 808) was not release. An oil phase was also not seen for LMP- 2 and 3, however, the oil may have been either emulsified in the aqueous phase along with the lipid carrier or not phase separated by the time the images were taken. The cloudiness in the LMP dissolution test was the result of the lipid carrier and not the oil.

LMPs 1, 2, and 3 were tested in a USP Type II sink dissolution experiment along with caprylic triglyceride (Miglyol 808) oil. 500 mgA of formulation was dosed into a 1000 mL vessel containing 900 mL of 2.0wt% SLS in pH 6.8 buffer. Samples were taken at 30 minutes and 17.5 hours. The method parameters are listed in Tables 3-A and 3-B. The results

are listed in Table 3-C.

**Table 3-A**

| | |
|---|---|
| Dissolution Apparatus | USP I/II |
| Vessels | 1L USP |
| Stirring | Standard paddles, 100 RPM |
| Temperature | 37°C |
| Media | 900mL/vessel 2.0wt% SLS in 50mM Sodium Phosphate Buffer, pH 6.8 |
| **Dose, concentration** | 500mg caprylic triglyceride, 0.556mg/mL |
| **Sampling** | 10mL through 10μm full flow filter fitted to Stainless Steel cannula w/ syringe |
| **Additional sampling** | 1) 2mL of 10μm filtrate, centrifuged at 21,000 RCF for 10 minutes. 2) 6mL of 10μm filtrate, filtered through 0.2μm nylon syringe filter (waste 4mL, collect 2mL) |
| **Dilution (10μ, 0.2μ, centrifuged)** | 1mL of filtrate or aqueous supernatant into 5mL IPA. Filter through 0.2μm nylon syringe filter into HPLC vial. |
| **Time points** | 0.5, 17.5 hours |

**Table 3-B**

| | | | |
|---|---|---|---|
| **System** | Agilent 1100 or 1200 | | |
| **Column** | Agilent Poroshell 120 EC-C18, 3.0 x 50mm, 2.7μm (P/N: 699975-302) | | |
| **Mobile Phase** | A) 90/10 ACN/H2O B) 40/60 ACN/IPA | | |
| **Gradient Program** | Minutes | %A | %B |
| | 0 | 70 | 30 |
| | 4.0 | 0 | 100 |
| | 8.2 | 0 | 100 |
| | 8.3 | 70 | 30 |
| | 12.5 | 70 | 30 |
| **Run Time** | 12.5 minutes | | |
| **Diluent** | **1:5** H2O:IPA | | |
| **Injection volume** | 4.0μL | | |
| **Detection** | CAD detection (Nebulizer Temperature: 35°C, Power Function: 1.30, Filter = 4, Range: 100pA) | | |
| **Column Temp** | 10.0°C | | |
| **Flow** | 0.60mL/min | | |
| **100% Concentration** | 0.12mg/mL (0.48μg on column) | | |

**Table 3-C**

| %LC Release | timepoint | 10μ filtered | centrifuged | 0.2u filtered |
|---|---|---|---|---|
| LMP-1 | 30 minutes | ND | ND | NA |
| | 17.5 hours | 1.6% ± 0.2% | 1.6% ± 0.1% | 1.6% ± 0.3% |
| LMP-1 (cryo-milled) | 30 minutes | 0.6% ± 0.1% | 0.8% ± 0% | 0.7% ± 0.1% |
| | 17.5 hours | 6.3% ± 0.3% | 6.5% ± 0.2% | 6.3% ± 0.4% |

(continued)

| %LC Release | timepoint | 10μ filtered | centrifuged | 0.2u filtered |
|---|---|---|---|---|
| LMP-2 | 30 minutes | 67.5% ± 0.4% | 45.3% ± 5.6% | NA |
| | 17.5 hours | 90.5% ± 0.3% | 75.4% ± 2.1% | 86% ± 0.3% |
| LMP-3 | 30 minutes | 60.6% ± 0.5% | 50.4% ± 6.7% | NA |
| | 17.5 hours | 71.4% ± 0.1% | 56.9% ± 0.5% | 87.8% ± 7.1% |
| Miglyol 808 | 30 minutes | 64% (n=1) | 31.6% ± 2% | NA |
| | 17.5 hours | 46.5% ± 0.5% | 27.3% ± 0.6% | 87% ± 7% |

It would be expected that the control sample, caprylic triglyceride (Miglyol 808), would achieve 100% release as the dose was a 3x sink the chosen media. However, this was not the case as the centrifuged sample only achieved a 30% recovery. It was observed that the oil dispersed into macroscopic droplets that were easily observed by eye. The size and number of these droplets did not appear to change overtime. Without being bound to any one theory, the oil droplets may be stabilized by the SLS in the media and that is more kinetically stable than being solubilized into the SLS micelles. The oil was dosed in a large excess (~80x) versus the solubility. The large excess of oil may have overcome the kinetic component and driven the solubility to it thermodynamic solubility for the solubility experiment.

LMP-1 has poor release of caprylic triglyceride (Miglyol 808) with no detectable peak (LOQ > 0.5%LC, approx.). After 17.5 hours all three isolation techniques resulted in a 1.6% LC release. LMP-2 exhibited the best overall performance of the three LMP formulations tested with 75 - 91 % LC release after 17.5 hours indicating the possibility for a desirable in vivo release profile.

After the initial round of dissolution testing, a portion of LMP-1 was cryo-milled using a Spex Freezer/Mill to fracture the LMPs to expose the interior of the particles, as well as increase the surface area. The dissolution increased about 4x over the intact LMPs, however, without being bound to any one theory, this may be due to only the increase in surface area. This data supports the hypothesis that caprylic triglyceride partitions into microcrystalline wax and will not release into a sink dissolution medium effectively eliminating microcrystalline wax from being a major component in an LMP formulation containing caprylic triglyceride.

**EXAMPLE** 4 - **Flow Aid**

Two flow aids were screened on LMP-1, described above in Table 1-1A, to assess the improvement in flow. Micronized talc and colloidal silicon dioxide were each dryblended at a 2wt% level with 50 gram sub-lots of LMP-1. This was done by adding the flow aid, blending in a Turbula, screening the mixture through a 600 μm screen, and finished with a final Turbula blend. Visually, the flow and handling properties were unchanged with the colloidal silicon dioxide, but significantly improved with the micronized talc. Subsequently, each of the remaining lots (LMP-2, LMP-3, LMP-4, described above in Table 1-A) were blended with 2 wt% talc. During this process, the material from LMP-4 self-agglomerated during the blending step. This suggests that the level of softness is non-recoverable. The remaining three LMP lots were further characterized with and without the flow aid.

The true density of LMP-1 and LMP-2 with 2% talc were measured as described in EXAMPLE 2. The values are in Table 2-A.

Bulk and tapped density were measured on selected LMP formulations to quantify the flowability before and after addition of 2% talc as a flow aid. The bulk and tapped volumes were used when calculating the Carr index which describes the flowability of powders. A powder with a Carr index greater than 25 typically has poor flowability while a value below 15 has a good flowability. BSV/TSV tests were performed on talced and un-talced LMP-1 and LMP-2, and talced LMP-3. LMP-4 was not tested because it agglomerated. The results are shown in Table 3-A. Addition of the 2% talc significantly improved the flowability of LMP-1 and LMP-2. The Carr Index decreased from 31 to 10 and 18 to 6 for LMP run's 1 and 2 respectively with the addition of talc. The Carr index numbers corroborate with visual assessment of flowability. The un-talced material flowed poorly while the talc LMPs flowed well. BSV/TSV was also performed on LMP-3 talced particles and resulted in a Carr Index value of 21 indicating LMP-3 did not flow as well as LMP-1 or -2.

| Initial LMP Formulation Runs | | | | | | |
|---|---|---|---|---|---|---|
| BSV/TSV results | | | Bare cores | | Cores with 2% talc | |
| Batch | Component | Lot No: BREC-1690- | Bulk Density | Carr Index | Bulk Density | Carr Index |
| LMP-1 | Microcrystaline Wax (193/198) | -028 | 0.39 | 31 | 0.52 | 10 |
| LMP-2 | Compritol 888 ATO | -030 | 0.48 | 18 | 0.54 | 6 |
| LMP-3 | Kolliphor CSL | -032 | Not tested | Not tested | 0.44 | 21 |
| LMP-4 | Dynasan 118 | -034 | Not tested | Not tested | Not tested | Not tested |

Table 4-A

EXAMPLE 5- Stiffening Agent

A stiffening agent is intended to increase the hardness and melt temperature of the formulation to improve the handleability and physical stability. Stiffening agents have high hardness and high melting temperature. The stiffening agents carnauba and candelilla wax were examined. Carnauba wax has a melting temperature of approximately 82 °C and has very high hardness. Candelilla was has a melting temperature of approximately 68 °C and is known for hardening other waxes. Carnauba and candelilla wax were chosen as stiffening agents because they have a similar structure to microcrystalline wax, but have shorter chain lengths. The similar structure is intended to improve the handling characteristics of the LMPs but avoid the poor dissolution performance of LMP-1. The formulations screened are listed in Table 5-A.

| Formulation | Miglyol 808 | Compritol 888 | Carnauba wax | Canelilla wax |
|---|---|---|---|---|
| LMP2 | 50 | 50 | -- | -- |
| LMP5 | 40 | 60 | -- | -- |
| LMP6 | 30 | 70 | -- | -- |
| LMP7 | 50 | 30 | 20 | -- |
| LMP8 | 50 | 30 | -- | 20 |
| LMP9 | 50 | 40 | 10 | -- |
| LMP10 | 45 | 55 | -- | -- |
| LMP11 | 50 | -- | 50 | -- |
| All values are based on weight percent | | | | |

Table 5-A

The melt screening was conducted. The excipients and caprylic triglyceride (Miglyol 808) oil were melted at 100 °C and mixed thoroughly. Small droplets were flicked onto a glass plate to observe the cooling rate. The congealed drops were collected and rubbed between ones fingers feeling for handleability. Finally, a small weigh boat was filled with melt and allowed to solidify. Observations were made on whether the solidified melt sweats oil. Each formulation was inspected for the following characteristics: do the excipients phase separate; how quickly do the droplets cool; does the solidified melt smear or feel oily when handled; how hard is solidified melt; and does solidified melt "sweat" oil. LMP-2 sample was included as a control to compare the formulations to.

The LMPs were rank ordered in terms of physical characteristics. The results are shown in Table 5-B. Reducing the caprylic triglyceride (Miglyol 808) loading significantly improved the handleability of the melts. The 30 % and 40 % loading formulations were much better than the 50% loading while the 45% loading was only marginally better. The 30 % and 40 % caprylic triglyceride (Miglyol) formulations were non-greasy in the fingers and were very hard. The 30 % caprylic triglyceride (Miglyol) drug loading was the best performing formulation while the 40% loading was tied for third. The candelilla wax formulation was soft and smeared easily was not carried forward. Detailed melt screen results are shown in Table 5-C.

| Ranking | Formulation | | Notes |
|---|---|---|---|
| 1 | LMP6 | 30/70 Miglyol/Compritol | LMP6 harder than LMP11 - light sweating |
| 2 | LMP11 | 50/50 Miglyol/Carnauba | Non-greasy - no sweating |
| 3 | LMP5 | 40/60 Miglyol/Compritol | LMP5 was harder than LMP7 - oil sweating |
| | LMP7 | 50/30/20 Miglyol/Compritol/Carnauba | Slightly more greasy than LMP11 - no sweating |
| 4 | -- | | |
| 5 | LMP9 | 50/40/10 Miglyol/Compritol/Carnauba | More greasy than LMP7 - no sweating |
| 6 | -- | | |
| 7 | -- | | |
| 8 | LMP8 | 50/30/20 Miglyol/Compritol/Candelilla | Very soft and greasy - no sweating |
| 9 | LMP10 | 45/55 Miglyol/Compritol | Smeared in hand - significant sweatting |
| 10 | LMP2 | 50/50 Miglyol/Compritol | Smeared in hand - significant sweatting |

**Table 5-B**

| Formulation | LMP2 (50/50) | LMP10 (45/55 | LMP5 (40/60) | LMP6 (30/70) | LMP7 (50/30/20) | LMP9 (50/40/10) | LMP8 (50/30/20) | LMP11 (50/50) |
|---|---|---|---|---|---|---|---|---|
| | Miglyol/Compritol | | | | Miglyol/Compritol/Carnauba | | Miglyol/Compritol/Candelilla | Miglyol/Carnanuba |
| Single Phase | Yes | Yes | Yes | Yes | Yes | Yes | Yes | Yes |
| | **Rank order: 2=5=6=7=8=9=10=11** | | | | | | | |
| Cooling Droplet | Good | Good | Good | Good | Good | Good | Good | Good |
| | **Rank order: 2=5=6=7=8=9=10=11** | | | | | | | |
| Melt Smearing | Yes | Yes | Not easily | No | Not easily | Not easily | Not Easily | No |
| | **Rank order: 6<11<5=7<9<8<10<2** | | | | | | | |
| Hardness | Soft | Soft | Brittle | Brittle | Brittle | Brittle | Soft | Brittle |
| | **Rank order: 6>11>5>7>9>8>10>2** | | | | | | | |
| Oil Sweating | Yes | Yes | Yes | Yes | No | No | No | No |
| | **Rank order: 11=7=9=8<6<5<10=2** | | | | | | | |

**Table 5-C**

The carnauba wax formulations had similar handleability characteristics at 50% drug loading to the 30 and 40 % caprylic triglyceride (Miglyol 808) / Compritol formulations, but were slightly softer and slightly greasier. The caprylic triglyceride (Miglyol 808) formulations were harder and had a more waxy texture. However, the carnauba wax formulations did not show oil sweating while the caprylic triglyceride (Miglyol 808) / Compritol formulations did. The caprylic triglyceride (Miglyol 808) / Compritol sweated which decreased with lower drug loading. No oil sweating was seen in formulations containing carnauba wax. Without being bound to any one theory, the clumping and poor flow of LMP-2 may have been caused by surface oil forming liquid bridges forming between particles.

A test was performed on the LMP- 5, 6, 9, and 11 to find the temperature at which the melt begins to cloud up or solidify in order to nominate spinning disk processing temperatures. The formulations were melted at 100°C and then the temperature was decreased in 5°C increments and held for a minimum of 15 minutes. The temperature range at which each melt clouded up at is shown below in Table 5-D.

| Formulation | LMP5 | LMP6 | LMP9 | LMP11 |
|---|---|---|---|---|
| Cloud Temperature | 65-70°C | 70-75°C | 65-70°C | 80-85°C |

**Table 5-D**

LMP- 5, 6, and 9 all cooled in a single phase and had a cloud temperature of 65-75 °C. Potential phase separation was seen in LMP-11. At 80-85°C, about half of the melt was solidified with the bottom half appearing to be carnauba wax rich and the top half was caprylic triglyceride (Miglyol 808) rich based on the color of the two phases. A small amount was scraped off the top after the melt was cooled to room temperature and it felt greasy and smeared easily when handled. This phase separation was not seen when creating a large melt block, which suggests the phase separation is slow and will likely not occur during spinning disk apparatus manufacture.

Based on the melt screen results, formulations with lower drug loading in Compritol are the most likely to have good dissolution performance and have improved handleability. The carnauba wax improved the physical characteristics of the melts while maintaining a 50% active loading.

**EXAMPLE** 6 - **Pharmaceutical Formulations- LMP-5, LMP-7, LMP-9, LMP-11**

Additional pharmaceutical formulations were prepared, as described herein and reflected in Table 6-A below.

| MSC Manufacturing Summary | | | | |
|---|---|---|---|---|
| Formulation No. | 5 | 7 | 9 | 11 |
| Mfg Ref | BREC1690-097 | BREC1690-098 | BREC1690-099 | BREC1690-100 |
| Mfg Date | 1-Sep-16 | | | |
| Composition | 40/60 Miglyol 808/Compritol 888 | 50/30/20 Miglyol 808/Compritol 888/Carnauba | 50/40/10 Miglyol 808/Compritol 888/Carnauba | 50/50 Miglyol 808/Carnauba |
| Atomizer | 4" heated stainless steel disc | | | |
| Pump System | 2 psi pressure head | | | |
| Batch Size | 500 grams | | | |
| Jacket Temp | 85°C | 85°C | 85°C | 95°C |
| Bag Temp | Ambient (22-24°C) | | | |
| Product Collected | 331 g | 319 g | 356 g | 318 g |
| Yield | 66% | 64% | 71% | 64% |
| Disk Speed | 1750 rpm | | | |
| Tank Pressure | 2 psi | | | |
| Gear pump speed | 63 rpm | | | |
| Run Time (min:sec) | 2:57 | 2:53 | 2:50 | n/a |
| Spray Rate (g/min) | 170 | 173 | 176 | n/a |

**Table 6-A**

The spinning disk apparatus set up was nearly identical to EXAMPLE 1, except the nylon disc was replaced with a heated stainless steel disk. The stainless steel disc was brought up to melt temperature before the start of each run, and would be used for scale up batches. The melt temperature for each formulation was roughly 10-15°C above the cloud point from Table 5-D. Excipients were melted and allowed to reach the desired melt temperature prior to adding the caprylic triglyceride (Miglyol). The oil was added and high shear mixed at 2500 rpm for about 10 minutes until melt temperature was reached.

**EXAMPLE** 7 - **Characterization** of Formulations **LMP-5, LMP-7, LMP-9, and LMP-11**

The formulations described in Table 6-A were characterized for physical characteristics, particle size and morphology, density and flow, melting point, and potency and purity.

The physical characterization was mainly done by visual and physical observations. The cores were observed in their collection bags and ranked on how well they flowed along with tendency of the LMP cores to clump. Physical stability of the cores was determined by handling and rubbing cores between ones fingers. The results of the physical observations are listed in Table 7-A.

| Formulation | Handleability | Flowability (No Talc) | Flowability (2% talc) |
|---|---|---|---|
| 50/50 Miglyol/carnauba | Very good | Med | Very Good |
| 40/60 Miglyol/Compritol | Good | Good | Very Good |
| 50/30/20 Miglyol/Compritol/carnauba | Med-poor | Poor | Good |
| 50/40/10 Miglyol/Compritol/carnauba | Poor | Poor | Good |

**Table 7-A**

Particle size distribution was evaluated using light microscopy. The LMPs were spread into a uniform layer on a glass slide and approximately 200-300 particles were measured using a calibrated particle sizing software. Selection bias was minimized by measuring every particle within the field of view. The D10, D50, D90 and D4,3 statistics were calculated using a mass weighted basis because the historical LMP size data from laser diffraction and sieve analysis is a mass/volume weighted average. The distribution statistics are shown in Table 7-B. The particle size is within the expected range with a D50 between 150 and 200 μm. The span - $\frac{D_{90}-D_{10}}{D_{50}}$ - range from around 0.6 to greater than 0.9, which is typical for the manufacturing scale.

| Formulation | D10 (μm) | D50 (μm) | D90 (μm) | D4,3 (μm) | Span |
|---|---|---|---|---|---|
| LMP5 | 110 | 149 | 205 | 155 | 0.633 |
| LMP7 | 129 | 169 | 238 | 179 | 0.641 |
| LMP9 | 136 | 183 | 300 | 201 | 0.897 |
| LMP11 | 140 | 193 | 318 | 215 | 0.925 |

**Table 7-B**

The LMPs were imaged using scanning electron microscopy (SEM) to assess particle size, shape, and morphology. FIG. 9 and FIG. 10 show representative images at different magnifications. Particle size and shape was qualitative assessed and can best be seen in FIG. 10. The particle size ranged from 50 to 350 μm in diameter, consistent with sizes measured using light microscopy. The majority of the LMPs are spherical with few small or satellite particles indicating good atomization.

FIG. 11 shows the surface morphology. The morphology is consistent with LMP- 1, 2, 3, and 4. The Compritol based LMPs, formulations LMP-5, LMP-7, and LMP-9, have a rough surface with significant surface undulations lacking any obvious pattern. Compritol alone typically exhibits a smooth surface with hexagonal surface crystals. The morphology indicates the caprylic triglyceride (Miglyol) alters the crystal structure of Compritol. The LMP-2 formulation has a similar surface morphology, however, there are more surface wrinkles on LMP-5 than seen on LMP-2. The carnauba wax based LMP-11 has a completely different morphology. Rather than a wrinkled surface, LMP-11 appears more like deflated bubbles. The morphology is more consistent with a spray layered coating rather than an spinning disk apparatus. Overall, the morphology of the LMPs is not typical. However, the morphology is consistent with that for LMP- 1, 2, 3, and 4.

The bulk and tapped density was measured and the Carr index calculated to quantify the flowability of LMPs dry blended with 2% talc as a flow aid. The results are shown in Table 7-C. The bulk density of the four formulations were similar, varying between 0.49 and 0.55 g/cm$^3$. The maximum Carr index was 10 indicating good flowability. This is in agreement with the visual observations of good or very good flowability for each formulation (see Table **7-AError! Reference source not found.).**

| Bulk and Tapped Density | | | | |
|---|---|---|---|---|
| | LMPs with 2% Talc | | | |
| | Lot BREC1690- | Bulk Density ($g/cm^3$) | Tapped Density ($g/cm^3$) | Carr Index |
| LMP5 | 097 | 0.55 | 0.59 | 7 |
| LMP7 | 098 | 0.53 | 0.58 | 9 |
| LMP9 | 099 | 0.49 | 0.55 | 10 |
| LMP11 | 100 | 0.55 | 0.58 | 6 |

Table 7-C

The formulations were characterized by DSC and compared to the thermal characteristics of the individual components, including caprylic triglyceride (Miglyol 808), and excipients Compritol 888 and carnauba wax (see Table 2-C). The LMP samples were analyzed using the Heat-Cool and Cool-Heat DSC methods described in EXAMPLE 2 and outlined in Table 2-B. Table 7-D contains the average of three replicates for each of the samples.

| Heat-Cool | | | | | |
|---|---|---|---|---|---|
| | Formulation | LMP-5 | LMP-7 | LMP-9 | LMP-11 |
| Melt | onset, °C | 58.9 ± 0.7 | 54.3 ± 0.1 | 56 ± 0.5 | 68.6 ± 0.4 |
| | max, °C | 66.9 ± 0.2 | 63.2 ± 0.1 | 64 ± 0.2 | 78.2 ± 0.1 |
| | enthalpy, J/g | 109.9 ± 3.5 | 90.3 ± 0.3 | 87.9 ± 0.6 | 93.5 ± 0 |
| Congeal | onset, °C | 64.3 ± 0.1 | 59.5 ± 0.3 | 61.3 ± 0.6 | 75.4 ± 0 |
| | max, °C | 63.1 ± 0.3 | 57.1 ± 0.6 | 57.7 ± 0.3 | 74.7 ± 0.3 |
| | enthalpy, J/g | 97.2 ± 1.4 | 80.8 ± 2.8 | 66 ± 1.2 | 78.1 ± 5.6 |
| Low Temp Congeal | onset, °C | NA | -56.4 ± 0.8 | -54.2 ± 0.1 | -59.8 ± 0 |
| | max, °C | NA | -58.9 ± 0.1 | -57.2 ± 0.1 | -61.3 ± 0 |
| | enthalpy, J/g | NA | 16.4 ± 0.4 | 18.2 ± 0.5 | 12.1 ± 0.2 |

Table 7-D

| Cool-Heat | | | | | |
|---|---|---|---|---|---|
| | Formulation | LMP-5 | LMP-7 | LMP-9 | LMP-11 |
| Exotherm, cooling | onset, °C | -12.7 ± 0.2 | -20.8 ± 0.5 | -17.6 ± 0.1 | -61.2 ± 0.6 |
| | max, °C | -14.8 ± 0.1 | -38.1 ± 0.2 | -25.1 ± 0 | -63.4 ± 0.9 |
| | enthalpy, J/g | 34.2 ± 0.1 | 29.8 ± 1.5 | 24.7 ± 1.7 | 9.6 ± 0.8 |
| Exotherm 1, heating | onset, °C | NA | NA | NA | -52.4 ± 0.2 |
| | max, °C | NA | NA | -8.3 ± 0.2 | -50.1 ± 0.1 |
| | enthalpy, J/g | NA | NA | NA | 2.9 ± 0.1 |
| Endotherm, heating | onset, °C | NA | NA | NA | NA |
| | max, °C | NA | -10.8 ± 0.3 | NA | -11.5 ± 1.4 |
| | enthalpy, J/g | NA | NA | NA | NA |
| Exotherm 2, heating | onset, °C | NA | NA | NA | NA |
| | max, °C | NA | -7.2 ± 0.7 | NA | -6.7 ± 2 |
| | enthalpy, J/g | NA | NA | NA | NA |

(continued)

| Cool-Heat | | | | | |
|---|---|---|---|---|---|
| | Formulation | LMP-5 | LMP-7 | LMP-9 | LMP-11 |
| **Melt 1 (Caprlyic Triglyceride)** | onset, °C | 4.9 ± 0 | 5.8 ± 0 | 5.8 ± 0 | 6.7 ± 0.5 |
| | max, °C | 9.3 ± 0.1 | 9.3 ± 0.1 | 9.4 ± 0 | 10.1 ± 0.5 |
| | enthalpy, J/g | 38.7 ± 0.1 | 40 ± 1 | 44.1 ± 0.4 | 24.7 ± 10.5 |
| **Melt 2 (Excipient)** | onset, °C | 59.4 ± 0.1 | 54.8 ± 0 | 57.8 ± 0 | 68.6 ± 0.8 |
| | max, °C | 67 ± 0.1 | 63.6 ± 0.1 | 64.2 ± 0.1 | 79.3 ± 0.5 |
| | enthalpy, J/g | 114.5 ± 0.5 | 92.7 ± 0.2 | 88.7 ± 0.6 | 84.1 ± 3.6 |

FIG. 12 and FIG. 13 show thermogram overlays for LMP- 2, 7, 9, and 11, which all contain 50% caprylic triglyceride (Miglyol 808) with Compritol 888 and/or carnauba wax. LMP- 7 and 9, containing both Compritol and carnauba wax, have a lower congealing and melt temp, and also showed the least handleability, poor flow, and mild agglomeration. LMP-11, with the highest carnauba wax content, exhibits a higher congealing temperature and melting points, and was easier to handle, and flowed better. Interestingly, LMP-2, which does not contain carnauba wax has a higher melting point than LMP- 7 and 9 which contain 20% and 10% carnauba wax, respectively. Also, LMP-2 at 50% caprylic triglyceride has a slightly higher melting point than LMP-5 which has 40% caprylic triglyceride. LMP- 2, 5, and 11 (formulations containing either Compritol or carnauba wax alone) all exhibit better handleability and flow compared to LMP- 7 and 9, which contain a blend of Compritol and carnauba. This is likely due to or related to a melting point depression for the formulations containing both excipients.

FIG. 14A-H compares the LMP formulations to the ingoing excipients (Compritol 888 and/or carnauba wax). The data show that for all four formulations, the addition of caprylic triglyceride (Miglyol 808) decreases the melt point of the excipient, indicating at least partial miscibility. LMP- 7 and 9 exhibit distinctive thermal melt patterns consistent with each individual excipient (Compritol 888 and carnauba wax), indicating that these excipients are not miscible with each other and are likely phase-separated in the solid state. It was observed that the handling properties of LMP- 7 and 9 containing two excipients, were worse than that of LMP- 2, 5, and 11 containing one excipient. This would suggest unfavorable mixing leading to melting point depression of the matrix.

Potency and purity of the LMP formulations was analyzed by HPLC-CAD as described herein at EXAMPLE 2 (see also Table 2-F). All peaks RRT = 1.36 to 2.55 are likely peaks due to excipients that were not extracted from the pure excipient control. Without wishing to be bound any one theory, the caprylic triglyceride (Miglyol 808) may be acting as a co-solvent for the excipient, aiding in extraction of additional components from the LMP matrix compared to the pure component alone for the control.

Results of the analysis are summarized in Table 7-E. Example chromatograms for each of the LMP's are shown in FIG. 14A-14D. The measured purity profile of caprylic triglyceride (Miglyol 808) is slightly changed from the purity analysis of LMP-1, 2, and 3.

All of the impurities present in the ingoing caprylic triglyceride (Miglyol 808), remain unchanged in amount with the exception of RRT = 0.31 & 0.34 for LMP-5- which shows an increase of 0.10% & 0.12%, respectively. Many peaks are present at low levels, and may be related to degradation of caprylic triglyceride, or differences in extraction of minor components present in Compritol 888 or carnauba wax. Without wishing to be bound to any one theory, these peaks may be related to the excipients. Including the unidentified peaks in the total impurities, the amount of potential impurities relative to the caprylic triglyceride (Miglyol) in LMP- 1, 2, and 3 is 0.83 - 2.00%.

**Table 7-E**

| RRT | Peak ID | Miglyol 808 % | LMP-5 % | LMP-7 % | LMP-9 % | LMP-11 % |
|---|---|---|---|---|---|---|
| 0.17 | | ND | ND | ND | ND | <LOQ |
| 0.27 | | ND | <LOQ | ND | ND | ND |
| 0.31 | | 0.34% | 0.44% | 0.37% | 0.36% | 0.36% |
| 0.34 | | 0.20% | 0.32% | 0.21% | 0.21% | 0.21% |
| 0.54 | | ND | 0.22% | 0.09% | 0.11% | ND |
| 0.62 | | <LOQ | 0.06% | 0.06% | 0.06% | 0.06% |
| 0.65 | | 0.07% | 0.07% | 0.06% | 0.07% | 0.06% |

(continued)

| RRT | Peak ID | Miglyol 808 % | LMP-5 % | LMP-7 % | LMP-9 % | LMP-11 % |
|---|---|---|---|---|---|---|
| 0.69 | | ND | 0.09% | 0.21% | ND | ND |
| 0.80 | | ND | 0.43% | 0.13% | ND | 0.08% |
| 0.83 | | ND | ND | ND | 0.19% | ND |
| 0.89 | | ND | ND | <LOQ | ND | ND |
| 0.91 | C6:C8:C8 | 0.06% | 0.06% | 0.06% | 0.07% | 0.06% |
| 0.98 | | ND | <LOQ | ND | ND | ND |
| **1.00** | **Caprylic Triglyceride** | **98.45%** | **96.62%** | **97.52%** | **97.40%** | **97.78%** |
| 1.07 | | ND | ND | ND | 0.07% | ND |
| 1.10 | | 0.08% | 0.09% | 0.09% | 0.08% | 0.10% |
| 1.11 | | ND | ND | ND | ND | 0.06% |
| 1.19 | C10:C8:C8 | 0.80% | 0.82% | 0.81% | 0.82% | 0.83% |
| 1.25 | | ND | 0.08% | ND | ND | ND |
| 1.29 | | ND | 0.07% | ND | ND | ND |
| 1.30 | | ND | ND | ND | <LOQ | 0.06% |
| 1.32 | | <LOQ | <LOQ | <LOQ | ND | <LOQ |
| 1.36 | | ND | ND | ND | 0.07% | 0.40% |
| 1.41 | | ND | ND | 0.06% | ND | 0.06% |
| 1.43 | | ND | 0.05% | 0.05% | 0.06% | ND |
| 1.47 | | ND | 0.05% | ND | ND | ND |
| 1.90 | | ND | 0.09% | 0.07% | <LOQ | 0.05% |
| 1.99 | | ND | 0.15% | 0.08% | <LOQ | ND |
| 2.09 | | ND | 0.44% | 0.18% | 0.11% | ND |
| 2.12 | | ND | <LOQ | 0.18% | 0.14% | ND |
| 2.20 | | ND | ND | ND | 0.24% | ND |
| --- | **Total Impurities** | **1.55%** | **3.55%** | **2.72%** | **2.67%** | **2.37%** |
| | **Assay (%LC)** | **NA** | **96.1%** | **95.9%** | **94.3%** | **94.1%** |
| LOQ>0.05%<br>*Peak corrected for area in compritol control. | | | | | | |

LMP- 5, 7, 9, and 11 formulations were tested in a USP Type II sink dissolution experiment sink performance test using methods outlined described in EXAMPLE 3 (Tables 3-A, 3-B). FIG. 16 shows that LMP- 5, 7, and 9 released to 100 ± 2 %LC by 24 hours. The release rate trends as expected, in that adding more carnauba wax to the LMP matrix slows the dissolution. This is because carnauba wax is more hydrophobic than Compritol 888. LMP-11 shows less than ideal performance and incomplete release at 24 hours. Adding a pore former to the base LMP-11 formulation may allow for increased dissolution performance by enhancing water permeability while maintaining the desirable physical and handling attributes.

LMP-5 (40/60 caprylic triglyceride (Miglyol)/Compritol) and LMP-11 (50/50 caprylic triglyceride (Miglyol)/carnauba) had the best handleability and physical stability of the four formulations. LMP-11 flowability was initially poor post manufacturing but improved significantly by the next day. Oil re-distribution from the surface to the core or crystal changes to the wax matrix may explain the phenomenon. Blending with two weight percent talc improved the flowability of each LMP formulation. LMP-7 (50/30/20 caprylic triglyceride (Miglyol)/Compritol/carnauba) and LMP-9 (50/40/10 caprylic triglyceride (Miglyol)/Compritol/carnauba) had disappointing handleability and physical stability. The cores were waxy, clumpy and smeared easily when handled, LMP-9 more so than LMP-7.

**EXAMPLE 8 - Stability of Formulations LMP-5, LMP-7, LMP-9, and LMP-11**

The stability of LMP-5 and LMP-11 was examined over one, three, and six months. Approximately 8 grams of LMP were loaded into an LDPE bag and closed with a twist tie. Samples were loaded into appropriate chamber open to the condition without sealing or adding desiccant. Time points and conditions are shown in Table 8-A. Samples were pulled and allowed to equilibrate to ambient lab conditions prior to sampling. Samples were stored at ambient conditions during and following analysis.

| Stability Ref# | Sample Description | Proposed Time Point | Condition | Pull Date |
|---|---|---|---|---|
| BREC1719-112A<br>BREC1719-112B | LMP formulation 5, lot BREC1690-097<br>LMP formulation 11, lot BREC1690-100 | Initial | N/A | 6-Oct-2016 |
| BREC1719-112A<br>BREC1719-112B | LMP formulation 5, lot BREC1690-097<br>LMP formulation 11, lot BREC1690-100 | 1 month | 5°C<br>25°C/60%RH<br>30°C/65%RH*<br>40°C/75%RH | 7-Nov-2016 |
| BREC1719-112A<br>BREC1719-112B | LMP formulation 5, lot BREC1690-097<br>LMP formulation 11, lot BREC1690-100 | 3 Months | 5°C<br>25°C/60%RH<br>30°C/65%RH*<br>40°C/75%RH | 6-Jan-2017 |
| BREC1719-112A<br>BREC1719-112B | LMP formulation 5, lot BREC1690-097<br>LMP formulation 11, lot BREC1690-100 | 6 Months (contingency) | 5°C<br>25°C/60%RH<br>30°C/65%RH*<br>40°C/75%RH | 6-Apr-2017 |

*contingency condition

**Table 8-A**

Dissolution of LMP-5 stability samples indicate that LMP-5 has undergone physical changes after 1-month storage, leading to changes in performance. LMP-5 dissolution results are shown in FIG. 17A. LMP-5 stored at 40°C/75% RH is most similar to initial performance. LMP-5 stored at 5°C had a similar dissolution rate through 60 minutes, followed by a drop in rate and extent with only 65%LC dissolved after 24 hours. After 1-month storage at 25°C/65%RH the dissolution rate increased significantly, leading to full release by approximately 3 hours. Visual observations of the LMPs after storage showed that the LMPs stored at 25°C/60%RH and 40°C/75%RH were similar in appearance and flow to the initial sample. LMP-5 stored at 5°C was agglomerated and appeared oily with very poor flow.

LMP-11 exhibited a different behavior than LMP-5 after 1 month stability storage. Results for LMP-11 are shown in FIG. 17B. The 25°C/60%RH and 40°C/75%RH samples showed similar release rate and extent as the initial LMP-11. However, the sample of LMP-11 stored at 5°C had a faster rate of dissolution. Without wishing to be bound by any one theory, it is possible that congealing or crystallization of caprylic triglyceride may have formed pores throughout the carnauba wax matrix, thereby increasing the dissolution rate.

LMP-5 1 month stability samples were tested after being held at ambient conditions for 1 week to test if additional annealing would occur for any of the stability samples. Results as compared to the initial test are shown in FIG. 17C. No differences in dissolution were observed for the 25°C/60%RH or 40°C/75%RH samples indicating that any annealing was complete after the 1 month storage and did not change upon storage at ambient conditions. The 5°C sample did improve slightly. Without being bound to any one theory, the oil that escaped the Compritol matrix during storage due to congealing of the caprylic triglyceride may have migrated back into the LMP matrix instead of free oil at the surface of the particles.

FIG. 18A-H show SEM images of LMP-5 morphology at 1-month 5°C has a somewhat more nodular surface compared to initial, and the surface of LMP-5 stored 1-month at 25°C/60%RH and 40°C/75%RH appears somewhat smoother than initial. FIG. 19A-H shows SEM images for LMP-11 at initial and after 1-month storage. LMP-11 stored at 5°C has a smoother surface compared to initial, and LMP-11 stored 1-month at 25°C/60%RH and 40°C/75%RH has a slightly rougher surface.

Thermal analysis of LMP-5 and LMP-11 at initial and after 1-month is summarized in Table 8-B. Changes to the thermal properties of LMP-5 after storage are minimal, however some small differences exist. The excipient melt occurs at a slightly higher temperature for all of the samples on stability relative to the initial formulation, however the heat of fusion for the melting and congealing events are similar in amplitude. Without wishing to be bound by any one theory, these small differences point to the possibility that the samples have undergone physical changes on stability and that the oil is more

phase separated from the Compritol than the initial sample.

Thermal signals for the LMP-11 on stability are very similar to the initial sample. The one observable difference is the melting of the caprylic triglyceride for the 5°C sample in the cool-heat method is much higher amplitude than the other samples. This increase supports the hypothesis from the dissolution data that the caprylic triglyceride has undergone phase separation from the carnauba wax at 5°C.

| Heat-Cool | | | | | |
|---|---|---|---|---|---|
| **Formulation** | | **LMP-5** | **LMP-5** | **LMP-5** | **LMP-5** |
| **Condition** | | **Initial** | 1 **month 5°C** | 1 **month 25°C/60%RH** | 1 **month 40°C/75%RH** |
| **Melt** | onset, °C | 58.9 ± 0.7 | 62.1 ± 0.2 | 62.1 ± 0.2 | 63.2 ± 0.1 |
| | max, °C | 66.9 ± 0.2 | 70.5 ± 0.2 | 70.5 ± 0.2 | 70 ± 0.2 |
| | enthalpy, J/g | 109.9 ± 3.5 | 118.5 ± 2 | 118.5 ± 2 | 118.2 ± 0.7 |
| **Congeal** | onset, °C | 64.3 ± 0.1 | 65.1 ± 0.2 | 65.1 ± 0.2 | 65.3 ± 0.1 |
| | max, °C | 63.1 ± 0.3 | 63.6 ± 1.2 | 63.6 ± 1.2 | 64.4 ± 0.2 |
| | enthalpy, J/g | 97.2 ± 1.4 | 72.4 ± 2.4 | 72.4 ± 2.4 | 72.5 ± 1.8 |
| **Low Temp Congeal** | onset, °C | NA | -38 ± 0.2 | -38.1 ± 0.2 | -38.2 ± 0.2 |
| | max, °C | NA | -41.9 ± 0.2 | -41.7 ± 0.3 | -42.4 ± 0.1 |
| | **enthalpy,** J/g | NA | 16.2 ± 3.2 | 14.7 ± 5.9 | 15.1 ± 1 |
| **Formulation** | | **LMP-11** | **LMP-11** | **LMP-11** | **LMP-11** |
| **Condition** | | **Initial** | **1 month 5°C** | **1 month 25°C/60%RH** | 1 **month 40°C/75%RH** |
| **Melt** | onset, °C | 68.6 ± 0.4 | 69.9 ± 0.3 | 69.2 ± 1.1 | 69.2 ± 0.9 |
| | max, °C | 78.2 ± 0.1 | 78.5 ± 0.4 | 78.4 ± 0.1 | 78.7 ± 0.4 |
| | enthalpy, J/g | 93.5 ± 0 | 63.4 ± 2.1 | 72.2 ± 10.1 | 70.9 ± 5.6 |
| **Congeal** | onset, °C | 75.4 ± 0 | 75.2 ± 0.3 | 75.2 ± 0.2 | 74.9 ± 0.1 |
| | max, °C | 74.7 ± 0.3 | 74.9 ± 0.4 | 74.8 ± 0.2 | 74.5 ± 0.5 |
| | enthalpy, J/g | 78.1 ± 5.6 | 76.8 ± 6.7 | 71.5 ± 9.4 | 66.1 ± 7.7 |
| **Low Temp Congeal** | onset, °C | -59.8 ± 0 | -60.1 ±+ 0.1 | -59.9 ± 0.2 | -60.3 ± 0.2 |
| | max, °C | -61.3 ± 0 | -61.6 ± 0.2 | -61.5 ± 0.2 | -61.9 ± 0.3 |
| | enthalpy, J/g | 12.1 ± 0.2 | 12.9 ± 0.4 | 13.1 ± 0.4 | 12.4 ± 0.3 |

**Table 8-B**

| Cool-Heat | | | | | |
|---|---|---|---|---|---|
| **Formulation** | | **LMP-5** | **LMP-5** | **LMP-5** | **LMP-5** |
| **Condition** | | **Initial** | **1 month 5°C** | **1 month 25°C/60%RH** | **1 month 40°C/75%RH** |
| **Exotherm, cooling** | onset, °C | -12.7 ± 0.2 | -13.2 ± 0.1 | -12.9 ± 0.1 | -14.2 ± 0 |
| | max, °C | -14.8 ± 0.1 | -15.8 ± 0.2 | -15.5 ± 0.2 | -16.8 ± 0.1 |
| | enthalpy, J/g | 34.2 ± 0.1 | 33 ± 2.4 | 34.9 ± 1.1 | 37 ± 0.6 |
| **Melt 1 (CT)** | onset, °C | 4.9 ± 0 | 6.8 ± 0.2 | 3.8 ± 0.2 | 3.5 ± 0 |
| | max, °C | 9.3 ± 0.1 | 10.1 ± 0.2 | 8.7 ± 0.1 | 8.6 ± 0.1 |
| | enthalpy, J/g | 38.7 ± 0.1 | 38.7 ± 1.1 | 37.8 ± 1.6 | 40.6 ± 0.6 |
| **Melt 2 (Excipient)** | onset, °C | 59.4 ± 0.1 | 62.3 ± 0.2 | 63.3 ± 0 | 64.1 ± 0.1 |
| | max, °C | 67 ± 0.1 | 70.6 ± 0.2 | 69.9 ± 0.1 | 70.3 ± 0.1 |
| | enthalpy, J/g | 114.5 ± 0.5 | 120.7 ± 2.3 | 116.2 ± 4.4 | 120.3 ± 2.2 |

(continued)

| Formulation | | LMP-11 | LMP-11 | LMP-11 | LMP-11 |
|---|---|---|---|---|---|
| Condition | | Initial | 1 month 5°C | 1 month 25°C/60%RH | 1 month 40°C/75%RH |
| Exotherm, cooling | onset, °C | -61.2 ± 0.6 | -59.8 ± 0.1 | -61.4 ± 0.1 | -61.5 ± 0.1 |
| | max, °C | -63.4 ± 0.9 | -61.6 ± 0.2 | -63.7 ± 0.4 | -63.4 ± 0.2 |
| | enthalpy, J/g | 9.6 ± 0.8 | 7.9 ± 0.6 | 9.2 ± 0.7 | 11.8 ± 0.3 |
| Melt 1 (CT) | onset, °C | 6.7 ± 0.5 | 6.7 ± 0.1 | 6.1 ± 0.2 | 5.9 ± 0.1 |
| | max, °C | 10.1 ± 0.5 | 9.8 ± 0.2 | 9.3 ± 0.3 | 9.1 ± 0.1 |
| | enthalpy, J/g | 24.7 ± 10.5 | 34.1 ± 0.7 | 19.1 ± 1 | 17.3 ± 1.1 |
| Melt 2 (Excipient) | onset, °C | 68.6 ± 0.8 | 68.8 ± 0.3 | 68.2 ± 0.5 | 67.5 ± 0.4 |
| | max, °C | 79.3 ± 0.5 | 78.4 ± 0.3 | 78.3 ± 0.4 | 78.4 ± 0.2 |
| | enthalpy, J/g | 84.1 ± 3.6 | 90.6 ± 2.5 | 94 ± 8.5 | 86.5 ± 3.8 |

LMP-5 was tested for Potency and Purity after storage for 1 month on stability. Results are shown in Table 8-C. There were no significant changes from the initial purity data for LMP-5 at 1 month on stability.

**Table 8-C**

| RRT | Peak ID | I LMP-5, Initial | I LMP-5, 5C | LMP-5, 25C/60%RH | LMP-5, 40C/75%RH I |
|---|---|---|---|---|---|
| 0.31 | | 0.44% | 0.29% | 0.38% | 0.36% |
| 0.34 | | 0.32% | 0.20% | 0.31% | 0.25% |
| 0.41 | | ND | 0.06% | 0.06% | 0.06% |
| 0.54 | | 0.22% | 0.16% | 0.15% | 0.15% |
| 0.62 | | 0.06% | ND | ND | ND |
| 0.65 | | 0.07% | 0.06% | 0.06% | 0.07% |
| 0.69 | | 0.09% | ND | ND | ND |
| 0.80 | | 0.43% | 0.45% | 0.45% | 0.44% |
| 0.91 | C6:C8:C8 | 0.06% | 0.06% | 0.07% | 0.07% |
| 0.98 | | <LOQ | ND | ND | ND |
| **1.00** | **Caprylic Triglyceride** | **96.62%** | **97.44%** | **97.23%** | **97.47%** |
| 1.10 | | 0.09% | 0.12% | 0.10% | 0.10% |
| 1.19 | C10:C8:C8 | 0.82% | 0.99% | 1.00% | 1.00% |
| 1.25 | | 0.08% | 0.08% | 0.08% | 0.08% |
| 1.26 | | ND | ND | ND | 0.15% |
| 1.29 | | 0.07% | 0.06% | 0.05% | 0.06% |
| 1.32 | | <LOQ | 0.07% | ND | 0.07% |
| 1.43 | | 0.05% | ND | 0.07% | ND |
| 1.47 | | 0.05% | ND | ND | ND |
| 1.90 | | 0.09% | ND | ND | ND |
| 1.99 | | 0.15% | ND | ND | ND |
| 2.09 | | 0.44% | ND | ND | ND |
| 2.12 | | <LOQ | ND | ND | ND |
| --- | **Total Impurities** | **3.38%** | **2.56%** | **2.77%** | **2.72%** |

(continued)

| RRT | Peak ID | I LMP-5, Initial | I LMP-5, 5C | LMP-5, 25C/60%RH | LMP-5, 40C/75%RH I |
|---|---|---|---|---|---|
| | **Assay (%LC)** | **96.1 ± 0.3%** | **97.2 ± 2.1%** | **99.5 ± 0.0%** | **99.4 ± 0.3%** |
| ND = not detected<br>LOQ>0.05% | | | | | |

The primary observation for LMP-5 and LMP-11 formulations after 1-month stability is that storage at 5°C likely causes phase separation, which impacts dissolution performance.

**EXAMPLE 9 - Dissolution Enhancers**

LMP-5 and LMP-11 had good handleability and physical characteristics but the dissolution rate not optimal. LMP-5 and LMP-11 released 80% of the API in 3 hours and > 24 hours respectively. Dissolution rate enhancers were added to maintain or improve the handleability while increasing the dissolution rate. Dissolution enhancers are water soluble or hydrophilic excipients that speed up dissolution by increasing the rate of water penetration into the LMP and/or increasing the transport of API out of the LMP formulation. The manufacturing process followed the same approach as the previous described.

Seven LMP formulations were manufactured. The manufacturing summary and handleability rankings are shown in Table 9-A and Table 9-B. The LMPs were blended with 2 wt% talc and ranked in terms of handleability and flowability. Hardness was determined by pressing the LMP particles between ones fingers and the weight boat to see how well the particles held up. The smear was determined by rubbing the LMPs between ones fingers and assessing on how the particles clumped up, smeared and softened.

The addition of the pore former improved the handleability for all formulations relative to LMP-5 except for BREC1690-155 (40/50/10 AC-1204/Compritol/Lecithin) and BREC1690-161 (60/30/10 AC-1204/carnauba wax/PVP k17). Both P407 and PVP k17 based LMPs showed excellent physical characteristics, even when the drug loading was increased to 50%. Only when the drug loading was pushed to 60% did the handleability become unacceptable. Consistent with the melt screening, the P407 formulation flow and physical characteristics seemed to improve the day after manufacture. The PVP-VA 64 particles were very hard, however during the manufacture they seemed to stick to each other and the collection cone. This would likely lead to significant manufacturing issues upon scale-up. This formulation is less desired for this reason. The Lecithin 90G resulted in very soft LMP particles that did not flow well or hold up when physically handled.

| MSC Manufacturing Summary | | | | | | | |
|---|---|---|---|---|---|---|---|
| Mfg Ref | BREC1690-155 | BREC1690-156 | BREC1690-157 | BREC1690-158 | BREC1690-160 | BREC1690-161 | BREC1690-162 |
| Mfg Date | 9 Nov 2016 | | 10 Nov 2016 | | 11 Nov 2016 | | |
| Composition | 40/50/10 AC-1204/ Compritol 888/ Lecithin 90G | 40/50/10 AC-1204/ Compritol 888/ P407 | 40/50/10 AC-1204/ Compritol 888/ PVP 17PF | 40/50/10 AC-1204/ Compritol 888/ PVP VA64 | 50/40/10 AC-1204/ Carnuba wax/ P407 | 60/30/10 AC-1204/ Carnuba wax/ PVP 17PF | 50/40/10 AC-1204/ Compritol 888/ PVP 17PF |
| Atomizer | 4" heated stainless steel disk | | | | | | |
| Disk Speed | 1750 rpm | | | | | | |
| Pump System | 8 psi | 2 psi | | | | | |
| Gear pump speed | 63 rpm | | | | | | |
| Jacket Temp | 85°C | 90°C | 90°C | 90°C | 110°C | 110°C | 110°C |
| Jacket Temp | 85°C | 90°C | 90°C | 90°C | 110°C | 110°C | 110°C |
| Bag Temp | Ambient (22-24°C) | | | | | | |
| Run Time (min:sec) | 2:23 | 4:05 | 3:05 | 3:05 | 2:14 | 2:05 | 2:50 |
| Spray Rate (g/min) | 210 | 122 | 162 | 162 | n/a* | n/a* | 177 |
| Batch Size | 500 g | | | | | | |
| Product Collected | 232 g | 330 g | 309 g | 155 g | 237 g | 154 g | 202 g |
| Yield | 46% | 66% | 62% | 31% | 47% | 31% | 40% |
| *A small leak in the feed system resulted in the low yeild and short run time | | | | | | | |

**Table 9-A**

**Table 9-B**

| | Formulation Weight % | | | | | | | Physical Characteristic | | |
|---|---|---|---|---|---|---|---|---|---|---|
| **LOT** | MCT | Compritol 888 | Carnauba Wax | Lecithin 90G | P407 | PVP k17 | PVP VA64 | Hardness | Smear | Flow |
| **BREC 1690-155** | 40 | 50 | | 10 | | | | 2 | 2 | 5 |
| **BREC 1690-156** | 40 | 50 | | | 10 | | | 8 | 7 | 8 |
| **BREC 1690-157-** | 40 | 50 | | | | 10 | | 9 | 10 | 7 |
| **BREC 1690-158** | 40 | 50 | | | | | 10 | 9 | 8 | 7 |
| **BREC 1690-160** | 50 | | 40 | | 10 | | | 8 | 7 | 7 |
| **BREC 1690-161** | 60 | | 30 | | | 10 | | 4 | 4 | 4 |
| **BREC 1690-162** | 50 | 40 | | | | 10 | | 7 | 6 | 8 |
| **LMP-5** | 40 | 60 | | | | | | 5 | 5 | 8 |
| **LMP-11** | 50 | 50 | | | | | | 10 | 9 | 9 |

The particle size distribution was evaluated using light microscopy. The LMPs were spread into a uniform layer on a glass slide and the particles were measured using a calibrated particle sizing software. Selection bias was minimized by measuring every particle within the field of view. The D10, D50, D90 and D4,3 statistics were calculated using a mass weighted basis because the historical LMP size data from laser diffraction and sieve analysis is a mass/volume weighted average. The particle size distribution statistics is shown below shown in Table 9-C. The particle size is within the expected range with a D50 between 150 and 250 μm. The spans range from around 0.4 to greater than 0.7, which is very tight indicating a uniform particle size distribution.

| Formulation | D10 (μm) | D50 (μm) | D90 (μm) | D4,3 (μm) | Span |
|---|---|---|---|---|---|
| BREC1690-155 | 141 | 183 | 243 | 186 | 0.56 |
| BREC1690-156 | 148 | 186 | 230 | 188 | 0.44 |
| BREC1690-157 | 136 | 171 | 222 | 175 | 0.51 |
| BREC1690-158 | 145 | 204 | 286 | 208 | 0.69 |
| BREC1690-160 | 178 | 230 | 283 | 233 | 0.46 |
| BREC1690-161 | 180 | 240 | 294 | 238 | 0.47 |
| BREC1690-162 | 185 | 234 | 275 | 230 | 0.39 |
| Test Ref: BREC1690-166 | | | | | |

**Table 9-C**

The particles were imaged using a Scanning Electron Microscope to assess particle morphology and surface characteristics of the different formulations. The particle morphology of all formulations was consistent with the light microscope observations, in that all formulations had spherical particles. The two formulations that were the softest (BREC1690-155, 161) had surfaces that appeared softer, oily, and less rigid. These two soft formulation were the only ones that differed from the surface structures of LMP- 5, 7, 9, and 11. Representative images are shown in FIGS. 20A-H.

The bulk and tapped density was measured and the Carr index calculated to quantify the flowability of LMPs dry blended with 2% talc as a flow aid. The results are shown below in Table 9-D (n=2 for all samples in 10mL cylinders). The bulk density of the 7 formulations was similar, varying between 0.49 and 0.54 g/cm$^3$. The maximum Carr index was 10 indicating good flowability, however, visually the worst flowing LMP formulation (BREC1690-161) had the same Carr index

as the other formulation and did not flow well. All other formulations visually flowed very well. Flowdex measurements were also performed on the two lead formulations, BREC1690-156 and BREC1690-162. The results are below in Table 9-D. Flowdex is a powder flow measurement in which the goal is to find the size of an orifice that the material will freely flow through. The material is poured into a cylinder container with a drop lever, and is allowed to settle for 30 seconds for consistency between tests. The lever is then dropped, which does not disturb the material, and then the material either flows through the hole or it does not. In order to pass the test the material must flow through the same size orifice 3 consecutive times.

| Lot | Bulk Density (g/mL) | Tap Density (g/mL) | Carr Index (%) | Flowdex (mm) |
|---|---|---|---|---|
| BREC1690-155 | 0.50 | 0.55 | 9 | -- |
| BREC1690-156 | 0.52 | 0.58 | 10 | 8 |
| BREC1690-157 | 0.53 | 0.58 | 8 | -- |
| BREC1690-158 | 0.52 | 0.56 | 9 | -- |
| BREC1690-160 | 0.52 | 0.57 | 9 | -- |
| BREC1690-161 | 0.49 | 0.54 | 9 | -- |
| BREC1690-162 | 0.54 | 0.58 | 7 | 12 |

**Table 9-D**

Formulations BREC1690- 155, 156, 160, 161, and 162 were tested in a sink dissolution performance test using methods outlined herein at EXAMPLE 3 (Tables 3-A and 3-B). The dissolution rate was increased by the addition of a water soluble component (PVP 17PF, PVP-VA 64 or poloxamer P407). Dissolution was tested on formulations BREC1690-155, 156, 160, 161, and 162. Results are shown in FIG. 21. Comparisons of previous Compritol based LMPs (LMP-2 and LMP-5) and BREC1690- 155, 156, and 162 with a dissolution enhancer are shown in FIG. 22. Comparison of previous carnauba wax LMP-11 and the carnauba wax formulations with a dissolution enhancer are shown in FIG. 23.

For all 5 formulations, the addition of a dissolution enhancer improved the dissolution rate verses the control formulation without the dissolution enhancer. Although the formulations containing carnauba wax improved greatly, the best performing carnauba wax formulations was still slower to release than LMP-5. Additional optimization may lead to faster release rates for the carnauba wax based formulations, but likely none as fast as a Compritol based formulation with a dissolution enhancer.

LMP-5 and BREC1690- 156, -157, and 162 were tested by *in vitro* digestion. The digestion medium composition is described in Table 9-E.

**Table 9-E**

| Ingredient | Concentration | Mass (g) for 250 mL |
|---|---|---|
| sodium tarodexoycholate (NaTDC) | 3 mM | 0.43 |
| phosphatidylcholine (PC) | 0.75 mM | 0.16 |
| digestion buffer | up to volume | |

The digestion buffer is described in Table 9-F.

**Table 9-F**

| Ingredient | Concentration | Mass (g) for 1 L of buffer |
|---|---|---|
| Tris-maleate | 2 mM | 0.47 |
| Sodium chloride | 150 mM | 8.77 |
| Calcium chloride dihydrate | 1.4mM | 0.21 |
| Sodium hydroxide | qs to pH 6.5 $\pm$ 0.1 | |
| Demineralized water | up to volume | |

The pancreatin suspension comprised supernatant of 0.2g/mL porcine pancreatin (8x USP specification) in digestion buffer. The dissolution method uses a 0.6N NaOH titrant with stirring at 37.0 ± 1.0°C and a pH of 6.5

FIG. 24 shows *in vitro* digestion profiles for BREC1690- 156, 157, and 165 compritol-based formulations are very similar to each other and to the caprylic triglyceride (Miglyol 808) oil control sample, and were all much faster than LMP-5 *in vitro* digestion. Potency of BREC1690- 156 and 162 was examined. It ranged from 96.9% LC to 98.1% LC.

## EXAMPLE 10 - Stability of BREC1690- 156 and 162

The stability of BREC1690- 156 and 162 was examined at two weeks and one month. BREC1690- 156 and 162 were added into an LDPE bag and closed with a twist tie. Approximately 9 grams were added per package for the following storage conditions: 25°C/60%RH, 30°C/65%RH, and 40°C/75%RH, and 6.5 grams were added per package for the 5°C and -20°C contingency storage conditions. Samples were placed in an appropriate stability chamber open to the condition, without sealing or adding desiccant. Samples were pulled and allowed to equilibrate to ambient conditions prior to sampling for testing. Contingency samples were stored at 5°C and -20°C were evaluated at each time point, and observations made of appearance.

After two weeks of storage on stability, the 25°C/60%RH and 40°C/75%RH condition samples for both formulations were tested for *in vitro* digestion performance. Results are shown in FIG. 25 and FIG. 26. The lot BREC1690-156 containing poloxamer P407 has no significant change relative to the initial digestion profile, indicating that there is likely no annealing effect. The digestion rate and extent are also very similar to the caprylic triglyceride (Miglyol 808) control sample. The lot BREC1690-162 LMP containing PVP 17PF had little change for the 25°C/60%RH sample, however the sample stored at 40°C/75%RH showed a faster initial digestion rate compared to the initial/T0 sample. The digestion is greater than the P407 LMP, and similar to the caprylic triglyceride (Miglyol 808) control. Based on these data, it is likely that the P407 LMP would not require an annealing step post-manufacture, whereas the PVP 17PF formulation may require annealing.

Images by SEM and camera were acquired for the BREC1690- 156 and 162, 2 week stability samples. (FIG. 27A-D, FIG. 27A-D.) Appearance of surface structures for the LMP on stability can be attributed to talc that was not present for the initial SEM images. BREC1690-156 LMP had minimal changes on stability, whereas the BREC1690-162 LMP had significant increases in surface roughness. Clumping was observed for many of the stability samples. However, all samples were easily broken up and returned to a free flowing powder after gentle agitation, with the exception of the 5°C samples which remained slightly greasy and clumped.

After one month of storage on stability, the 25°C/60%RH and 40°C/75%RH condition samples for both formulations were tested for *in vitro* digestion performance. Results are shown in FIG. 24 and FIG. 25. The lot BREC1690-156 LMP containing poloxamer P407 has no significant change relative to the initial digestion profile, indicating that there is likely no annealing effect. The digestion rate and extent are also very similar to the caprylic triglyceride (Miglyol 808) control sample. The lot BREC1690-162 LMP containing PVP 17PF had little change for the 25°C/60%RH sample, however the sample stored at 40°C/75%RH showed a faster initial digestion rate compared to the initial/T0 sample and similar to the 2 week sample stored at the same condition. The digestion was greater than the P407 LMP, and similar to the caprylic triglyceride (Miglyol 808) control. Based on these data, it is likely that the P407 LMP would not require an annealing step post-manufacture, whereas the PVP 17PF formulation may require annealing for less than or equal to 2 weeks as the digestion rate has not changed significantly from the 2 week analysis.

Images by SEM and camera were acquired for BREC1690- 156 and 162, 1 month stability samples. (FIG. 29A-D and FIG. 30A-D.) BREC1690-156 LMP had minimal changes on stability, whereas the BREC1690-162 LMP had significant increases in surface roughness. Clumping was observed for many of the stability samples. However, all samples were easily broken up and returned to a free flowing powder after gentle agitation, with the exception of the 5°C samples which remained slightly greasy and clumped.

## EXAMPLE 11 - Additional Matrix Forming Excipients

Additional pharmaceutical formulations were prepared in accordance with the spinning disk procedures described in the examples above and evaluated via an initial melt screen. Viability of tested formulations were compared to a baseline sample of 50/40/10 MCT/Compritol/PVP-17 (BREC1690-162). Approximately twenty unique formulations varying in matrix and excipient ratios were evaluated. Test formulations were hand tested for softness and gauged against the physical attributes of BREC1690-162. A positive result is generated if the test formulation has rapid congealing behavior and physical robustness comparable to BREC1690-162. An intermediate result is a formulation with acceptable physical robustness likely to have incomplete release of the MCT, and a negative result is a formulation that is too soft in hand.

In sum, melt screening showed that hydrogenated castor oil (HCO) alone does not provide desired matrix forming properties, but does provide good results when used in combination with other waxes and excipients. Rice bran wax and

carnauba wax both show good properties alone or in combination.

Based on the results of the melt screening experiments, exemplary formulations are as described herein and reflected in Table 11-A below.

**Table 11-A**

| Formulation |
|---|
| 50/25/25 MCT/HCO/Carnauba Wax |
| 50/40/10 MCT/Rice Bran Wax/PVP-17 |
| 50/30/20 MCT/Carnauba Wax/Gelucire 50/13 |
| 50/50 MCT/Rice Bran Wax |

All of the above formulations had good miscibility with the active material in the molten state with the exception of 50/40/10 MCT/Rice Bran Wax/PVP-17, which rapidly phase separates into what appears (based on the color of the phases) to be a PVP/MCT phase and a molten Rice Bran Wax phase, with some PVP suspended in the Rice Bran Wax phase. Miscibility of the MCT oil in pure Rice Bran wax was excellent to the point that no agitation was required to form a continuous phase. Interestingly, the HCO/Carnauba Wax system readily forms a continuous phase in the liquid state, but appears to undergo phase separation of the excipients on congealing.

Congealing kinetics during "flicking" of hot melt was comparable across all the above formulations, with congealing of droplets comparable in size to the target diameter of LMPs rapidly congealing in 1-2 seconds.

The poor performance of the HCO alone was surprising, as its high melting temperature is known to play a large role in the hardness of the congealed material. HCO (Tm = 85-88 °C per USP32-NF27) has a higher melt temperature than Compritol (Tm = 65-77 °C per PhEur 6.0, unspecified in USP-NF), previously shown to be a good matrix forming agent, with good hardness in the 50/40/10 MCT/Compritol/PVP-17 formulation.

Without intending to be limited by theory, it is believed that this suggests that the fatty acid chain length of the glyceride matrix as well as the level of esterification of the glyceride plays a role in the underlying mechanism by which the Compritol/PVP-17 system is able to effectively encapsulate the MCT oil and form a hard particle. Compritol is a mixture of mono-, di-, and triglycerides of behenic acid (C22), while HCO is primarily the triglyceride of hydroxystearate (C18). The presence of alcohol groups in the fatty acid chain may also play a role in the softness of the HCO formulations. It is also worth noting that the Compritol is able to solubilize a proportion of the PVP-17 while HCO was unable to disperse the polymer, much less solubilize it. This suggests that the dissolution of the PVP-17 into the Compritol may further add to the observed physical stability of the formulation.

The carnauba wax/Gelucire-based formulation was observed to initially be soft and slightly pliable, but hardened up significantly on storage overnight at room temperature. Without intending to be limited by theory, this is believed to be due to the additional time allowing the long chains of the carnauba wax to rearrange and pack more tightly. This change was not noted in the HCO/carnauba wax system. This effect may be mitigated by the emulsifying action of the Gelucire 50/13. Previous Carnauba-based formulations made use of the much more hydrophilic dissolution enhancer Poloxamer 407.

Both rice bran wax-based formulations exude liquid oil when significant pressure is applied to the material, but show no oil leaking otherwise in the solid state. Again, without intending to be limited, this illustrates that the MCT oil is distributed throughout the Rice Bran Wax matrix in liquid oil pockets on congealing of the matrix rather than remaining miscible with the matrix.

## EXAMPLE 12 -Additional Dissolution Enhancers

Increased levels of dissolution enhancer as well as the alternative hydrophilic dissolution enhancer GELUCIRE® 50/13 were evaluated to investigate and improve release of the MCT oil from the LMPs. GELUCIRE® 50/13 was selected as an exemplary polyoxylglyceride. It is more hydrophobic than Poloxamer, but maintains sufficient hydrophilic character to enable dissolution in aqueous media.

Exemplary formulations are presented in Table 12-A below, with a 50% MCT loading.

**Table 12-A**

| Formulation Ref (BREC-2131-) | | | |
|---|---|---|---|
| **021** | Carnauba Wax (30%) | NA | P407 (20%) |
| **022** | Carnauba Wax (30%) | NA | Gelucire 50/13 (20%) |

(continued)

| **Formulation Ref (BREC-2131-)** | | | |
|---|---|---|---|
| **031** | Microcrystalline Wax (40%) | NA | P407 (10%) |

The 50/30/20 MCT/Carnauba Wax/P407 was too soft for further testing, but could be enhanced with a stiffening agent. The 50/40/10 MCT/Microcrystalline Wax/P407 system is considered an "intermediate" result because while the physical stability of the formulation was acceptable, it is considered unlikely to have good dissolution performance at this relatively low loading of surfactant.

**EXAMPLE 13 - Additional Formulation Optimization**

Select formulations described herein were selected for further investigation and manufacturing via a Melt-Spray-Congeal (MSC) process. Exemplary formulations are provided in Table 13-A below.

**Table 13-A**

| **Rank** | |
|---|---|
| **1** | 40/25/25/10 MCT/HCO/Carnauba Wax/Gelucire 50_13 |
| **2** | 45/50/5 MCT/Rice Bran Wax/Gelucire 50_13 |
| **3** | 40/50/10 MCT/Rice Bran Wax/Gelucire 50_13 |
| **NA** | 45/35/10/5 MCT/Rice Bran Wax/PVP-17/Gelucire 50_13 |

All the formulations tested rapidly congealed and solidified to a sufficient hardness to prototype for MSC processing with the exception of the Rice Bran Wax/PVP-17/Gelucire 50_13 system. All the above formulations had good miscibility with the active material in the molten state, with the exception of 45/35/10/5 MCT/Rice Bran Wax/PVP-17/Gelucire 50_13 and 40/50/10 MCT/Rice Bran Wax/Gelucire 50_13, which were observed to form a fine emulsion visible as a haze.

Gelucire 50_13 was added to the 50/40/10 MCT/Rice Bran Wax/PVP-17 in order to stabilize the components into a single phase, but the PVP-17 formed hard agglomerates and was unable to be dispersed. Testing was suspended on that observation. This approach may still be tested with the dissolution enhancer Poloxamer 407, which is more like PVP-17 in hydrophilicity than the Gelucire 50_13. Previous testing showed that a small amount of Poloxamer may improve dispersion of the PVP-17 in the melt.

According to the results of initial testing, six formulations were manufactured. The formulations manufactured are given in Table 13-B below.

**Table 13-B**

| | |
|---|---|
| **BREC2131-065** | 50/40/10 MCT/Compritol/PVP-17 |
| **BREC2131-066** | 40/48/12 MCT/Compritol/PVP-17 |
| **BREC2131-057** | 50/35/15 MCT/Carnauba Wax/Gelucire 50_13 |
| **BREC2131-058** | 40/40/20 MCT/Carnauba Wax/Gelucire 50_13 |
| **BREC2131-059** | 50/50 MCT/Rice Bran Wax |
| **BREC2131-060** | 50/50 MCT/Microcrystalline wax |

A manufacturing summary for the prototype manufacturing of the above six formulations is given in Table 13-C below.

| MSC Manufacturing Summary | | | | | | |
|---|---|---|---|---|---|---|
| Mfg Ref | BREC2131-057 | BREC2131-058 | BREC2131-059 | BREC2131-060 | BREC2131-065 | BREC2131-066 |
| Composition | 50/35/15 CT/Carnauba/Ge50_13 | 40/40/20 CT/Carnauba/Ge50_13 | 50/50 CT/Rice Bran Wax | 50/50 CT/Microcrystalline wax | 50/40/10 CT/Compritol/PVP-17 | 40/48/12 CT/Compritol/PVP-17 |
| Batch Size (g) | 700 | | | 270* | 700 | |
| Throughput (kg/hr) | 10.1 | 8.1 | 8.8 | 10.9 | 10.9 | 9.4 |
| Product Collected (g) | 556 | 420 | 479 | 178 | 568 | 597 |
| Flowability | Excellent | Excellent | Good | Good | Initially good clumps on storage | Initially good clumps on storage |
| Particle Hardness | Good | Good | Good | Excellent | Good | Excellent |
| Processability | Good | Good | Good | Good | Good | Good |

*batch size reduced due to supply chain issues sourcing the same grade of material. Total sample quantity remaining from melt screening used to manufacture prototype

**Table 13-C**

Atomization was assessed by high-speed camera for all formulations. All formulations were further confirmed to be processable at the manufacturing conditions by the observation of ligamentous and uniform atomization.

The particle shape and surface morphology of each formulation was evaluated by SEM and is shown in FIGS. 31A-35C. FIGS. 31A-C show SEM images of 50/35/15 MCT/Carnauba Wax/Gelucire 50/13 LMPs at 50x (FIG. 31A), 300x (FIG. 31B), and 1500x magnification (FIG. 31C). FIGS. 32A-C show SEM images of 40/40/20 MCT/Carnauba Wax/Gelucire 50/13 LMPs at 50x (FIG. 32A), 300x (FIG. 32B), and 1500x magnification (FIG. 32C). FIGS. 33A-C show SEM images of 50/50 MCT/Rice Bran Wax LMPs at 50x (FIG. 33A), 300x (FIG. 33B), and 1500x magnification (FIG. 33C). FIGS. 34A-C show SEM images of 50/40/10 MCT/Compritol/PVP-17 LMPs at 50x (FIG. 34A), 300x (FIG. 34B), and 1500x magnification (FIG. 34C). FIGS. 35A-C show SEM images of 40/48/12 MCT/Compritol/PVP-17 LMPs at 50x (FIG. 35A), 300x (FIG. 35B), and 1500x magnification (FIG. 35C).

It is intended that the invention not be limited to the particular embodiment disclosed as the best mode contemplated for carrying out this invention, but that the invention will include all embodiments falling within the scope of the appended claims.

## Claims

1. A pharmaceutical formulation comprising between 30% and 50% by weight of the total composition of caprylic triglyceride, and at least one matrix forming agent present in an amount of between about 20% and about 70% by weight of the total composition, wherein the at least one matrix forming agent is selected from the group consisting of glyceryl dibehenate, carnauba wax, and combinations thereof.

2. The pharmaceutical composition of any of the preceding claims, wherein the matrix forming agent is present in an amount of between about 40% and about 70% by weight of the total composition, optionally wherein the caprylic triglyceride is present in an amount of between about 40% and about 50% by weight of the total composition and the matrix forming agent is present in an amount of between about 50% and about 60% by weight of the total composition.

3. The pharmaceutical composition of any of the preceding claims, wherein the pharmaceutical composition comprises a dissolution enhancer,

   optionally wherein the dissolution enhancer is selected from the group consisting of poly(ethylene glycol)-block-poly(propylene glycol)-block-poly(ethylene glycol) copolymer, polyethylene glycol copolymer, starch, rice starch, lecithin, polyvinylpyrrolidone (PVP), polyoxylglycerides, polyvinylpyrrolidone-vinyl acetate copolymer (PVP-VA), hydroxypropyl methylcellulose (HPMC), hypromellose acetate succinate (HPMCAS), Low-substituted hydroxypropyl cellulose (L-HPC), sorbitan esters, polyethylene glycol, sorbitan fatty acid esters, and combinations thereof,

   optionally wherein the dissolution enhancer is present in an amount of about 1% to about 20% by weight of the total composition, and

optionally wherein the dissolution enhancer is poly(ethylene glycol)-block-poly(propylene glycol)-block-poly(ethylene glycol) copolymer.

**4.** A pharmaceutical composition of any of the preceding claims, wherein the pharmaceutical composition comprises a flow aid,

optionally wherein the flow aid is selected from the group consisting of amphorous silica gel, silica aerogel, magnesium alumino metasilicates, calcium silicate, ordered mesoporous silica, micronized talc, and combinations thereof,
optionally wherein the flow aid is present in an amount of 0.1% to 5% by weight of the total composition.

**5.** A pharmaceutical composition of any of the preceding claims, wherein the pharmaceutical composition comprises a stiffening agent,

optionally wherein the stiffening agent is selected from the group consisting of carnauba wax, candelilla wax, and combinations thereof,
optionally wherein the stiffening agent is present in an amount of 1% to 60% by weight of the total composition.

**6.** The pharmaceutical composition of any of the preceding claims, wherein the composition is comprised of discrete particles including the caprylic triglyceride and glyceride, optionally wherein the particles have an average diameter, as measured according to the description, of between about 50 μm and about 350 μm, or of between about 100 μm and about 300 μm, optionally wherein the particles exhibit a Carr Index of flowability of less than 10%.

**7.** The composition of any of the preceding claims, wherein the purity of the caprylic triglyceride is at least 95%.

**8.** The pharmaceutical composition of any of the preceding claims for use in a method of treating a disease or disorder associated with reduced cognitive function, the method comprising administering the composition to the subject in an amount effective to elevate ketone body concentrations in said subject to thereby treat said disease or disorder, optionally wherein the method further comprises determining if the patient lacks the ApoE4 genotype.

**9.** The pharmaceutical composition for use according to claim 8, wherein the disease or disorder associated with reduced cognitive function is selected from Alzheimer's disease and Age-Associated Memory Impairment.

**10.** The pharmaceutical composition for use according to claim 8 or claim 9, wherein the composition is administered at a dose of about 0.05 g/kg/day to about 10 g/kg/day.

## Patentansprüche

**1.** Pharmazeutische Formulierung, umfassend zwischen 30 Gew.-% und 50 Gew.-% der Gesamtzusammensetzung Capryltriglycerid und zumindest ein matrixbildendes Mittel, das in einer Menge von zwischen etwa 20 Gew.-% und etwa 70 Gew.-% der Gesamtzusammensetzung vorhanden ist, wobei das zumindest eine matrixbildende Mittel aus der aus Glyceryldibehenat, Carnaubawachs und Kombinationen davon bestehenden Gruppe ausgewählt ist.

**2.** Pharmazeutische Zusammensetzung nach einem der vorangegangenen Ansprüche, wobei das matrixbildende Mittel in einer Menge zwischen etwa 40 Gew.-% und etwa 70 Gew.-% der Gesamtzusammensetzung vorhanden ist, gegebenenfalls wobei das Capryltriglycerid in einer Menge zwischen etwa 40 Gew.-% und etwa 50 Gew.-% der Gesamtzusammensetzung vorhanden ist und das matrixbildende Mittel in einer Menge von etwa zwischen 50 Gew.-% und etwa 60 Gew.-% der Gesamtzusammensetzung vorhanden ist.

**3.** Pharmazeutische Zusammensetzung nach einem der vorangegangenen Ansprüche, wobei die pharmazeutische Zusammensetzung einen Auflösungsverbesserer umfasst, wobei der Auflösungsverbesserer gegebenenfalls aus der aus Poly(ethylenglykol)-Block-Poly(propylenglykol)-Block-Poly(ethylenglykol)-Copolymer, Polyethylenglykol-Copolymer, Stärke, Reisstärke, Lecithin, Polyvinylpyrrolidon (PVP), Polyoxylglyceriden, Polyvinylpyrrolidon-Vinylacetat-Copolymer (PVP-VA), Hydroxypropylmethylcellulose (HPMC), Hypromelloseacetatsuccinat (HPMCAS), niedersubstituierter Hydroxypropylcellulose (L-HPC), Sorbitanestern, Polyethylenglykol, Sorbitanfettsäureestern und Kombinationen daraus bestehenden Gruppe ausgewählt ist,

wobei der Auflösungsverbesserer gegebenenfalls in einer Menge von etwa 1 Gew.-% bis etwa 20 Gew.-% der Gesamtzusammensetzung vorhanden ist und
wobei der Auflösungsverbesserer gegebenenfalls Poly(ethylenglykol)-Block-Poly(propylenglykol)-Block-Poly(ethylenglykol)-Copolymer ist.

**4.** Pharmazeutische Zusammensetzung nach einem der vorangegangenen Ansprüche, wobei die pharmazeutische Zusammensetzung eine Strömungshilfe umfasst,

wobei die Strömungshilfe gegebenenfalls aus der aus amorphem Siliciumdioxidgel, Siliciumdioxidaerogel, Magnesiumaluminometasilikaten, Calciumsilikat, geordnetem mesoporösem Siliciumdioxid, mikronisiertem Talk und Kombinationen daraus bestehenden Gruppe ausgewählt ist,
wobei die Strömungshilfe gegebenenfalls in einer Menge von 0,1 Gew.-% bis 5 Gew.-% der Gesamtzusammensetzung vorhanden ist.

**5.** Pharmazeutische Zusammensetzung nach einem der vorangegangenen Ansprüche, wobei die pharmazeutische Zusammensetzung ein Versteifungsmittel umfasst,

wobei das Versteifungsmittel gegebenenfalls aus der aus Carnaubawachs, Candelillawachs und Kombinationen daraus bestehenden Gruppe ausgewählt ist,
wobei das Versteifungsmittel gegebenenfalls in einer Menge von 1 Gew.-% bis 60 Gew.-% der Gesamtzusammensetzung vorhanden ist.

**6.** Pharmazeutische Zusammensetzung nach einem der vorangegangenen Ansprüche, wobei die Zusammensetzung aus diskreten Teilchen, einschließlich des Capryltriglycerids und des Glycerids, besteht, wobei die Teilchen gegebenenfalls einen mittleren Durchmesser, wie gemäß der Beschreibung gemessen, von zwischen etwa 50 μm und etwa 350 μm oder von zwischen etwa 100 μm und etwa 300 μm aufweisen, wobei die Teilchen gegebenenfalls einen Carr-Index der Strömbarkeit von weniger als 10 % aufweisen.

**7.** Zusammensetzung nach einem der vorangegangenen Ansprüche, wobei die Reinheit des Capryltriglycerids zumindest 95 % beträgt.

**8.** Pharmazeutische Zusammensetzung nach einem der vorangegangenen Ansprüche zur Verwendung in einem Verfahren zur Behandlung einer Erkrankung oder Störung in Zusammenhang mit reduzierter kognitiver Funktion, wobei das Verfahren das Verabreichen der Zusammensetzung an das Individuum in einer Menge umfasst, die wirksam ist, um die Ketonkörperkonzentrationen in dem Individuum zu erhöhen, um dadurch die Erkrankung oder Störung zu behandeln,
wobei das Verfahren gegebenenfalls weiters das Bestimmen umfasst, ob dem Patienten der ApoE4-Genotyp fehlt.

**9.** Pharmazeutische Zusammensetzung zur Verwendung nach Anspruch 8, wobei die Erkrankung oder die Störung in Zusammenhang mit reduzierter kognitiver Funktion aus Morbus Alzheimer und altersbedingter Gedächtnisverschlechterung ausgewählt ist.

**10.** Pharmazeutische Zusammensetzung zur Verwendung nach Anspruch 8 oder Anspruch 9, wobei die Zusammensetzung in einer Dosis von etwa 0,05 g/kg/Tag bis etwa 10 g/kg/Tag verabreicht wird.

## Revendications

**1.** Formulation pharmaceutique comprenant entre 30 % et 50 % en poids de la composition totale de triglycéride caprylique, et au moins un agent de formation de matrice présent en une quantité comprise entre environ 20 % et environ 70 % en poids de la composition totale, dans laquelle le au moins un agent de formation de matrice est choisi dans le groupe comprenant dibéhénate de glycéryle, cire de carnauba, et des combinaisons de ceux-ci.

**2.** Composition pharmaceutique selon l'une quelconque des revendications précédentes, dans laquelle l'agent de formation de matrice est présent en une quantité comprise entre environ 40 % et environ 70 % en poids de la composition totale, facultativement dans laquelle le triglycéride caprylique est présent en une quantité comprise entre environ 40 % et environ 50 % en poids de la composition totale et l'agent de formation de matrice est présent en une quantité comprise entre environ 50 % et environ 60 % en poids de la composition totale

3. Composition pharmaceutique selon l'une quelconque des revendications précédentes, dans laquelle la composition pharmaceutique comprend un activateur de dissolution, facultativement dans laquelle l'activateur de dissolution est choisi dans le groupe comprenant un copolymère de poly(éthylène glycol)-bloc-poly(propylène glycol)-bloc-poly(éthylène glycol), un copolymère de polyéthylène glycol, de l'amidon, de l'amidon de riz, de la lécithine, de la polyvinylpyrrolidone (PVP), des polyoxylglycérides, un copolymère de polyvinylpyrrolidone-acétate de vinyle (PVP-VA), de l'hydroxypropylméthylcellulose (HPMC), de l'acétate-succinate d'hypromellose (HPMCAS), de l'hydroxypropylcellulose faiblement substituée (L-HPC), des esters de sorbitane, du polyéthylène glycol, des esters d'acides gras de sorbitane, et des combinaisons de ceux-ci, facultativement dans laquelle l'activateur de dissolution est présent en une quantité d'environ 1 % à environ 20 % en poids de la composition totale, et facultativement dans laquelle l'activateur de dissolution est un copolymère de poly(éthylène glycol)-bloc-poly(propylène glycol)-bloc-poly(éthylène glycol).

4. Composition pharmaceutique selon l'une quelconque des revendications précédentes, dans laquelle la composition pharmaceutique comprend un adjuvant d'écoulement, facultativement dans laquelle l'adjuvant d'écoulement est choisi dans le groupe constitué de gel de silice amorphe, d'aérogel de silice, d'alumino-métasilicates de magnésium, de silicate de calcium, de silice mésoporeuse ordonnée, de talc micronisé, et de combinaisons de ceux-ci, facultativement dans laquelle l'adjuvant d'écoulement est présent en une quantité de 0,1 % à 5 % en poids de la composition totale.

5. Composition pharmaceutique selon l'une quelconque des revendications précédentes, dans laquelle la composition pharmaceutique comprend un agent raidisseur, facultativement dans laquelle l'agent raidisseur est choisi dans le groupe constitué de la cire de carnauba, de la cire de candelilla, et de combinaisons de celles-ci, facultativement dans laquelle l'agent raidisseur est présent en une quantité de 1 % à 60 % en poids de la composition totale.

6. Composition pharmaceutique selon l'une quelconque des revendications précédentes, dans laquelle la composition est constituée de particules discrètes comprenant le triglycéride caprylique et le glycéride, facultativement dans laquelle les particules présentent un diamètre moyen, tel que mesuré selon la description, compris entre environ 50 $\mu$m et environ 350 $\mu$m, ou compris entre environ 100 $\mu$m et environ 300 $\mu$m, facultativement dans laquelle les particules présentent un indice de fluidité de Carr inférieur à 10 %.

7. Composition selon l'une quelconque des revendications précédentes, dans laquelle la pureté du triglycéride caprylique est d'au moins 95 %.

8. Composition pharmaceutique selon l'une quelconque des revendications précédentes à utiliser dans un procédé de traitement d'une maladie ou d'un trouble associé(e) à une fonction cognitive réduite, le procédé comprenant l'administration de la composition au sujet en une quantité efficace pour élever les concentrations de corps cétoniques chez ledit sujet afin de traiter ladite maladie ou ledit trouble, facultativement dans lequel le procédé comprend en outre l'étape consistant à déterminer si le patient est dépourvu du génotype ApoE4.

9. Composition pharmaceutique à utiliser selon la revendication 8, dans laquelle la maladie ou le trouble associé(e) à une fonction cognitive réduite est choisi(e) parmi la maladie d'Alzheimer et une altération de la mémoire associée à l'âge.

10. Composition pharmaceutique à utiliser selon la revendication 8 ou la revendication 9, dans laquelle la composition est administrée à une dose d'environ 0,05 g/kg/jour à environ 10 g/kg/jour.

FIG. 1A

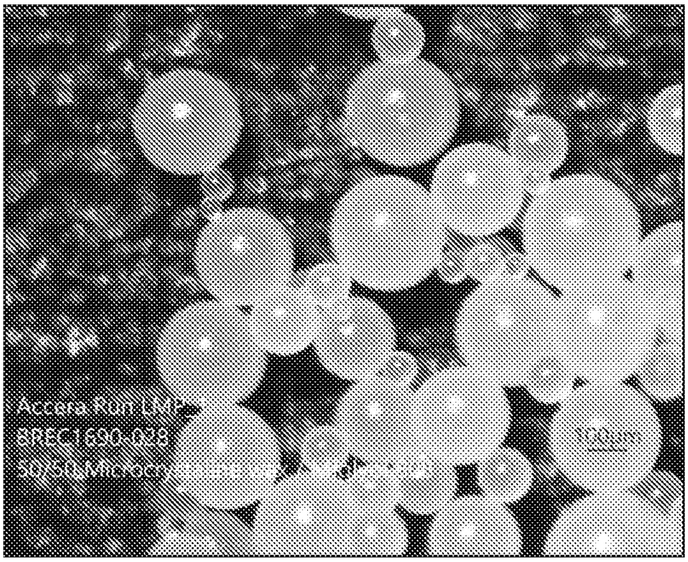


FIG. 1B

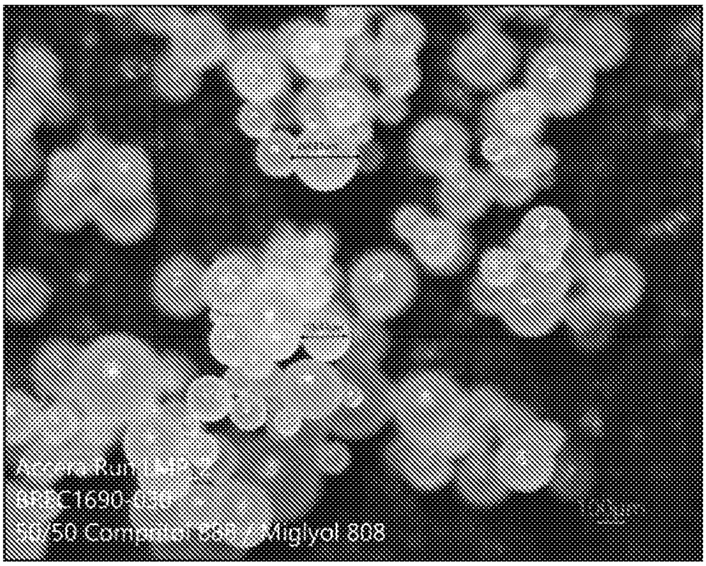

FIG. 1C

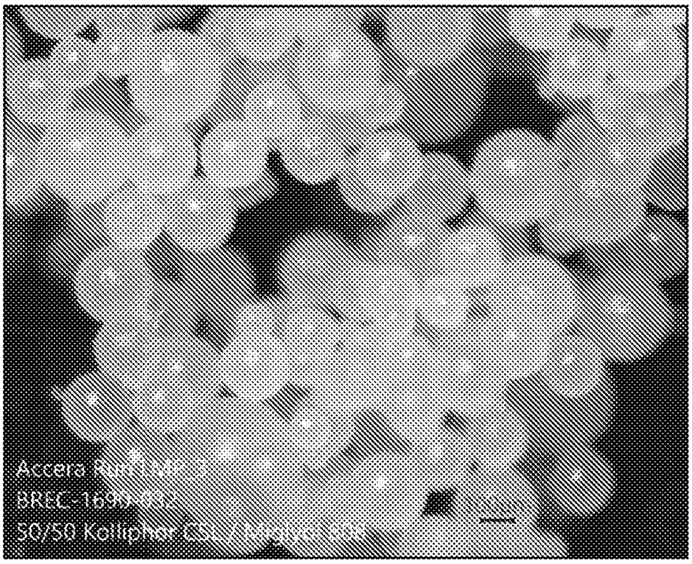

FIG. 1D

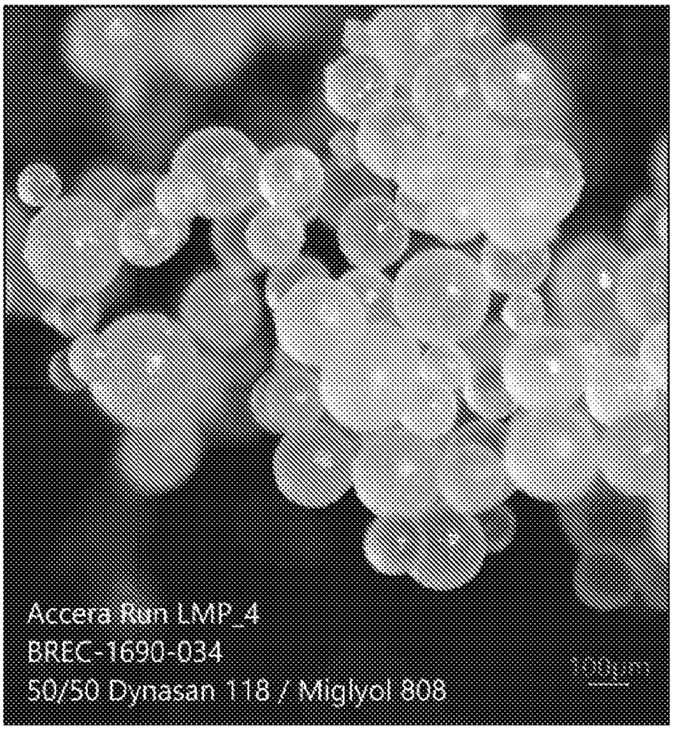
Accera Run LMP_4
BREC-1690-034
50/50 Dynasan 118 / Miglyol 808
100µm

FIGs. 2A-D.

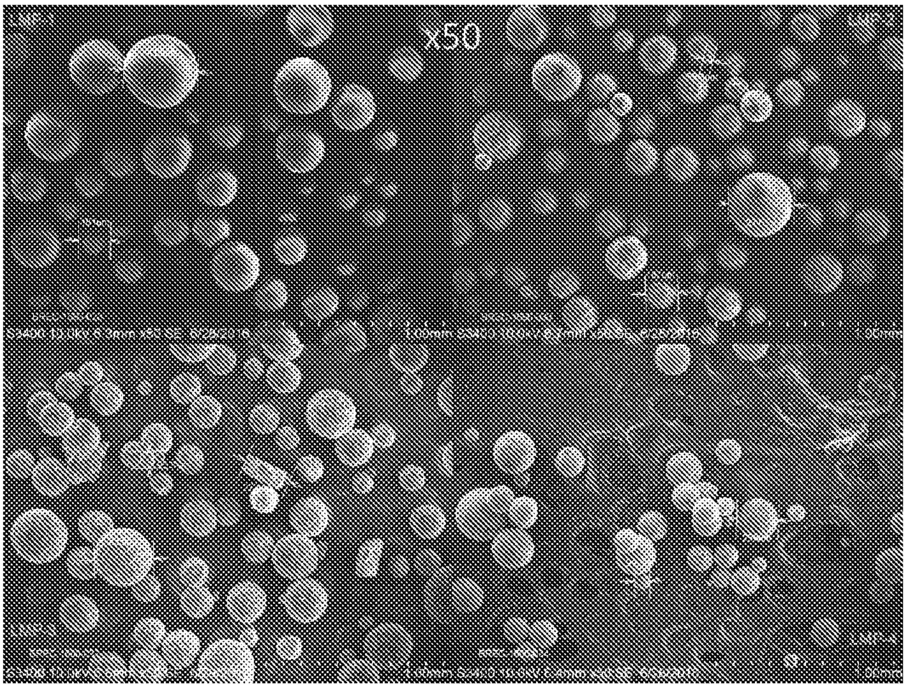


FIGs. 3A-D

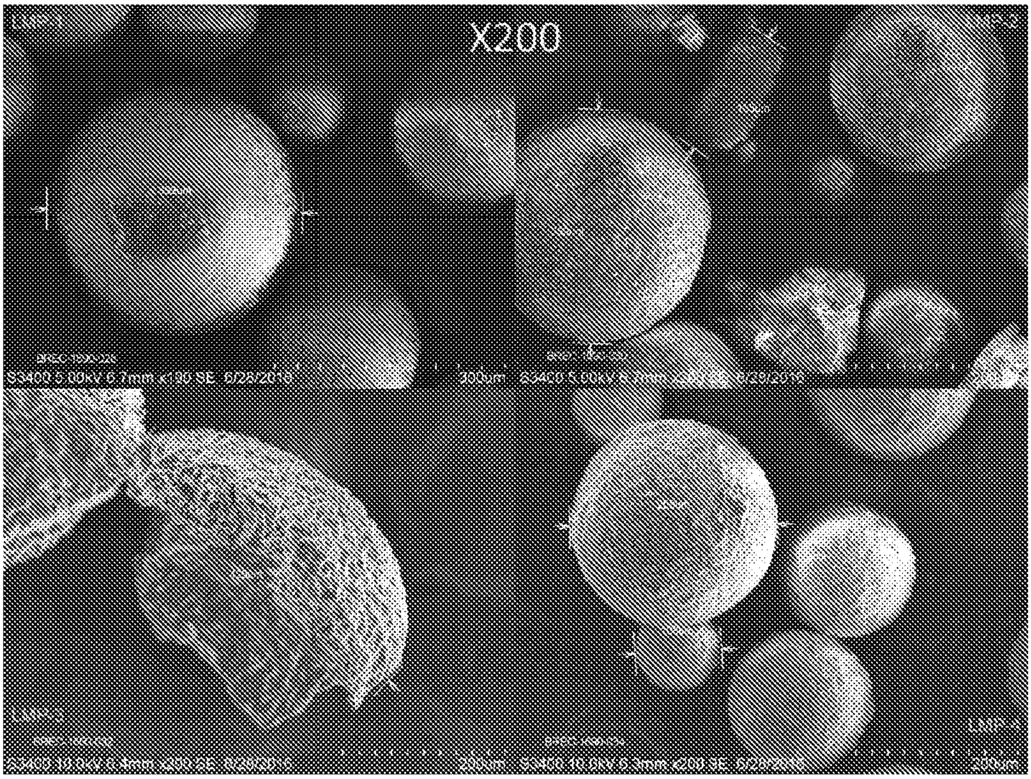

FIGs. 4A-D

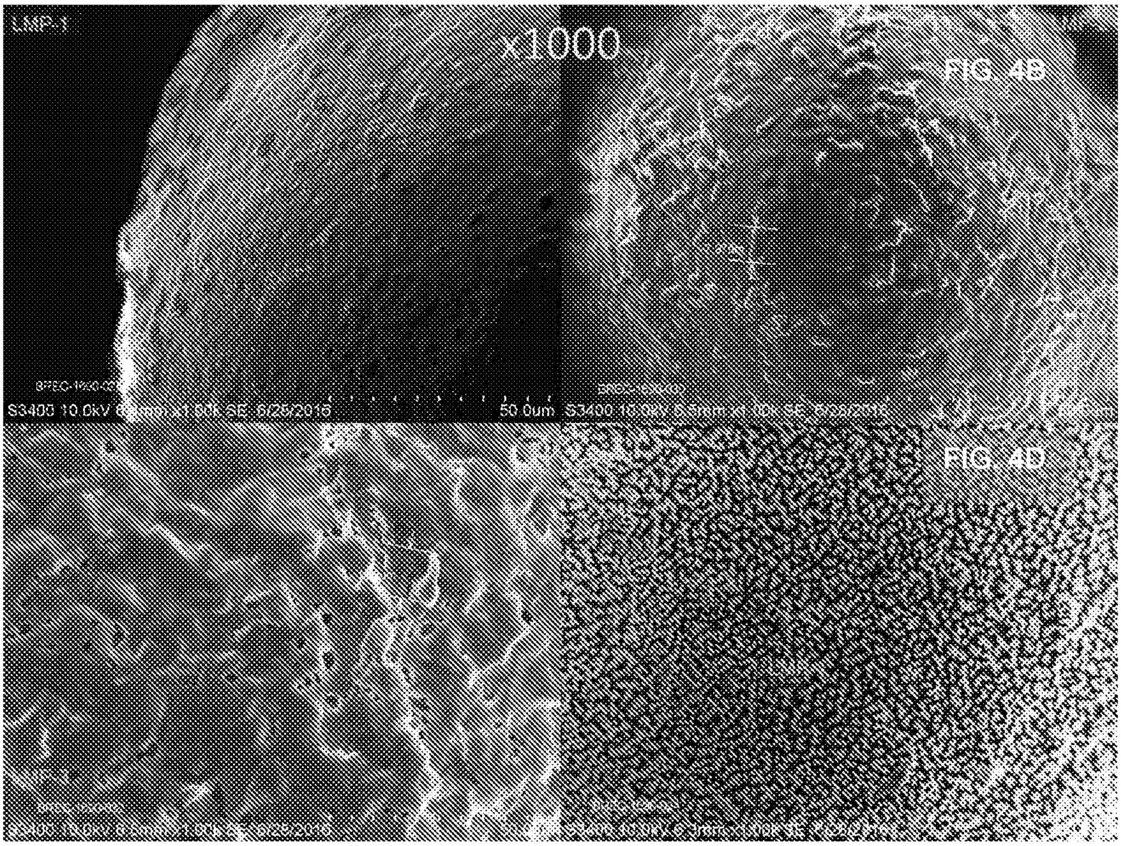

FIG. 5A

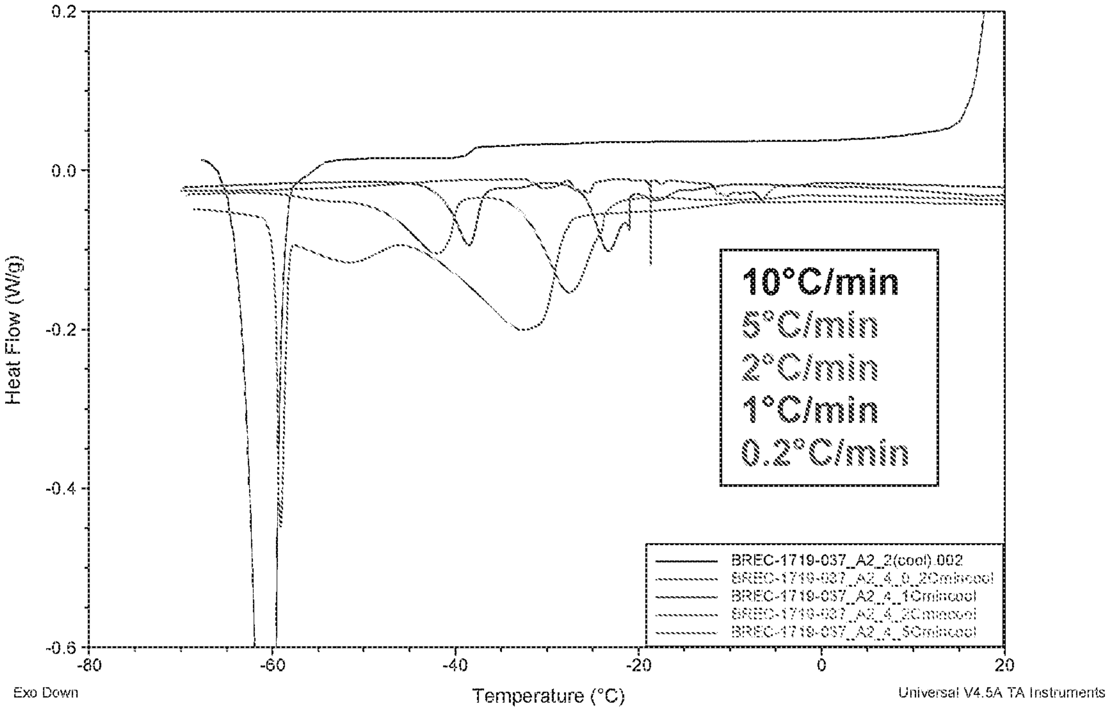
0.2
0.0
-0.2
-0.4
-0.6
Heat Flow (W/g)
10°C/min
5°C/min
2°C/min
1°C/min
0.2°C/min
BREC-1719-037_A2_2(cool).002
BREC-1719-037_A2_4_0_2Cmincool
BREC-1719-037_A2_4_1Cmincool
BREC-1719-037_A2_4_2Cmincool
BREC-1719-037_A2_4_5Cmincool
-80
-60
-40
-20
0
20
Exo Down
Temperature (°C)
Universal V4.5A TA Instruments

FIG. 5B

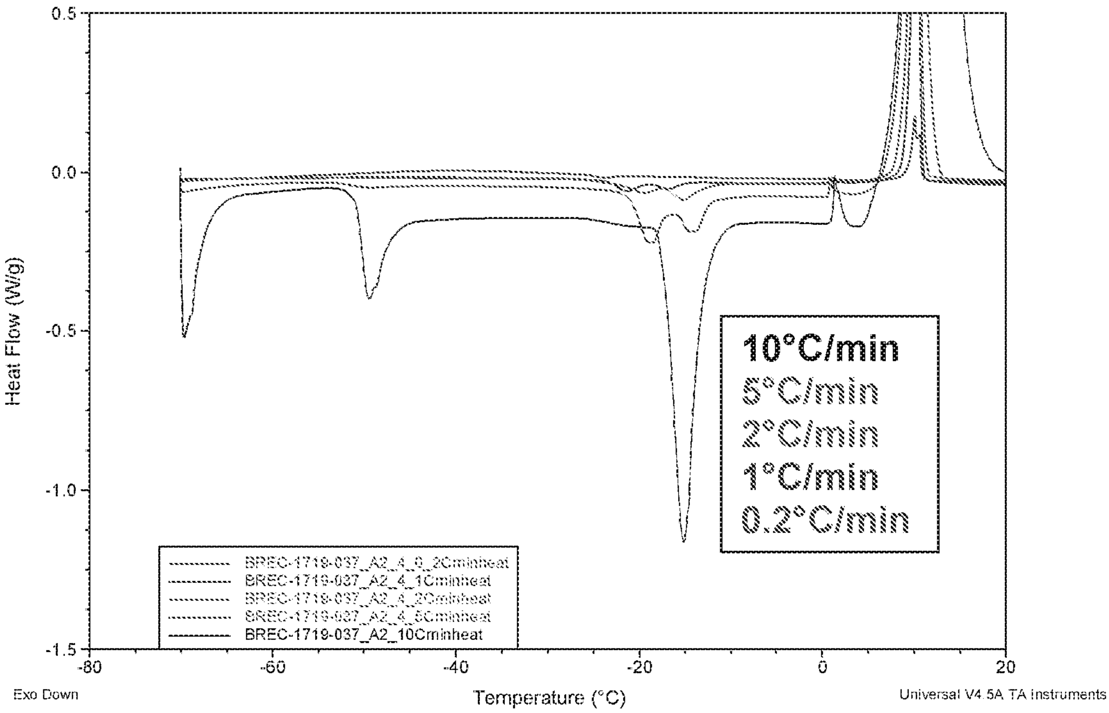
0.5
0.0
-0.5
-1.0
-1.5
Heat Flow (W/g)
10°C/min
5°C/min
2°C/min
1°C/min
0.2°C/min
BREC-1719-037_A2_4_0_2Cminheat
BREC-1719-037_A2_4_1Cminheat
BREC-1719-037_A2_4_2Cminheat
BREC-1719-037_A2_4_5Cminheat
BREC-1719-037_A2_10Cminheat
-80
-60
-40
-20
0
20
Exo Down
Temperature (°C)
Universal V4.5A TA Instruments

FIG. 5C

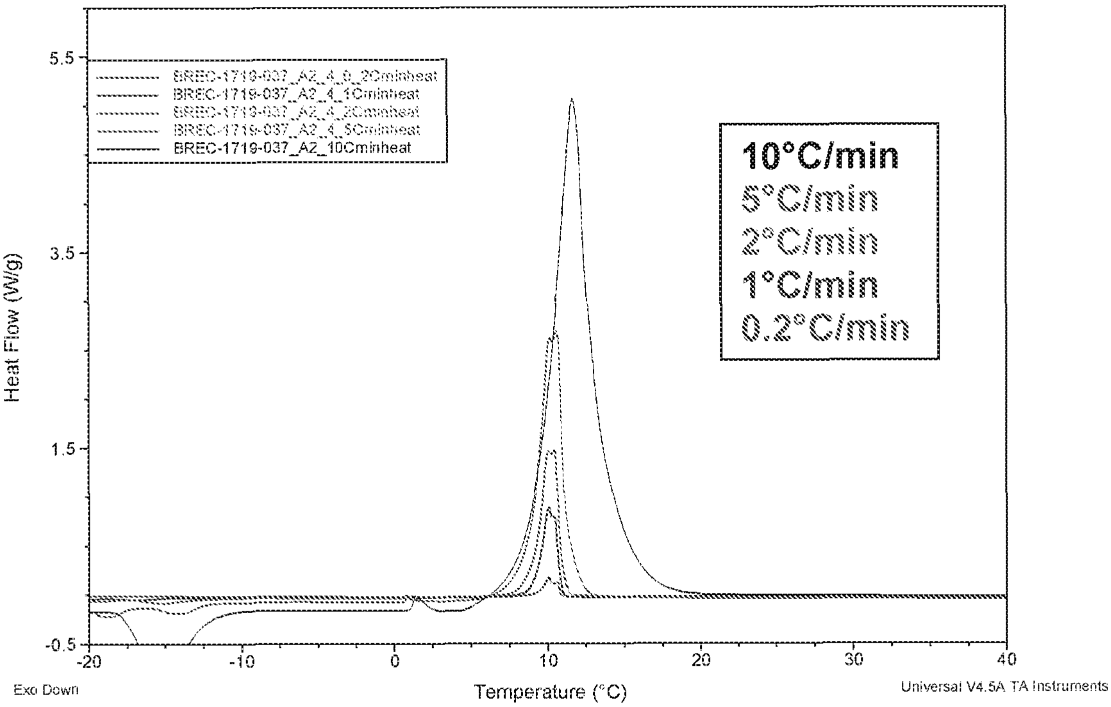
BREC-1719-037_A2_4_0_2Cminheat
BREC-1719-037_A2_4_1Cminheat
BREC-1719-037_A2_4_2Cminheat
BREC-1719-037_A2_4_5Cminheat
BREC-1719-037_A2_10Cminheat
10°C/min
5°C/min
2°C/min
1°C/min
0.2°C/min
5.5
3.5
1.5
-0.5
Heat Flow (W/g)
-20
-10
0
10
20
30
40
Exo Down
Temperature (°C)
Universal V4.5A TA Instruments

FIG. 6

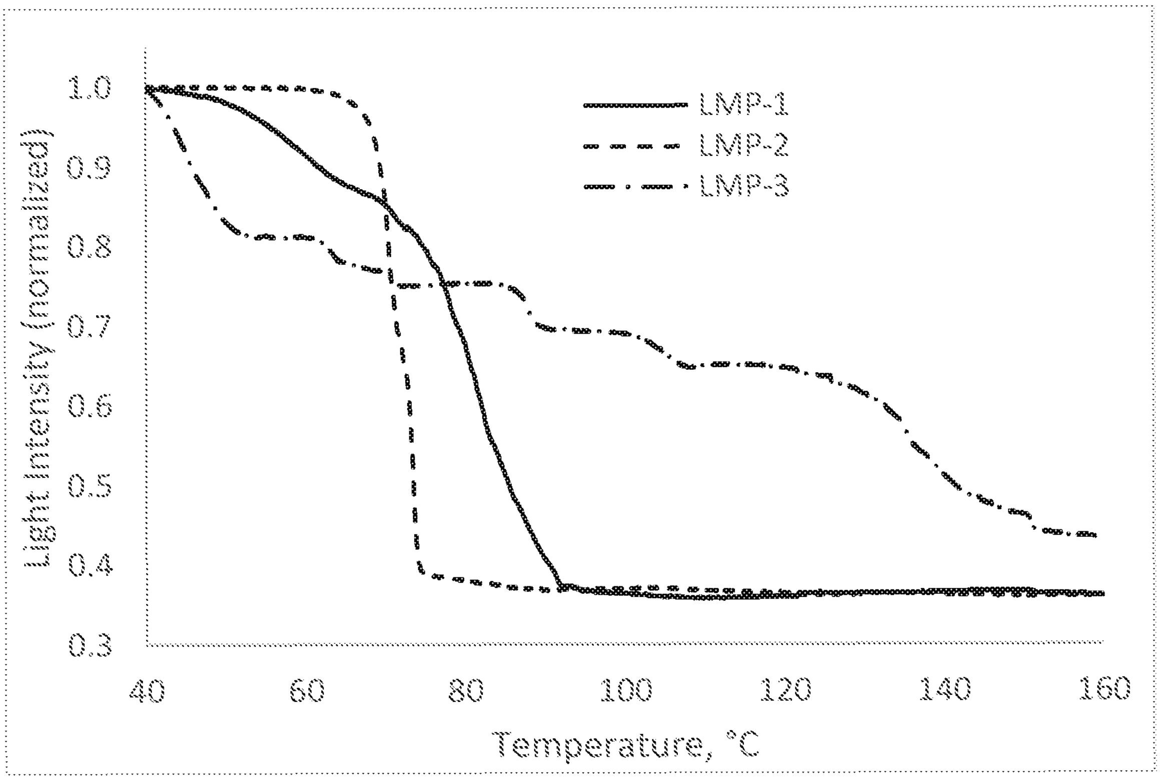
LMP-1
LMP-2
LMP-3
1.0
0.9
0.8
0.7
0.6
0.5
0.4
0.3
Light Intensity (normalized)
40
60
80
100
120
140
160
Temperature, °C

FIGs. 7A-C

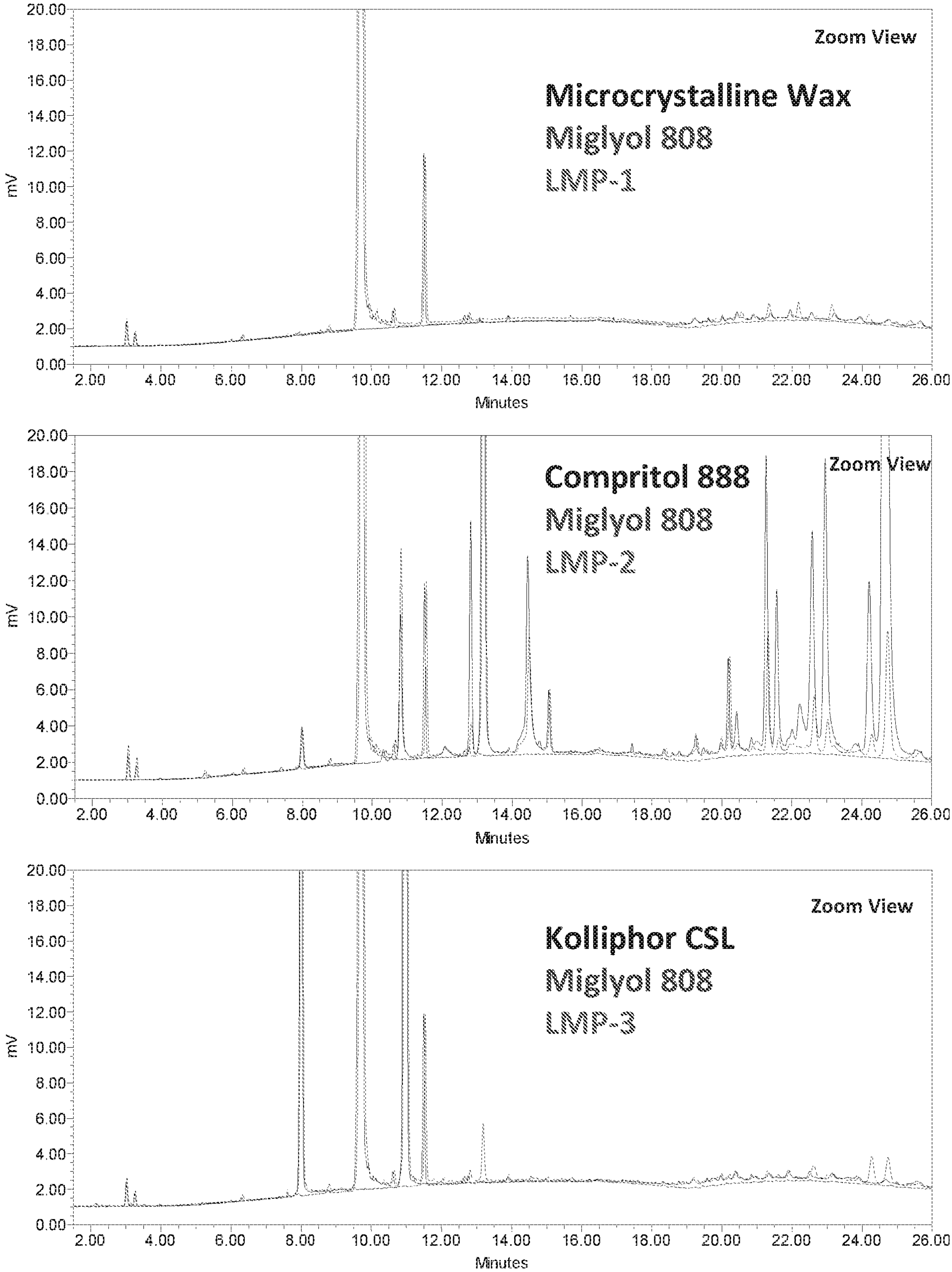
Zoom View
Microcrystalline Wax
Miglyol 808
LMP-1
mV
Minutes
Compritol 888
Miglyol 808
LMP-2
Zoom View
mV
Minutes
Zoom View
Kolliphor CSL
Miglyol 808
LMP-3
mV
Minutes

| | Body Temperature ~37°C | Room Temperature ~25°C | Cold Temperature ~5°C | NOTES/OBSERVATIONS |
|---|---|---|---|---|
| LMP-1 0.25% SLS solution | 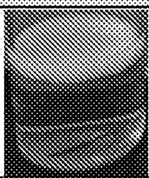 | 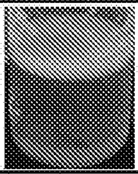 | 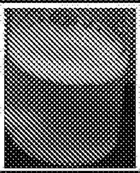 | Body temperature test sample still appears clear after the run |
| LMP-2 0.25% SLS solution | 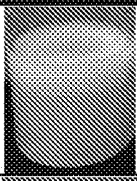 | 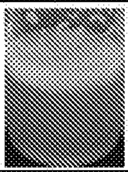 | 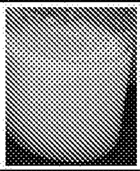 | The room temperature sample appears the 'clearest' |
| LMP-3 0.25% SLS solution | 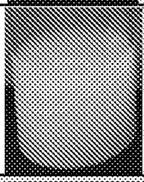 | 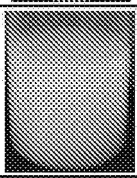 | 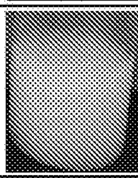 | The body temperature (~37°C) sample was much higher viscosity than the other temperatures |
| LMP-1 0.5% SLS solution | 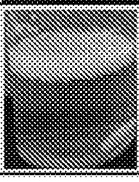 | 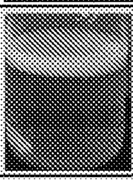 | 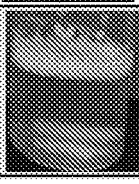 | 0.5% SLS dissolution test with LMP-1 visually appears the same across all temperatures |

FIG. 8

| 0.25% SLS/water solution 'CONTROL' | 250mg Miglyol 808 in 0.25% SLS/Water solution | 250mg Miglyol 808 in 0.5% SLS/Water solution | |
|---|---|---|---|
| 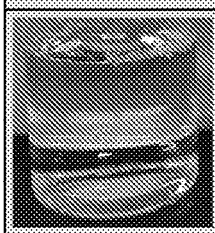 | 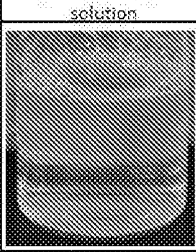 | 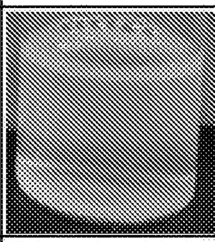 | Photos approximately 10 minutes after stirring |
| 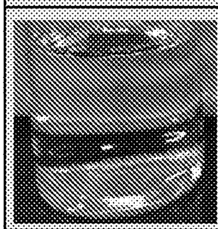 | 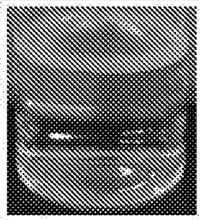 | 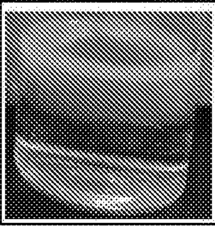 | Photos after sitting all weekend |
| 250mg of Miglyol 808 was added to each of the 0.25% SLS and the 0.5% SLS water solutions and stirred for 20 minutes. Left most column is pure 0.25% SLS/water solution without any oil. | | | |

FIG. 9

FIGs. 10A-D

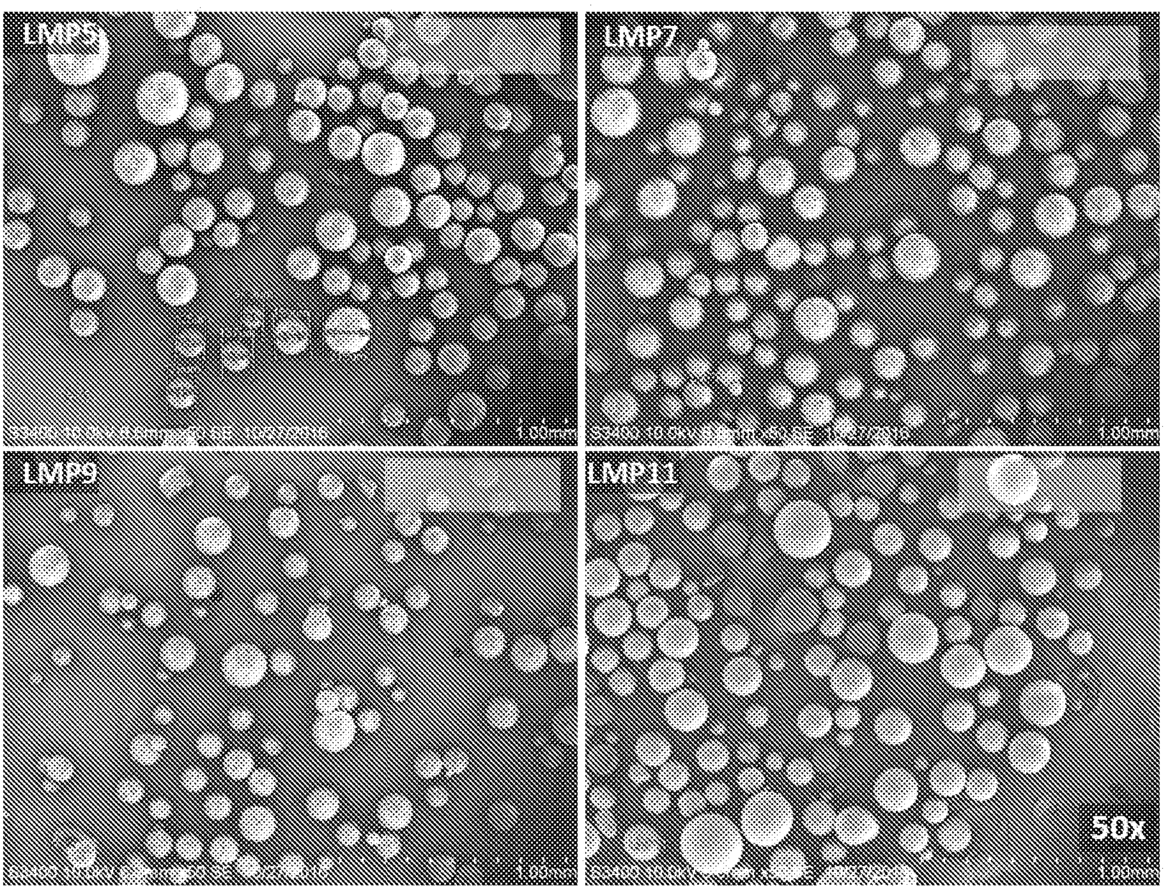

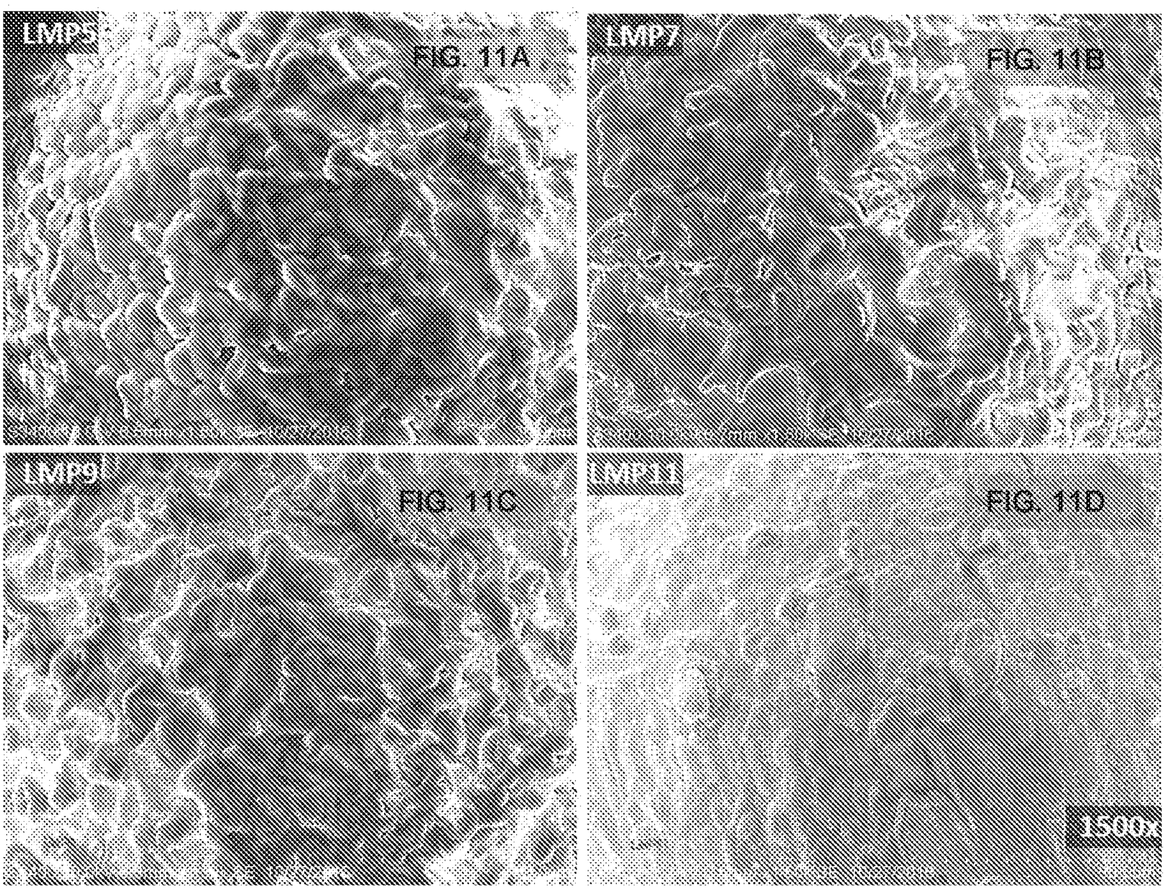


FIG. 11A

FIG. 11B

FIG. 11C

FIG. 11D

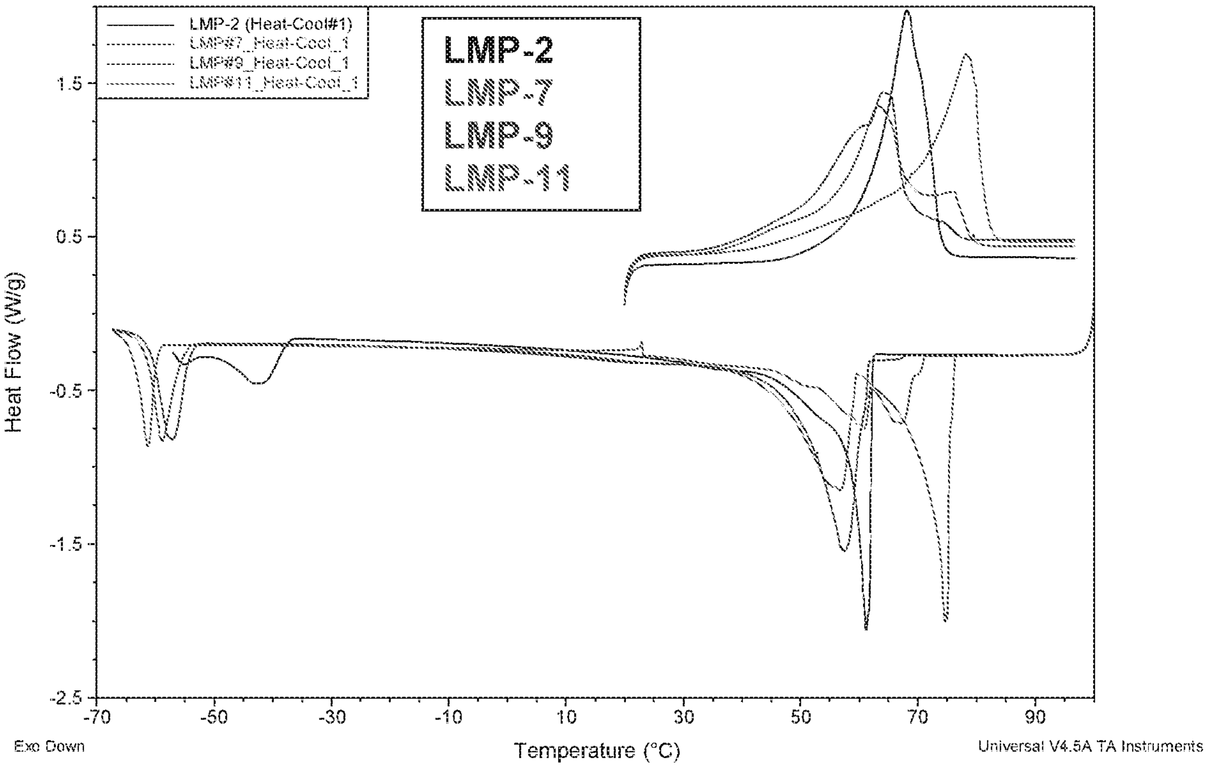
LMP-2 (Heat-Cool#1)
LMP#7_Heat-Cool_1
LMP#9_Heat-Cool_1
LMP#11_Heat-Cool_1
LMP-2
LMP-7
LMP-9
LMP-11
1.5
0.5
-0.5
-1.5
-2.5
Heat Flow (W/g)
-70
-50
-30
-10
10
30
50
70
90
Exo Down
Temperature (°C)
Universal V4.5A TA Instruments

FIG. 12

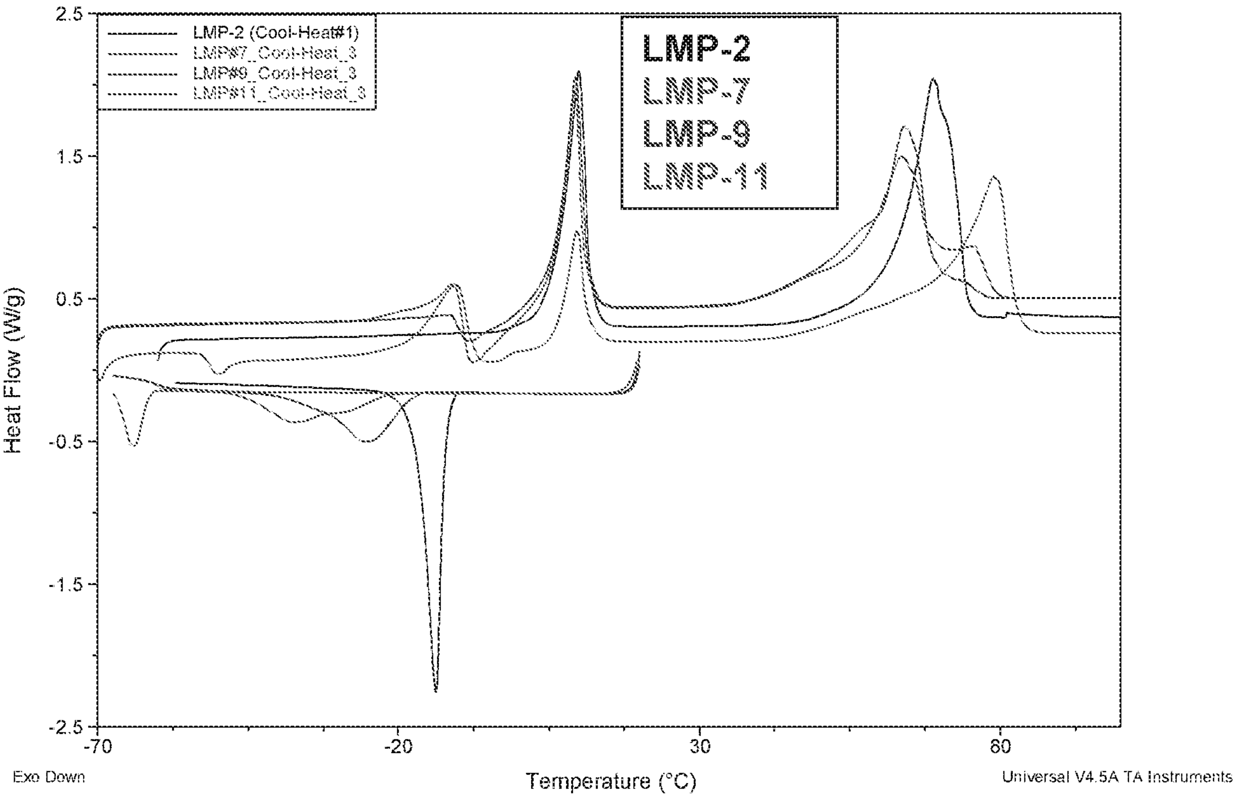
LMP-2 (Cool-Heat#1)
LMP#7_Cool-Heat_3
LMP#9_Cool-Heat_3
LMP#11_Cool-Heat_3
LMP-2
LMP-7
LMP-9
LMP-11
2.5
1.5
0.5
-0.5
-1.5
-2.5
-70
-20
30
80
Heat Flow (W/g)
Temperature (°C)
Exo Down
Universal V4.5A TA Instruments

FIG. 13

FIGs. 14A-H

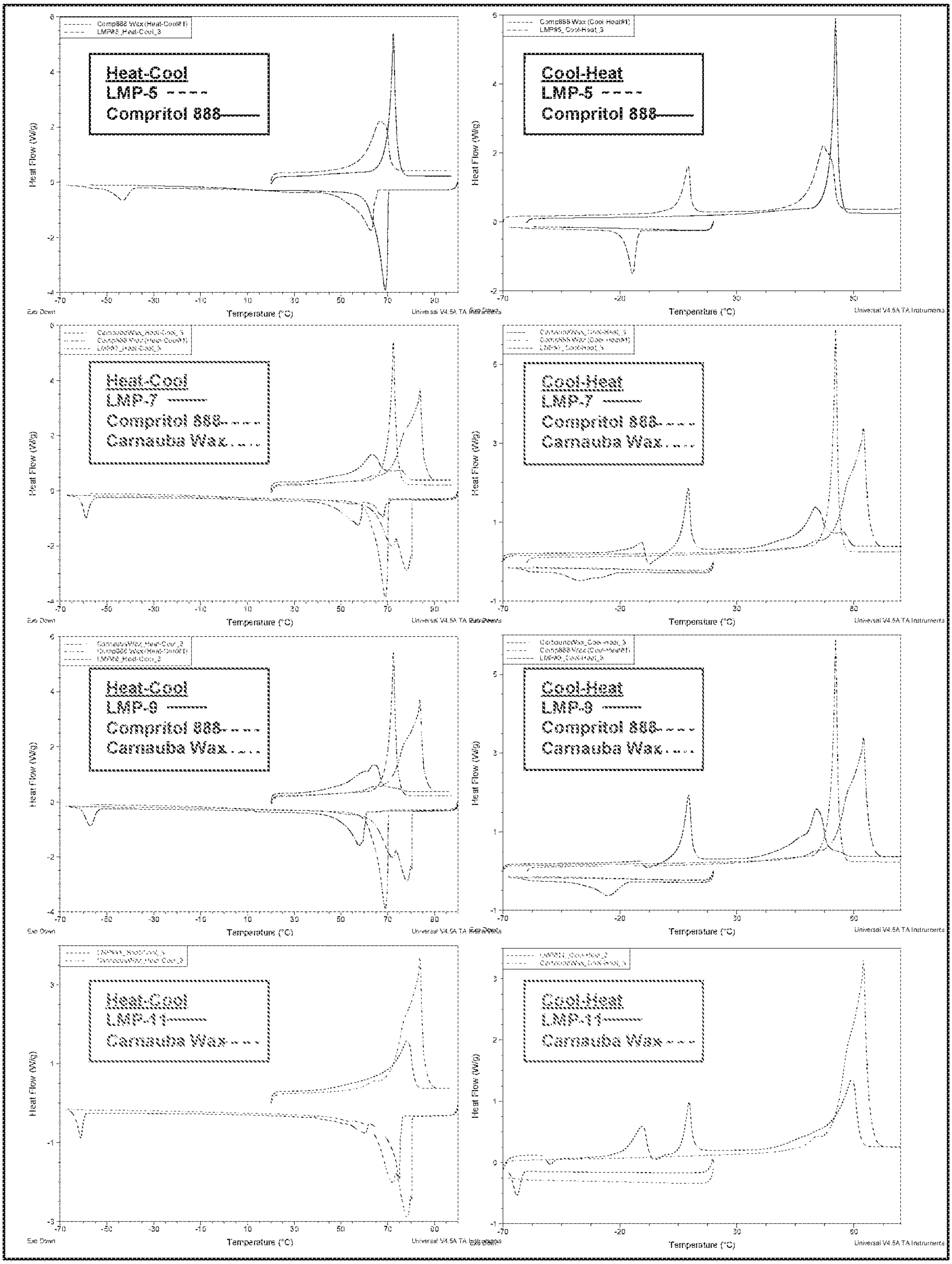
Heat-Cool
LMP-5
Compritol 888
Cool-Heat
LMP-5
Compritol 888
Heat-Cool
LMP-7
Compritol 888
Carnauba Wax
Cool-Heat
LMP-7
Compritol 888
Carnauba Wax
Heat-Cool
LMP-9
Compritol 888
Carnauba Wax
Cool-Heat
LMP-9
Compritol 888
Carnauba Wax
Heat-Cool
LMP-11
Carnauba Wax
Cool-Heat
LMP-11
Carnauba Wax
Heat Flow (W/g)
Temperature (°C)

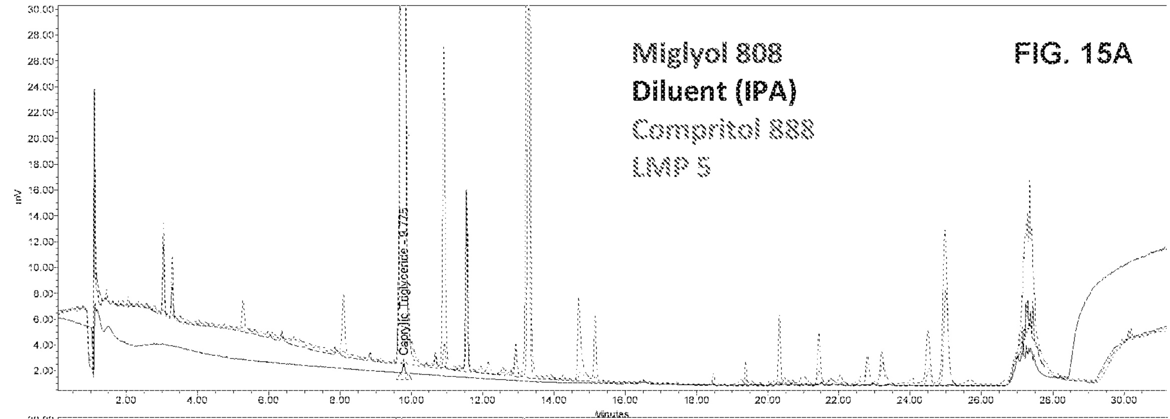

FIG. 15A

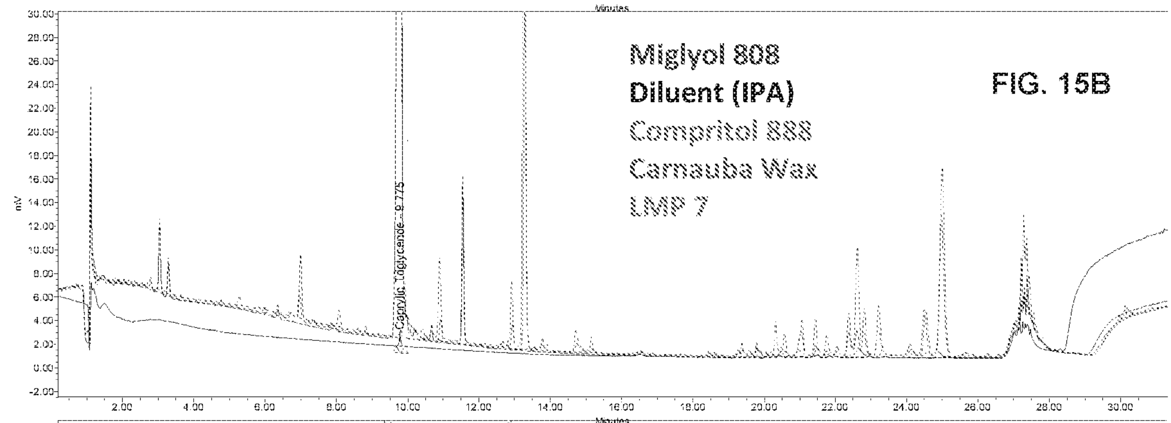

FIG. 15B

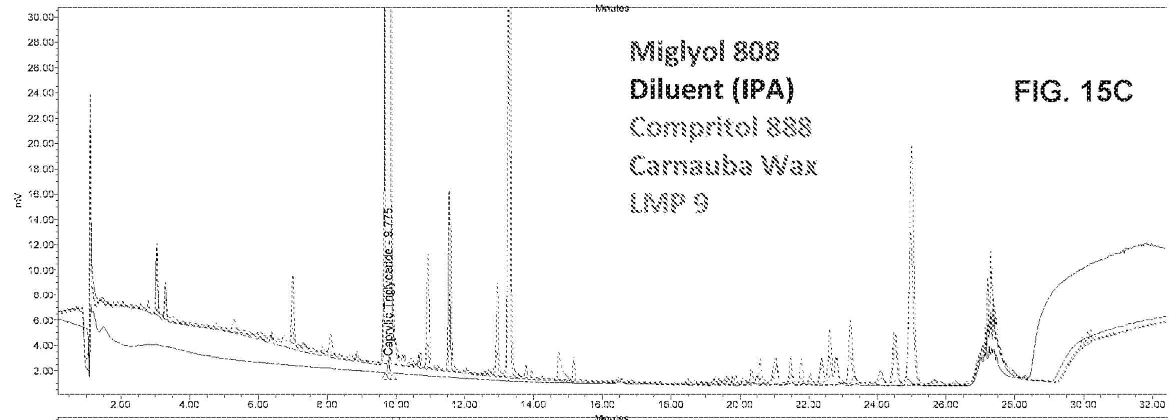

FIG. 15C

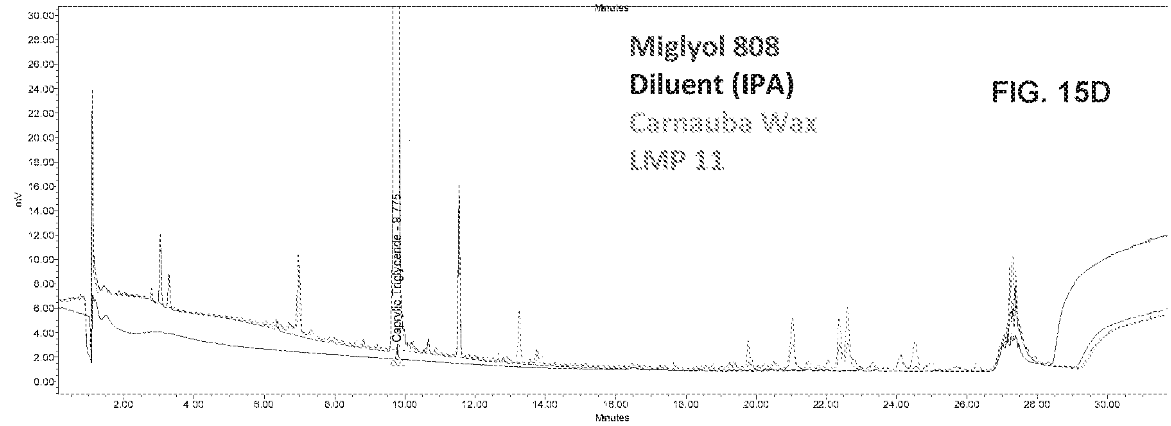

FIG. 15D

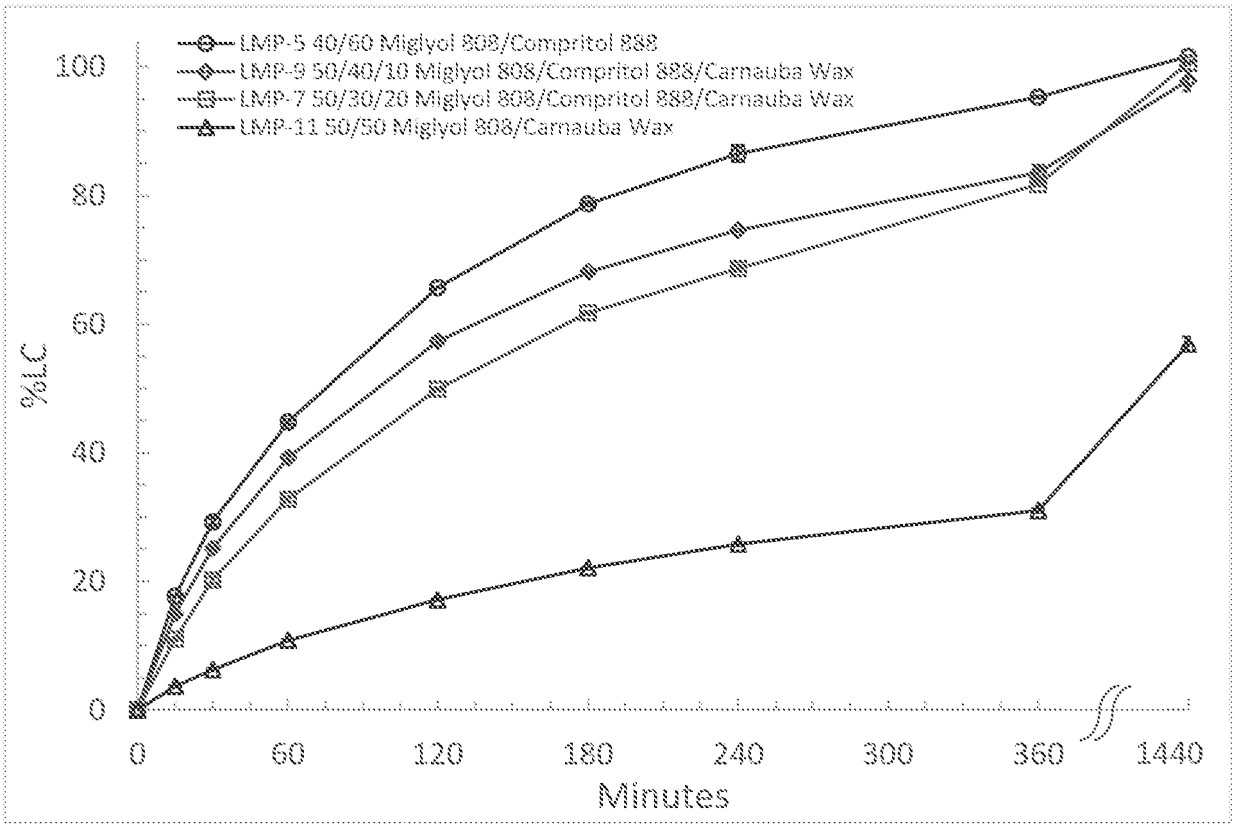
LMP-5 40/60 Migiyol 808/Compritol 888
LMP-9 50/40/10 Miglyol 808/Compritol 888/Carnauba Wax
LMP-7 50/30/20 Miglyol 808/Compritol 888/Carnauba Wax
LMP-11 50/50 Migiyol 808/Carnauba Wax
%LC
0
20
40
60
80
100
0
60
120
180
240
300
360
1440
Minutes

FIG. 16

FIG. 17A

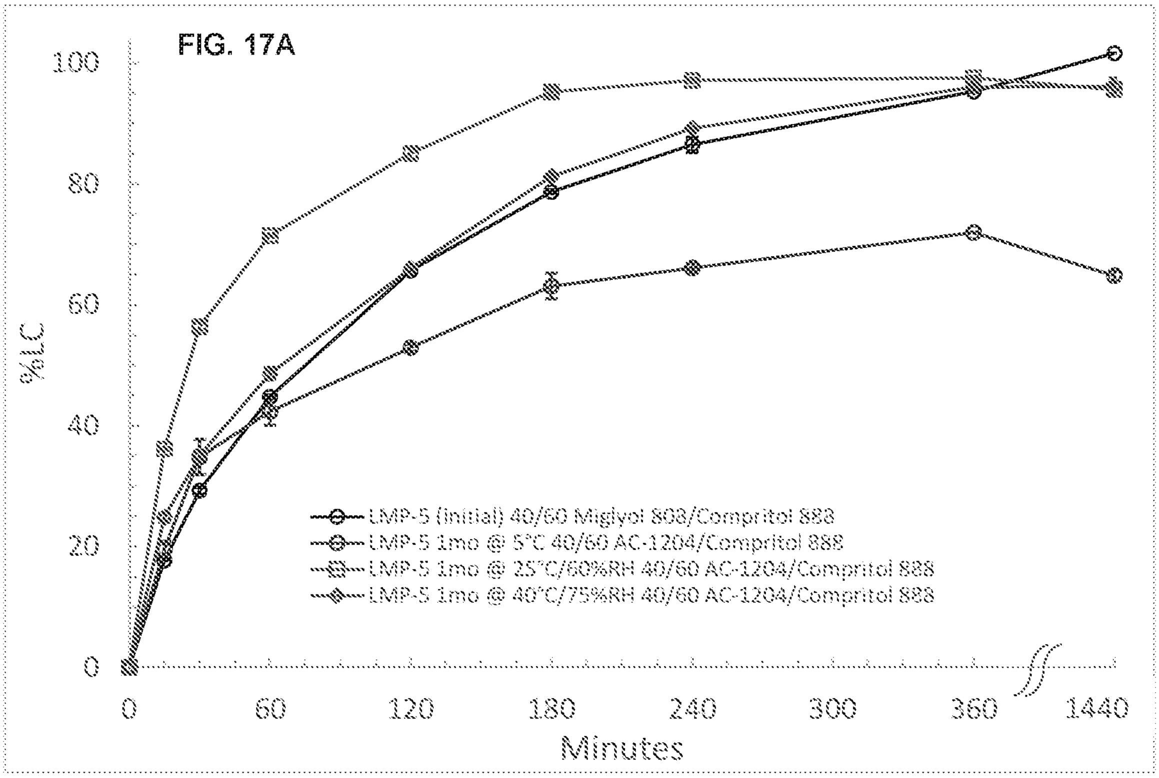


FIG. 17B

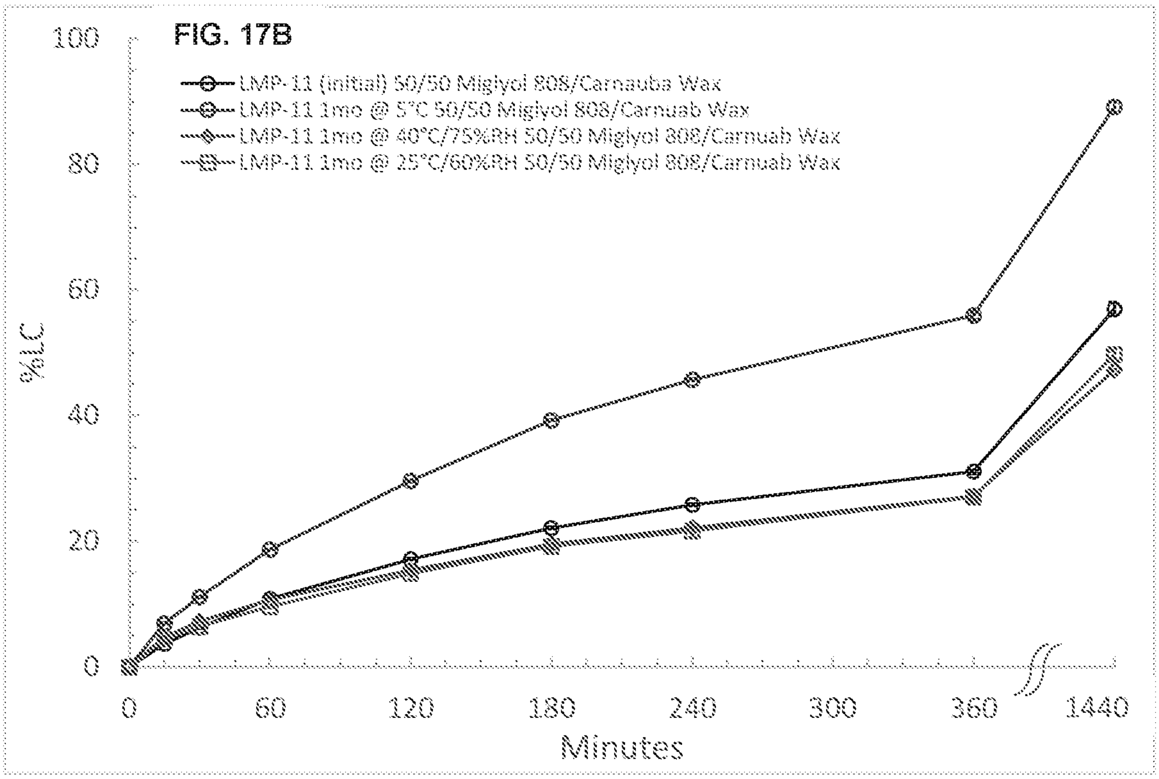

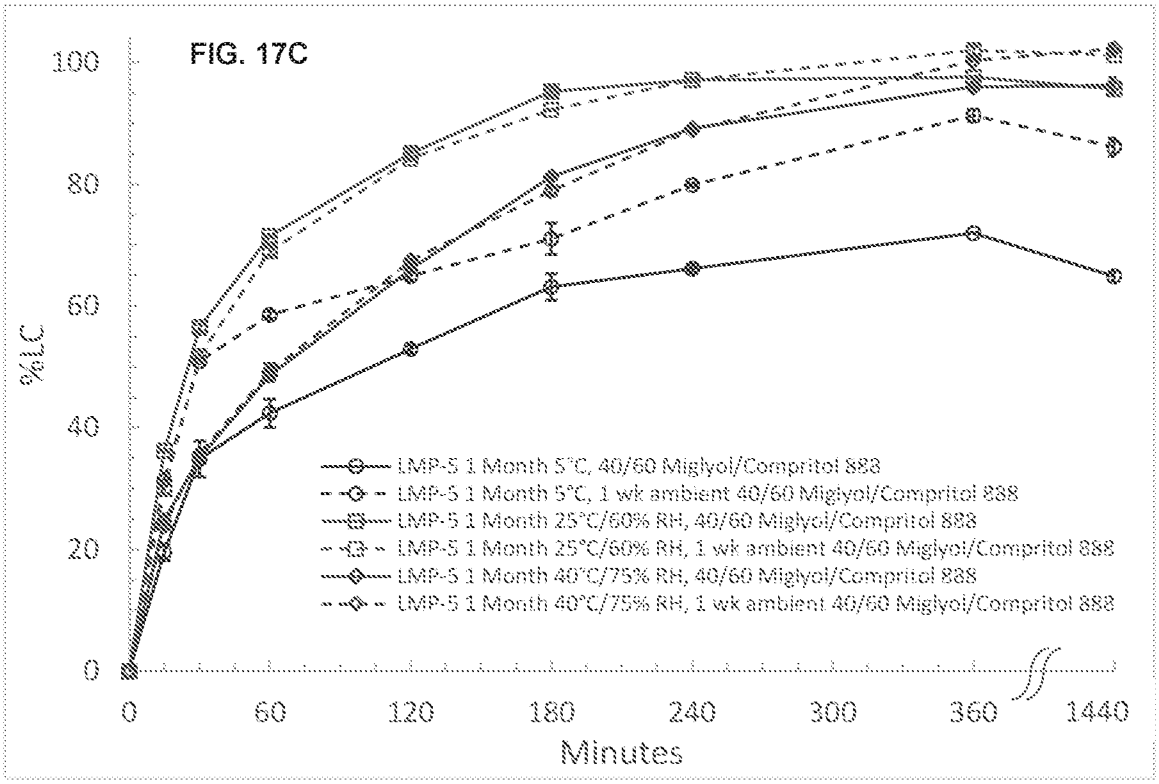
FIG. 17C
%LC
0
20
40
60
80
100
0
60
120
180
240
300
360
1440
Minutes
LMP-5 1 Month 5°C, 40/60 Miglyol/Compritol 888
LMP-5 1 Month 5°C, 1 wk ambient 40/60 Miglyol/Compritol 888
LMP-5 1 Month 25°C/60% RH, 40/60 Miglyol/Compritol 888
LMP-5 1 Month 25°C/60% RH, 1 wk ambient 40/60 Miglyol/Compritol 888
LMP-5 1 Month 40°C/75% RH, 40/60 Miglyol/Compritol 888
LMP-5 1 Month 40°C/75% RH, 1 wk ambient 40/60 Miglyol/Compritol 888

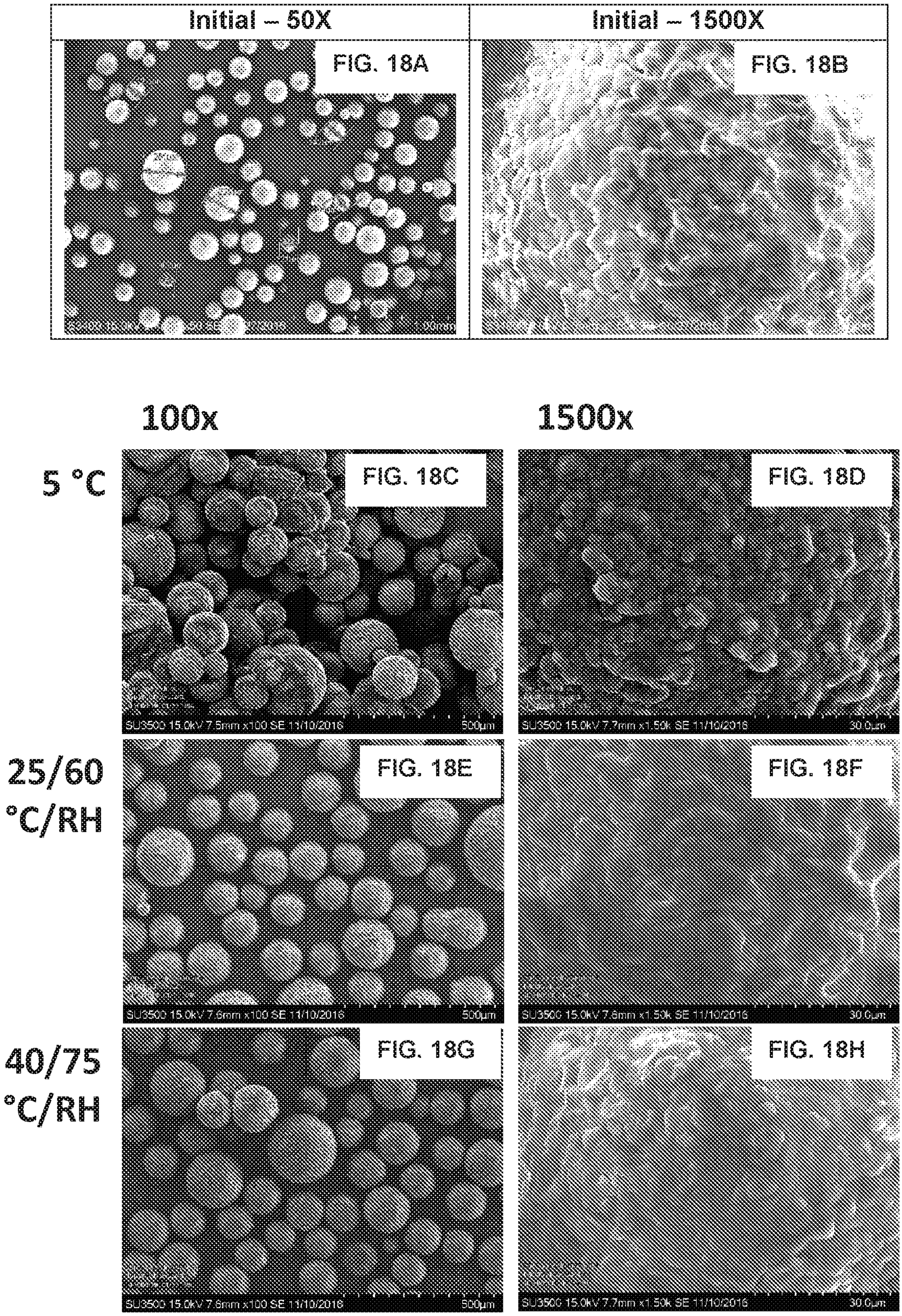
Initial – 50X
Initial – 1500X
FIG. 18A
FIG. 18B
100x
1500x
5 °C
FIG. 18C
FIG. 18D
SU3500 15.0kV 7.5mm x100 SE 11/10/2016
500µm
SU3500 15.0kV 7.7mm x1.50k SE 11/10/2016
30.0µm
25/60 °C/RH
FIG. 18E
FIG. 18F
SU3500 15.0kV 7.6mm x100 SE 11/10/2016
500µm
SU3500 15.0kV 7.6mm x1.50k SE 11/10/2016
30.0µm
40/75 °C/RH
FIG. 18G
FIG. 18H
SU3500 15.0kV 7.6mm x100 SE 11/10/2016
500µm
SU3500 15.0kV 7.7mm x1.50k SE 11/10/2016
30.0µm

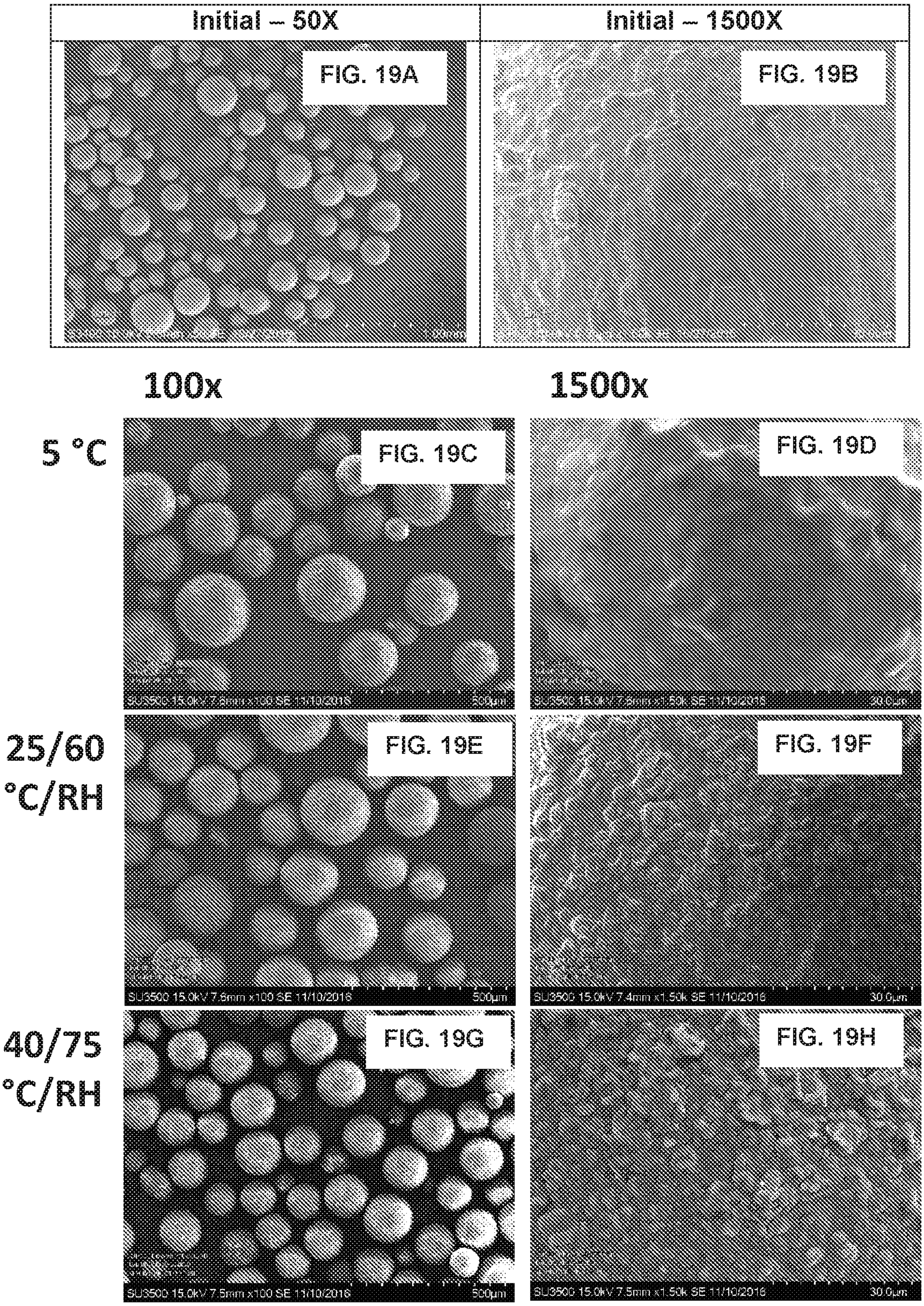


FIG. 19A
FIG. 19B
FIG. 19C
FIG. 19D
FIG. 19E
FIG. 19F
FIG. 19G
FIG. 19H

FIGs. 20A-H

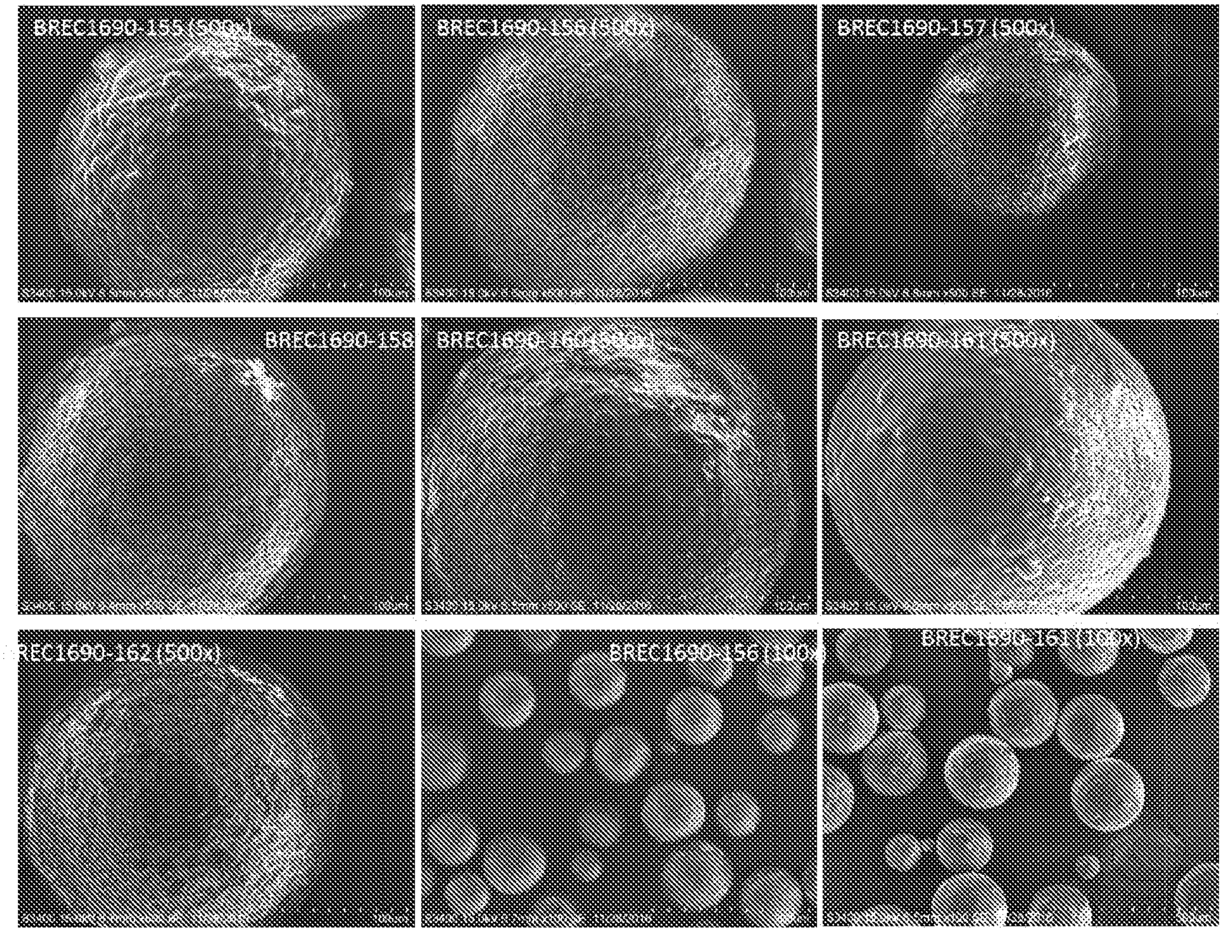
BREC1690-155 (500x)
BREC1690-156 (500x)
BREC1690-157 (500x)
BREC1690-158
BREC1690-160 (500x)
BREC1690-161 (500x)
REC1690-162 (500x)
BREC1690-156 (100x)
BREC1690-161 (100x)

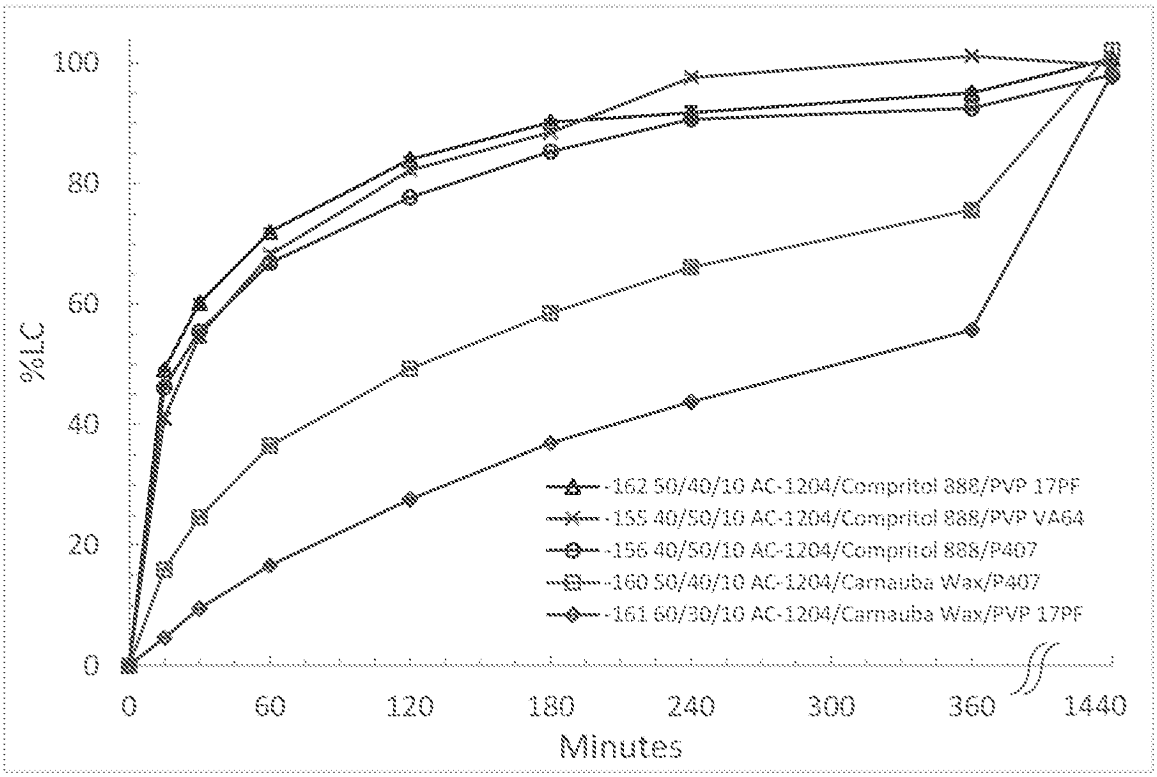
100
80
60
40
20
0
%LC
0
60
120
180
240
300
360
1440
Minutes
-162 50/40/10 AC-1204/Compritol 888/PVP 17PF
-155 40/50/10 AC-1204/Compritol 888/PVP VA64
-156 40/50/10 AC-1204/Compritol 888/P407
-160 50/40/10 AC-1204/Carnauba Wax/P407
-161 60/30/10 AC-1204/Carnauba Wax/PVP 17PF

FIG. 21

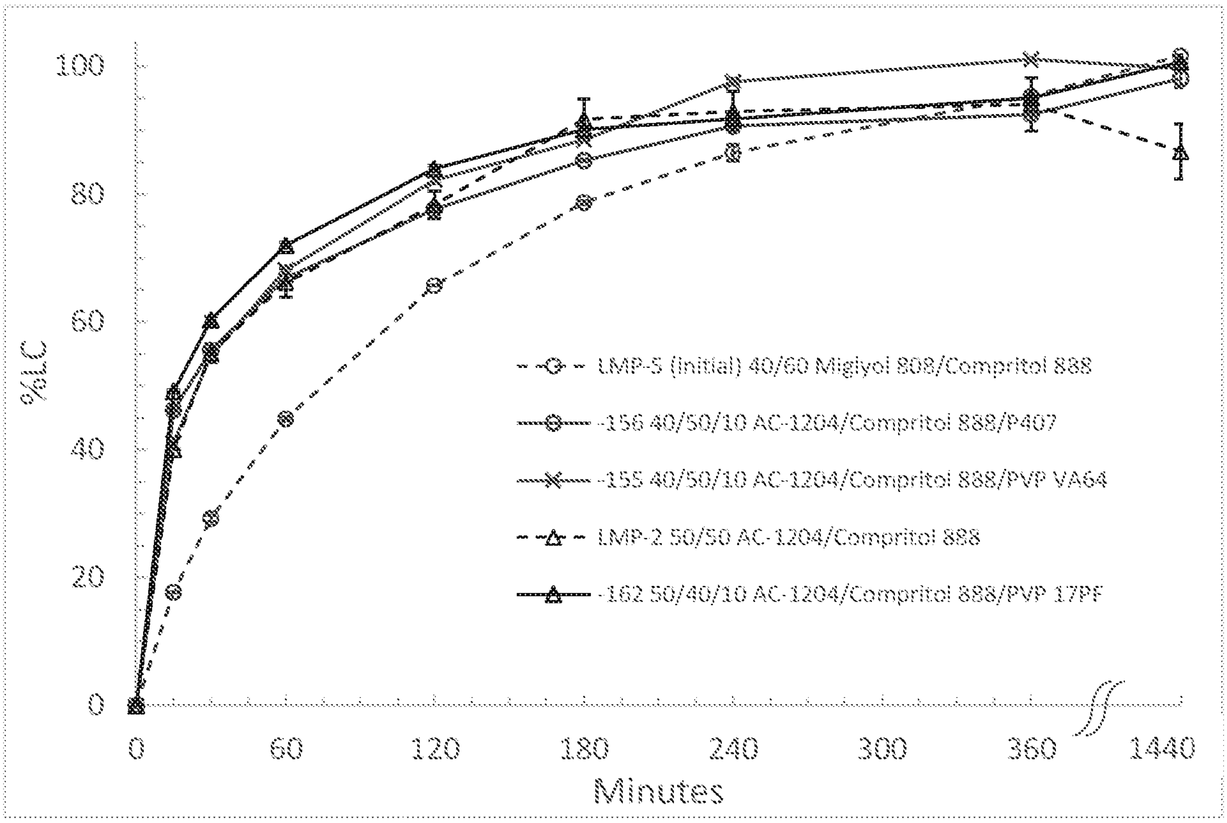
100
80
60
40
20
0
%LC
0
60
120
180
240
300
360
1440
Minutes
LMP-5 (initial) 40/60 Migiyol 808/Compritol 888
-156 40/50/10 AC-1204/Compritol 888/P407
-155 40/50/10 AC-1204/Compritol 888/PVP VA64
LMP-2 50/50 AC-1204/Compritol 888
-162 50/40/10 AC-1204/Compritol 888/PVP 17PF

FIG. 22

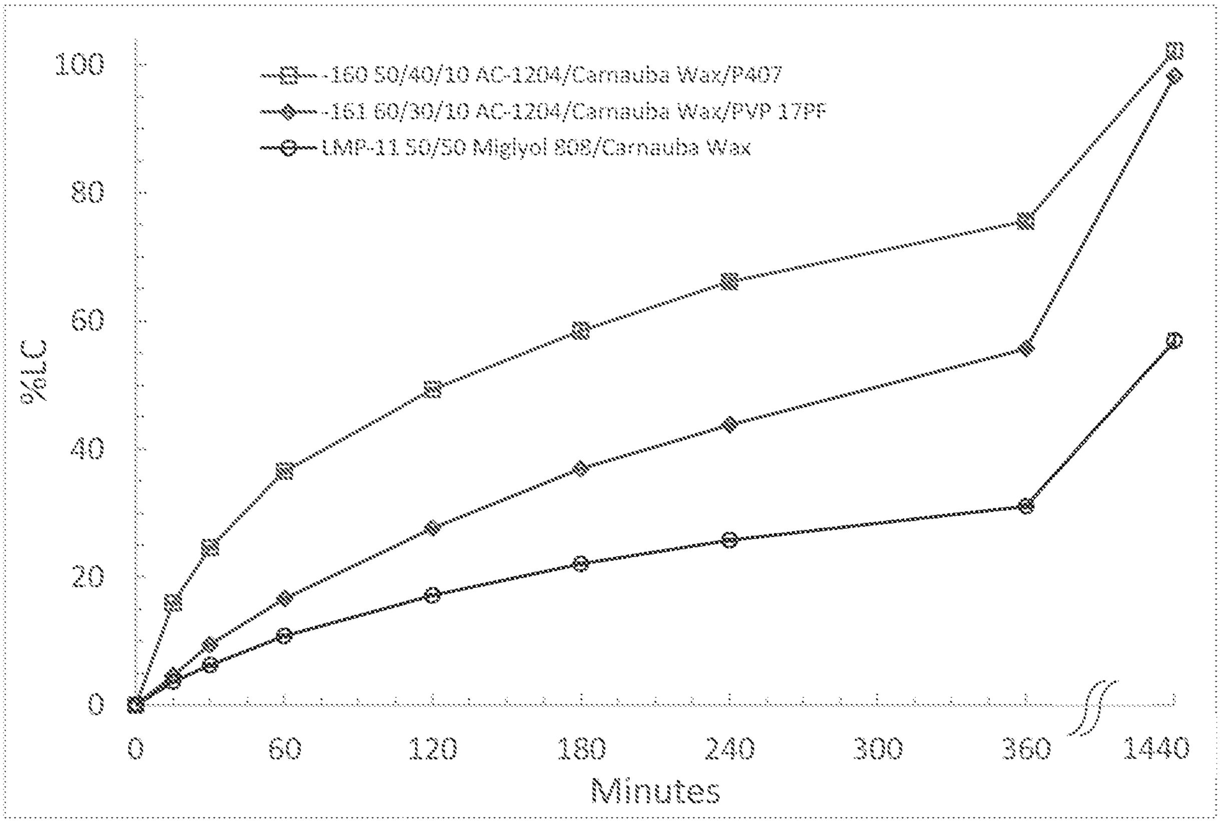
100
80
60
40
20
0
%LC
-160 50/40/10 AC-1204/Carnauba Wax/P407
-161 60/30/10 AC-1204/Carnauba Wax/PVP 17PF
LMP-11 50/50 Migiyol 808/Carnauba Wax
0
60
120
180
240
300
360
1440
Minutes

FIG. 23

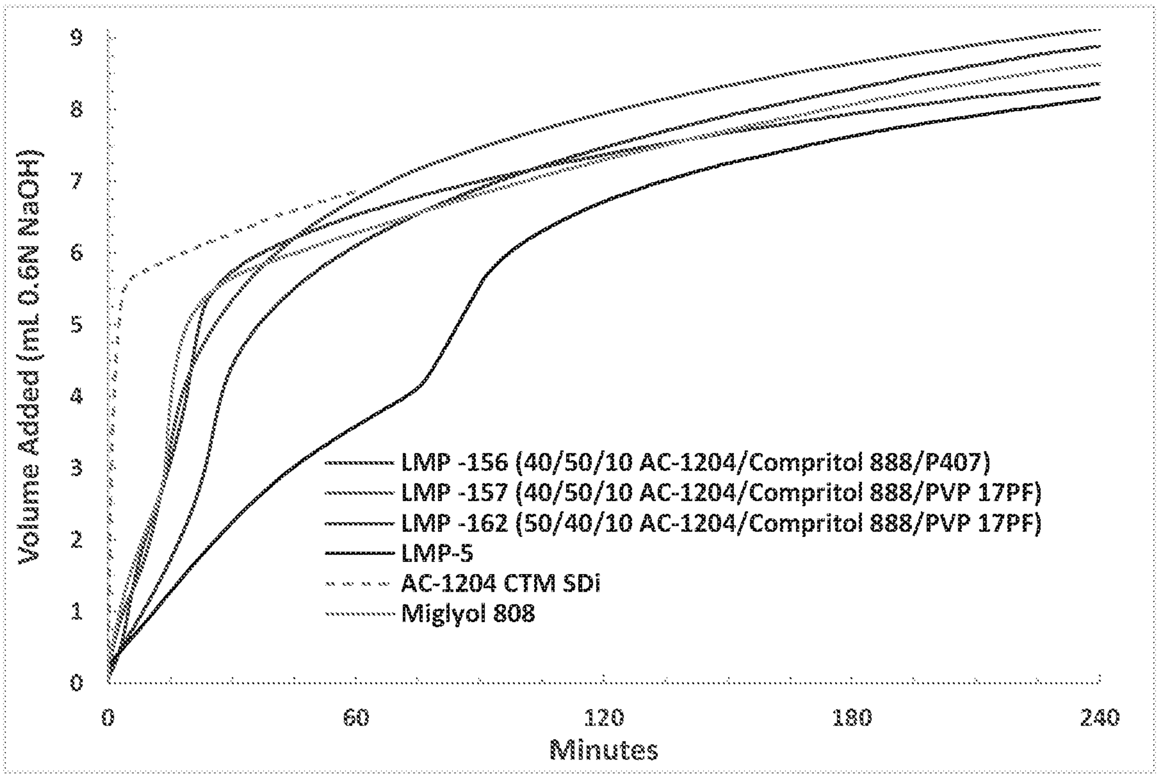

Volume Added (mL 0.6N NaOH)
0
1
2
3
4
5
6
7
8
9
0
60
120
180
240
Minutes
LMP -156 (40/50/10 AC-1204/Compritol 888/P407)
LMP -157 (40/50/10 AC-1204/Compritol 888/PVP 17PF)
LMP -162 (50/40/10 AC-1204/Compritol 888/PVP 17PF)
LMP-5
AC-1204 CTM SDi
Miglyol 808


FIG. 24

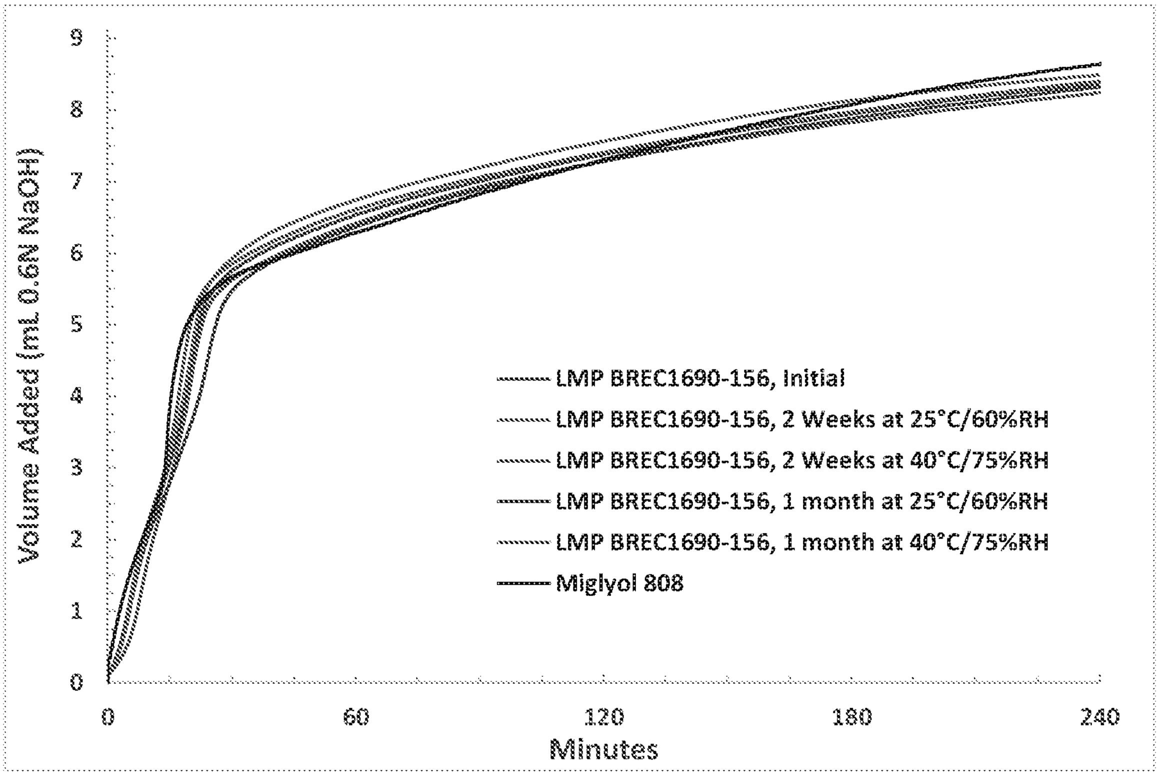

Volume Added (mL 0.6N NaOH)
0
1
2
3
4
5
6
7
8
9
0
60
120
180
240
Minutes
LMP BREC1690-156, Initial
LMP BREC1690-156, 2 Weeks at 25°C/60%RH
LMP BREC1690-156, 2 Weeks at 40°C/75%RH
LMP BREC1690-156, 1 month at 25°C/60%RH
LMP BREC1690-156, 1 month at 40°C/75%RH
Miglyol 808


FIG. 25

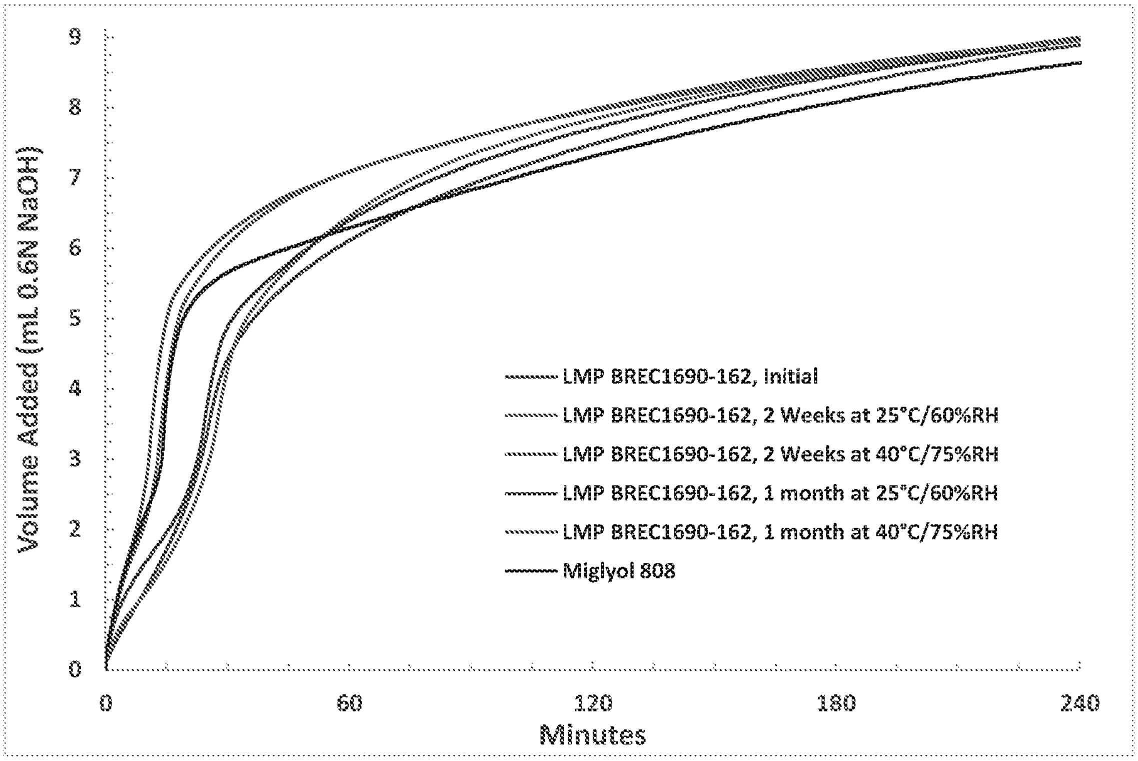


FIG. 26

FIG. 27A FIG. 27B FIG. 27C FIG. 27D

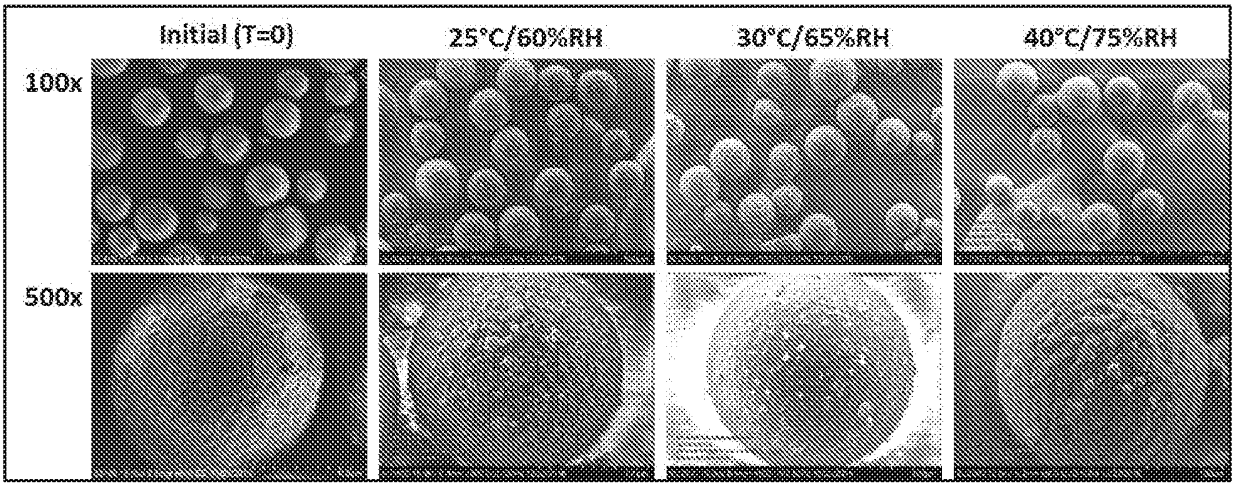

100x
500x

FIG. 28A
Initial (T=0)

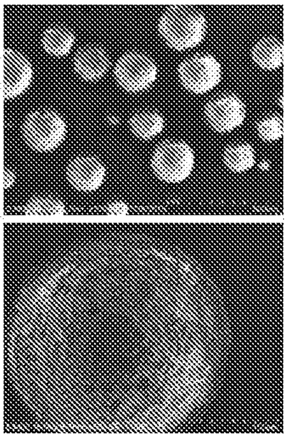

FIG. 28B
25°C/60%RH

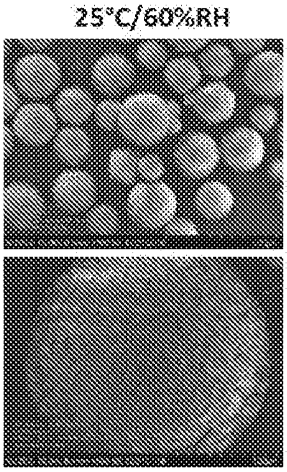

FIG. 28C
30°C/65%RH

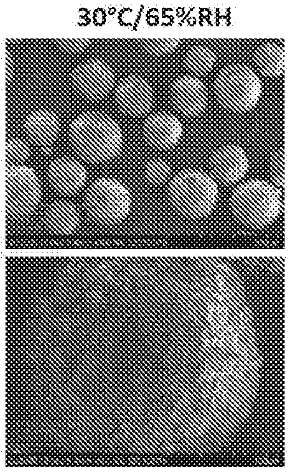

FIG. 28D
40°C/75%RH

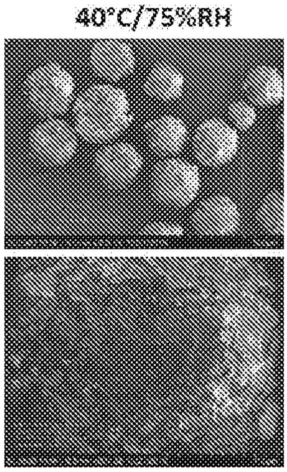

100x
500x

FIG. 29A
Initial (T=0)

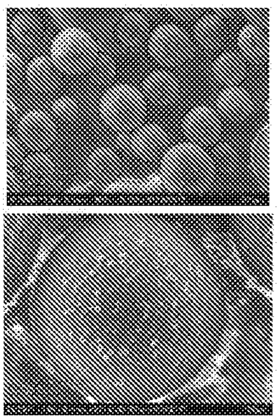

FIG. 29B
25°C/60%RH

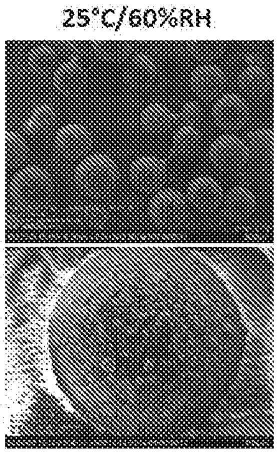

FIG. 29C
30°C/65%RH

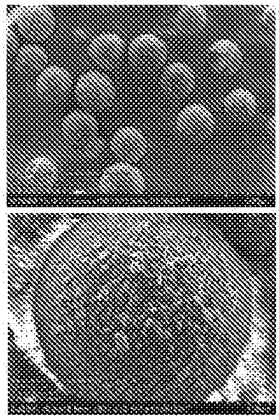

FIG. 29D
40°C/75%RH

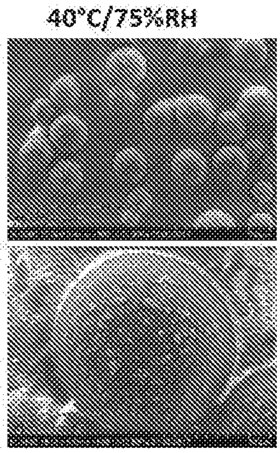

100x
500x

FIG. 30A
Initial (T=0)

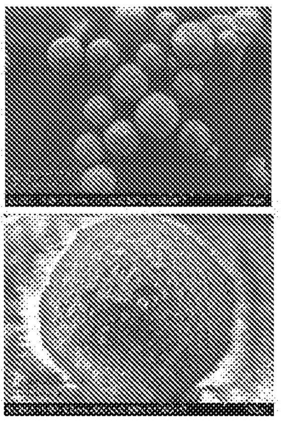

FIG. 30B
25°C/60%RH

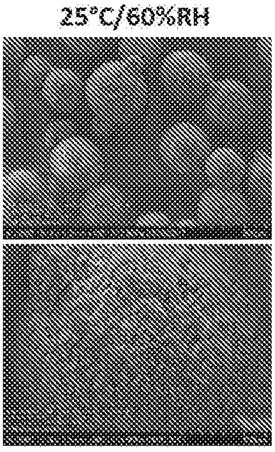

FIG. 30C
30°C/65%RH

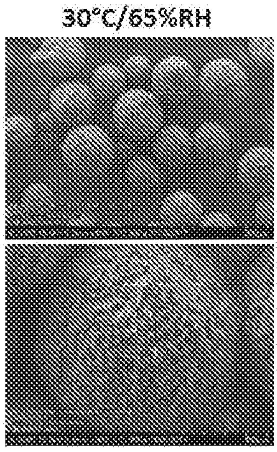

FIG. 30D
40°C/75%RH

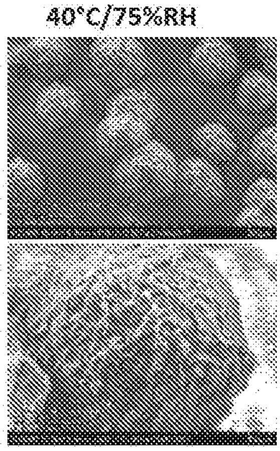

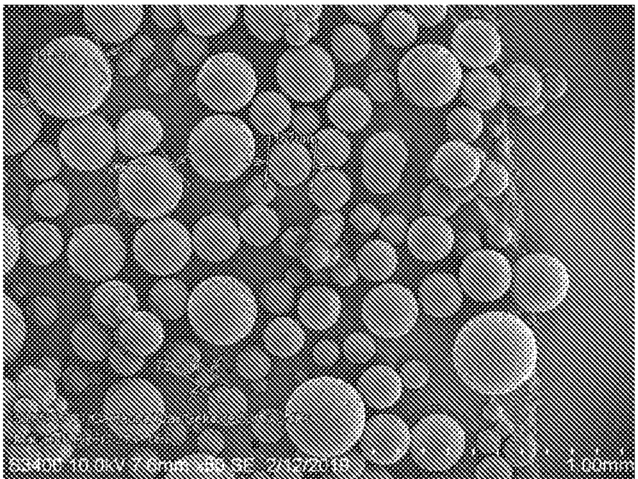

FIG. 31A

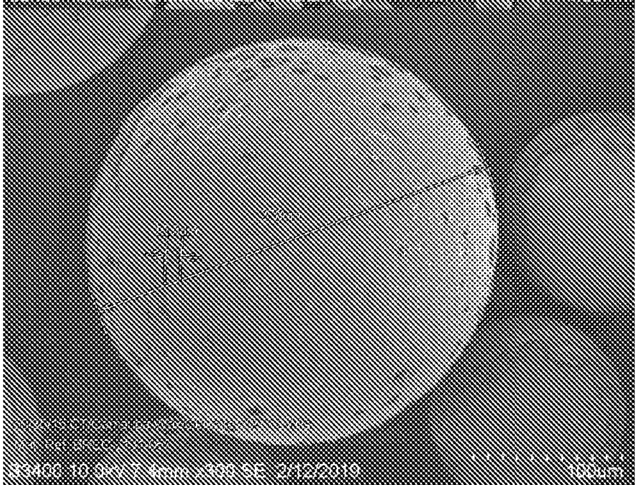

FIG. 31B

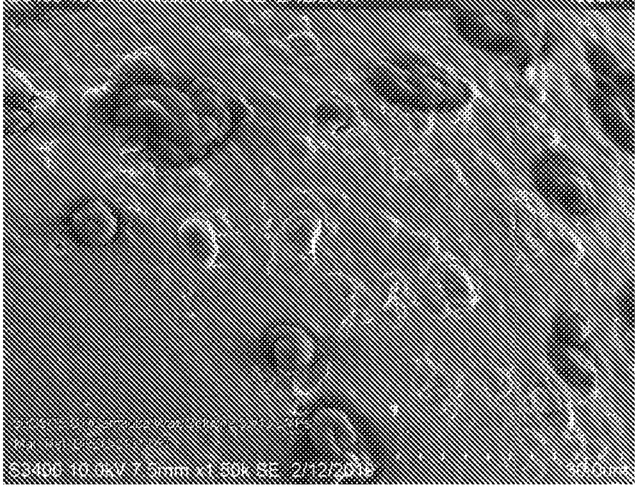

FIG. 31C

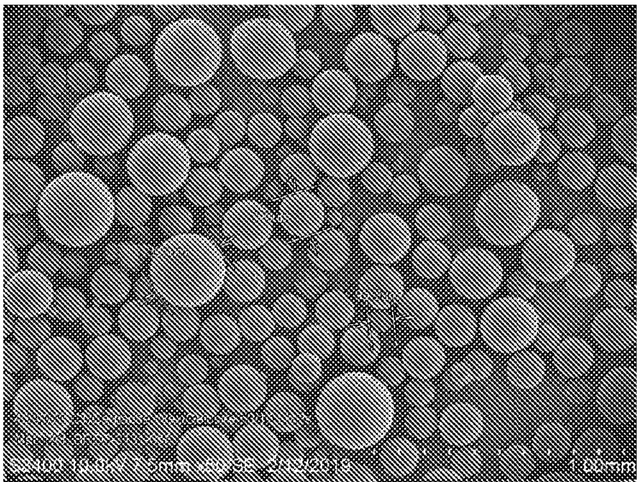

FIG. 32A

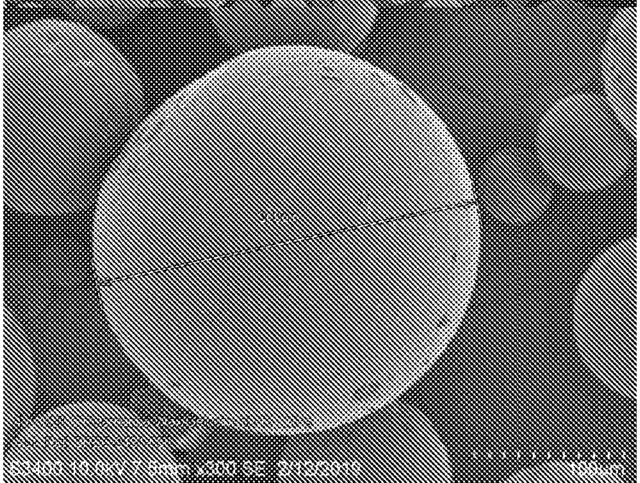

FIG. 32B

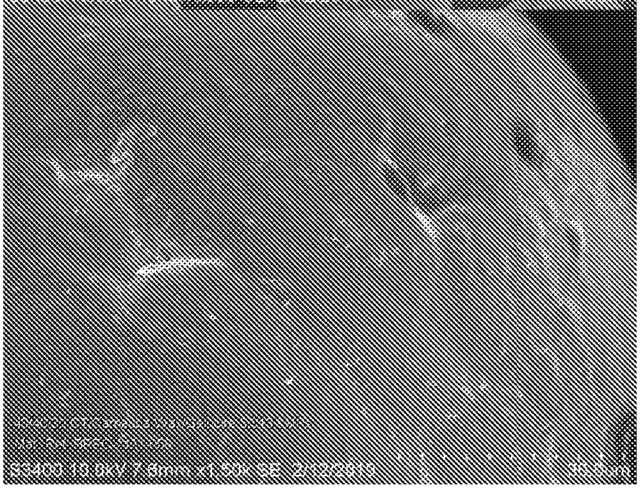

FIG. 32C

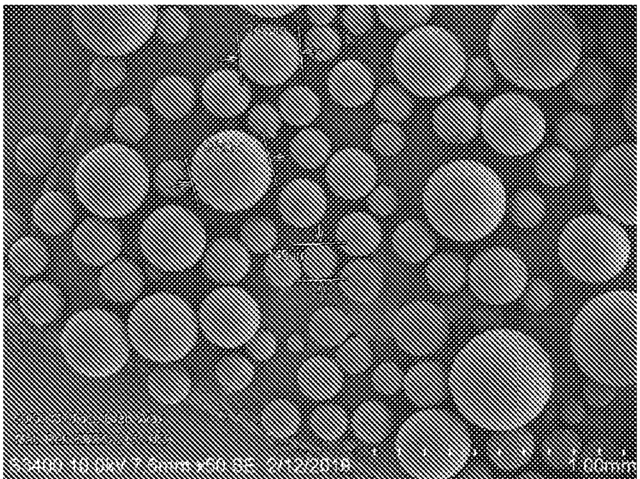

FIG. 33A

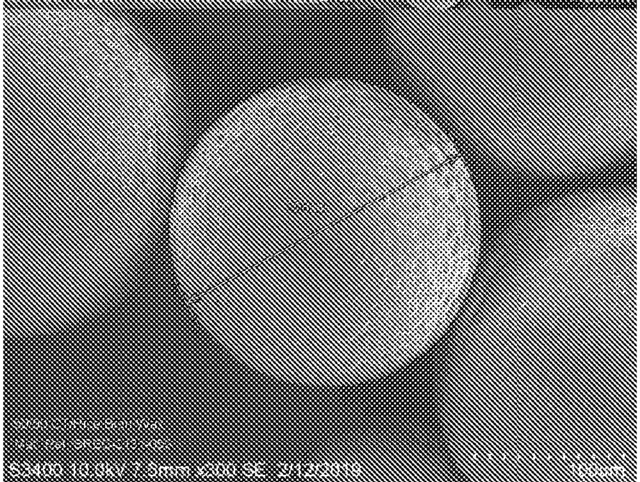

FIG. 33B

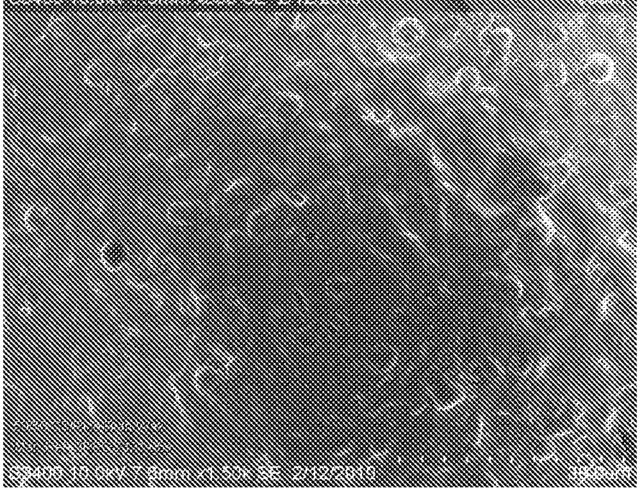

FIG. 33C

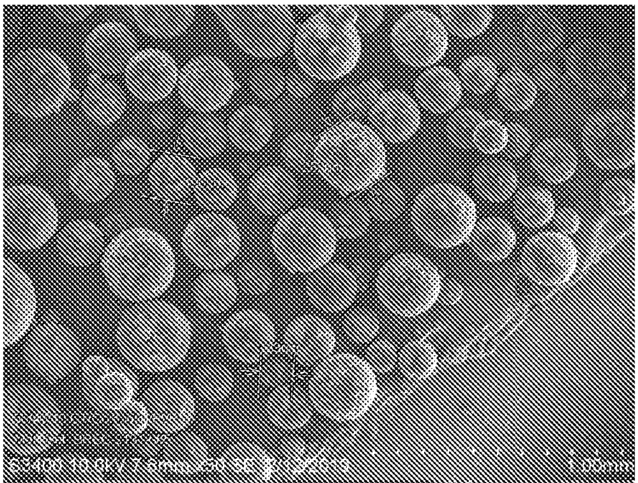

FIG. 34A

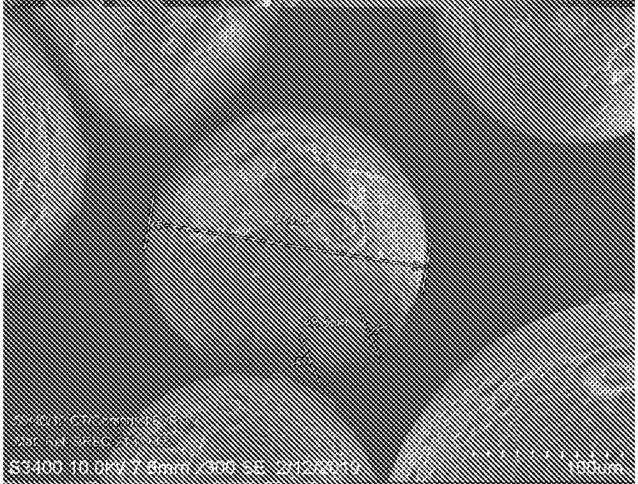

FIG. 34B

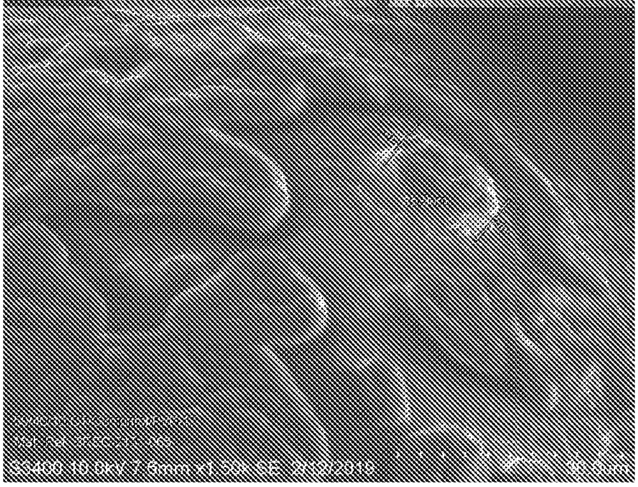

FIG. 34C

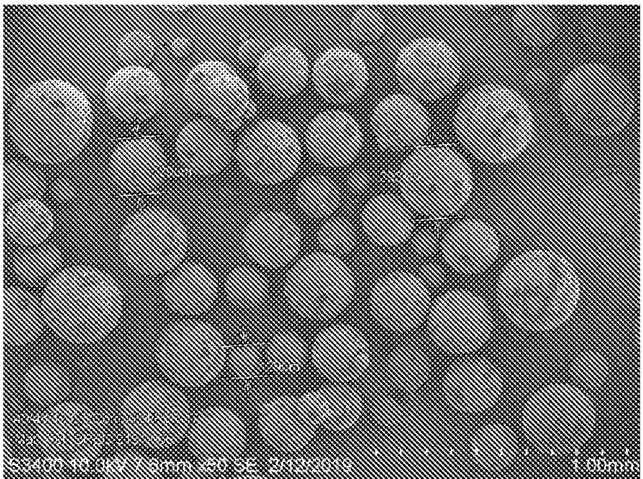

FIG. 35A

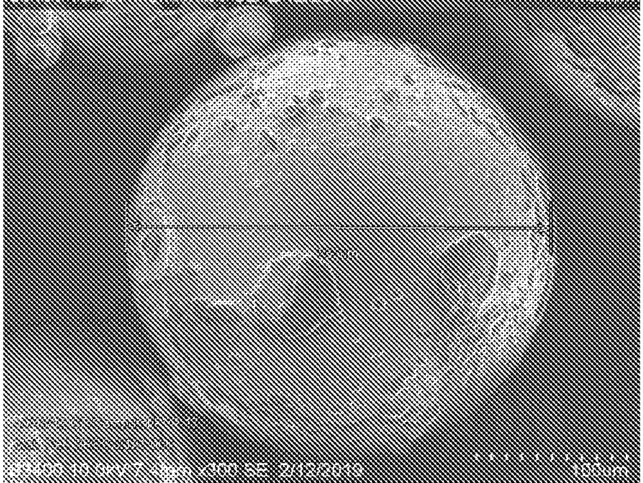

FIG. 35B

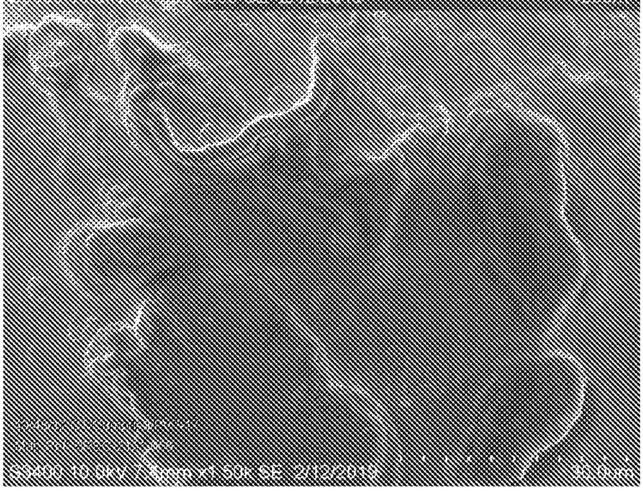

FIG. 35C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2319508 A1
- WO 2017149392 A1


### Non-patent literature cited in the description


- **HASSELBALCH, S.G. et al.** Changes in cerebral blood flow and carbohydrate metabolism during acute hyperketonemia. *Am J Physiol*, 1996, vol. 270, E746-51
- **VENEMAN, T. et al.** Effect of hyperketonemia and hyperlacticacidemia on symptoms, cognitive dysfunction, and counterregulatory hormone responses during hypoglycemia in normal humans. *Diabetes*, 1994, vol. 43, 1311-7